# EUROPÄISCHE PATENTSCHRIFT

(11) **EP 2 146 959 B1**
(45) Veröffentlichungstag und Bekanntmachung des Hinweises auf die Patenterteilung: **19.06.2013**
(21) Anmeldenummer: 08735181.3
(22) Anmeldetag: 11.04.2008
(51) Int. Cl.: C07D 211/14, C07D 213/70, C07D 401/04, C07C 311/08, A61K 31/444, A61K 31/18, A61P 29/00

(54) **VANILLOID-REZEPTOR LIGANDEN UND IHRE VERWENDUNG ZUR HERSTELLUNG VON ARZNEIMITTELN**
VANILLOID RECEPTOR LIGANDS AND THE USE THEREOF FOR THE PRODUCTION OF PHARMACEUTICALS
LIGANDS DE RÉCEPTEUR VANILLOÏDE ET LEUR UTILISATION POUR LA FABRICATION DE MÉDICAMENTS

(30) Priorität: 13.04.2007 DE 102007017884
(43) Veröffentlichungstag der Anmeldung: 27.01.2010
(73) Patentinhaber: Grünenthal GmbH, 52078 Aachen (DE)
(72) Erfinder: FRANK, Robert, 52070 Aachen (DE); BAHRENBERG, Gregor, 52156 Monschau-Konzen (DE); CHRISTOPH, Thomas, 52080 Aachen (DE); SCHIENE, Klaus, 41363 Jüchen (DE); DE VRY, Jean, 2200 Herentals (BE); SAUNDERS, Derek, John, 52072 Aachen (DE); LEE, Jeewoo, Seoul 151-742 (KR)
(74) Vertreter: Brosch, Oliver
(86) Internationale Anmeldenummer: PCT/EP2008/002884
(87) Internationale Veröffentlichungsnummer: WO 2008/125296

(56) Entgegenhaltungen:
- WO-A-2005/003084
- WO-A-2006/122773
- WO-A-2007/045462
- WO-A1-2006/101318

## Beschreibung

Die vorliegende Erfindung betrifft neue Vanilloid-Rezeptor-Liganden, Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Ein geeigneter Ansatzpunkt zur Behandlung von Schmerz; insbesondere von Schmerz ausgewählt aus der Gruppe bestehend aus akutem Schmerz, chronischem Schmerz, neuropathischem Schmerz und visceralem Schmerz, besonders bevorzugt von neuropathischem Schmerz; stellt der Vanilloid-Rezeptor vom Subtyp 1 (VR1/TRPV1) dar, der häufig auch als Capsaicin-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Entzündungen; Störungen des kardiovaskulären Systems; Störungen der Nahrungsaufnahme; Medikamentenabhängigkeit; Medikamentenmißbrauch und insbesondere Harninkontinenz.

Die WO 2005/003084 betrifft 4-(Methylsulfonylamino)-phenyl-Analoga als Vanilloid-Rezeptor-Antagonisten und deren pharmazeutische Zusammensetzungen.

Die WO 2006/122773 offenbart Pentafluorosulphanyl-substituierte Verbindungen, Verfahren zu deren Herstellung, Arzneimittel enthaltend diese Verbindungen sowie die Verwendung dieser Verbindungen zur Herstellung von Arzneimitteln.

Aus der WO 2007/045462 sind ebenfalls Vanilloid-Rezeptor-Liganden, Verfahren zu deren Herstellung, Arzneimittel enthaltend diese Verbindungen, sowie die Verwendung dieser Verbindungen zur Herstellung von Arzneimitteln bekannt.

Die WO 2006/101318 beschreibt Verbindungen, deren Isomere oder pharmazeutisch verträgliche Salze als Vanilloid-Rezeptor-(Vanilloid-Rezeptor 1, VR1, TRPV1)-Antagonisten und pharmazeutische Zusammensetzungen.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass die substituierten Verbindungen der nachstehend angegebenen allgemeinen Formel I eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanilloid-Rezeptoren 1 (VR1/TRPV1) vermittelt werden. Ebenfalls verfügen die substituierten Verbindungen der nachstehend angegebenen allgemeinen Formel I über eine antientzündliche Aktivität.

Ein Gegenstand der vorliegenden Erfindung sind daher substituierte Verbindungen der allgemeinen Formel I, 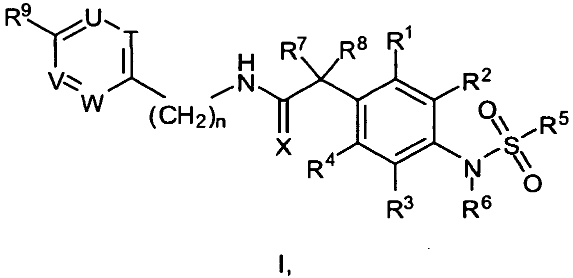 worin
X für O, S oder N-C≡N steht;
n für 0, 1, 2, 3 oder 4 steht;
R¹, R², R³ und R⁴, unabhängig voneinander, jeweils für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹⁰; -NR¹¹R¹²; -OR¹³; -SR¹⁴; -C(=O)-NHR¹⁵; -C(=O)-NR¹⁶R¹⁷; -S(=O)₂-NHR¹⁸; -S(=O)₂-NR¹⁹R²⁰; -C(=O)-OR²¹; - C(=O)-R²²; -S(=O)-R²³; -S(=O)₂-R²⁴ oder für einen linearen oder verzweigten, gesättigten oder ungesättigten, unsubstituierten oder wenigstens einfach substituierten aliphatischen C₁₋₁₀ Rest stehen;
R⁵ für -NH₂; -NHR²⁵; -NR²⁶R²⁷ oder für einen linearen oder verzweigten, gesättigten oder ungesättigten, unsubstituierten oder wenigstens einfach substituierten aliphatischen C₁₋₁₀ Rest steht;
R⁶ für -C(=O)-R²⁸ oder für einen linearen oder verzweigten, gesättigten oder ungesättigten, unsubstituierten oder wenigstens einfach substituierten aliphatischen C₁₋₁₀ Rest steht;
R⁷ und R⁸, unabhängig voneinander, jeweils für einen Wasserstoff-Rest;
für einen linearen oder verzweigten, gesättigten oder ungesättigten, unsubstituierten oder wenigstens einfach substituierten aliphatischen C₁₋₁₀ Rest;
für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest stehen; mit der Maßgabe, dass R⁷ und R⁸ nicht jeweils für einen Wasserstoff-Rest stehen;
oder .
R⁷ und R⁸ jeweils zusammen mit dem sie verbindenden Kohlenstoffatom als Ringglied einen gesättigten oder ungesättigten, unsubstituierten oder wenigstens einfach substituierten 3-, 4-, 5- oder 6-gliedrigen cycloaliphatischen Rest bilden;
T für C-R²⁹ und U für C-R³⁰ und V für N und W für C-R³²
oder
T für C-R²⁹ und U für N und V für C-R³¹ und W für C-R³²
oder
T für N und U für C-R³⁰ und V für C-R³¹ und W für C-R³²
oder
T für N und U für N und V für C-R³¹ und W für C-R³²
oder
T für N und U für C-R³⁰ und V für N und W für C-R³²
oder
T für C-R²⁹ und U für N und V für N und W für C-R³²
steht;
R⁹ für F; Cl; Br; I; -SF₅, -NO₂; -CF₃; -CN; -NH₂; -OH; -SH; -C(=O)-NH₂; -S(=O)₂-NH₂; -C(=O)-NH-OH; -C(=O)-OH: -C(=O)-H; -S(=O)₂-OH; -NHR¹⁰; -NR¹¹R¹²; - OR¹³; -SR¹⁴; -C(=O)-NHR¹⁵; -C(=O)-NR¹⁶R¹⁷; -S(=O)₂-NHR¹⁸; -S(=O)2-NR¹⁹R²⁰; -C(=O)-OR²¹; -C(=O)-R²²; -S(=O)-R²³; -S(=O)₂-R²⁴;
für einen linearen oder verzweigten, gesättigten oder ungesättigten, unsubstituierten oder wenigstens einfach substituierten aliphatischen C₁₋₁₀ Rest;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest steht;
R¹⁰, R¹¹, R¹², R¹³, R¹⁴, R¹⁵, R¹⁶, R¹⁷, R¹⁸, R¹⁹, R²⁰, R²¹, R²², R²³ und R²⁴, unabhängig voneinander, jeweils
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder 2- bis 6-gliedrige Heteroalkylen-Gruppe gebunden sein kann, stehen;
R²⁵, R²⁶ und R²⁷, unabhängig voneinander, jeweils für einen linearen oder verzweigten, gesättigten oder ungesättigten, unsubstituierten oder wenigstens einfach substituierten aliphatischen C₁₋₁₀ Rest stehen;
R²⁸ für einen linearen oder verzweigten, gesättigten oder ungesättigten, unsubstituierten oder wenigstens einfach substituierten aliphatischen C₁₋₁₀ Rest steht;
R²⁹, R³⁰ und R³¹, unabhängig voneinander, jeweils für H; F; Cl; Br; I; -SF₅; -NO₂; -CF₃; -CN; -NH₂; -OH; -SH; -C(=O)-NH₂; -S(=O)₂-NH₂; -C(=O)-NH-OH; - C(=O)-OH; -C(=O)-H; -S(=O)₂-OH; -NHR¹⁰; -NR¹¹R¹²; -OR¹³; -SR¹⁴; -C(=O)-NHR¹⁵; -C(=O)-NR¹⁶R¹⁷; -S(=O)₂-NHR¹⁵; -S(=O)₂-NR¹⁹R²⁰; -C(=O)-OR²¹; - C(=O)-R²²; -S(=O)-R²³; -S(=O)₂-R²⁴;
für einen linearen oder verzweigten, gesättigten oder ungesättigten, unsubstituierten oder wenigstens einfach substituierten aliphatischen C₁₋₁₀ Rest;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann, steht;
R³² für H; F; Cl; Br; I; -SF₅; -NO₂; -CF₃; -CF₂Cl; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR³³; - N³⁴R³⁵ -OR³⁶; -SR³⁷; -C(=O)-NHR³⁸; -C(=O)-NR³⁹R⁴⁰; -S(=O)₂-NHR⁴¹;-S(=O)₂-NR⁴²R⁴³; -C(=O)-OR⁴⁴; -C(=O)-R⁴⁵; -S(=O)-R⁴⁶; -S(=O)₂-R⁴⁷; -C(=NH)-NH₂; -C(=NH)-NH-R⁴⁸; -N=C(NH₂)₂; -N=C(NHR⁴⁹)(NHR⁵⁰);
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann, steht;
R³³, R³⁴, R³⁵, R³⁶ R³⁷, R³⁸ R³⁹, R⁴⁰, R⁴¹, R⁴², R⁴³, R⁴⁴, R⁴⁵, R⁴⁶, R⁴⁷, R⁴⁸, R⁴⁹ und R⁵⁰, unabhängig voneinander, jeweils
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder 2- bis 6-gliedrige Heteroalkylen-Gruppe gebunden sein kann, stehen;
oder
R³⁴ und R³⁵ jeweils zusammen mit dem sie verbindenden Stickstoffatom als Ringglied einen gesättigten oder ungesättigten, unsubstituierten oder mit 1, 2, 3, 4 oder 5 Resten R⁵¹ substituierten 4-, 5-, 6-, 7-, 8- oder 9-gliedrigen, ggf. wenigstens ein weiteres Heteroatom als Ringglied aufweisenden heterocycloaliphatischen Rest bilden, der mit einem gesättigten oder ungesättigten, unsubstituierten oder wenigstens einfach substituierten mono-oder polyzyklischen Ringsystem kondensiert sein kann;
R⁵¹ für -NHR⁵², -NR⁵³R⁵⁴ oder für einen linearen oder verzweigten, gesättigten oder ungesättigten, unsubstituierten oder wenigstens einfach substituierten aliphatischen C₁₋₁₀ Rest;
R⁵² R⁵³ und R⁵⁴, unabhängig voneinander, jeweils für -C(=O)-R⁵⁵; für einen linearen oder verzweigten, gesättigten oder ungesättigten, unsubstituierten oder wenigstens einfach substituierten aliphatischen C₁₋₁₀ Rest
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann, stehen;
und
R⁵⁵ für einen linearen oder verzweigten, gesättigten oder ungesättigten, unsubstituierten oder wenigstens einfach substituierten aliphatischen C₁₋₁₀ Rest steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate;
wobei
die vorstehend genannten aliphatischen C₁₋₁₀ Reste ggf. jeweils mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), - NH(C₁₋₅-Alkyl), -N(C₁₋₅-Alkyl)(C₁₋₅-Alkyl), -C(=O)-O-C₁₋₅-Alkyl, -O-C(=O)-C₁₋₅-Alkyl, - O-Phenyl, Phenyl, -OCF₃ und -SCF₃ substituiert sein können;
die vorstehend genannten 2- bis 6-gliedrigen Heteroalkylen-Gruppen, C₁₋₆-AlkylenGruppen und C₂₋₆-Alkenylen-Gruppen und C₂₋₆-Alkinylen-Gruppen ggf. jeweils mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert sein können;
die vorstehend genannten Heteroalkylen-Gruppen jeweils ggf. 1, 2 oder 3 Heteroatom(e) unabhängig voneinander ausgewählt aus der Gruppe bestehend aus Sauerstoff, Schwefel und Stickstoff (NH) als Kettenglied(er) aufweisen;
die vorstehend genannten (hetero)cycloaliphatischen Reste ggf. jeweils mit 1, 2, 3, 4 oder 5 Substituenten unabhängig voneinander ausgewählt aus der Gruppe bestehend aus -C₁₋₆-Alkylen-OH, =CH₂, -O-C₁₋₅-Alkylen-Oxetanyl, -C₁₋₅-Alkylen-O-C₁-₅-Alkylen-Oxetanyl,-CH₂-NH-C₁₋₅-Alkyl, -CH₂-N(C₁₋₅-Alkyl)₂, -N[C(=O)-C₁₋₅-Alkyl]-Phenyl, -CH₂-O-C₁₋₅-Alkyl, Oxo (=O), Thioxo (=S), F, Cl, Br, I, -CN, -CF₃, -SF₅, -OH, - O-C₁₋₅-Alkyl, -O-C(=O)-C₁₋₅-Alkyl, -NH₂, -NO₂, -O-CF₃, -S-CF₃, -SH, -S-C₁₋₅-Alkyl, -C₁-₅-Alkyl, -C(=O)-C₁₋₅-Alkyl, -C(=O)-OH, -C(=O)-O-C₁₋₅-Alkyl, -NH-C₁₋₅-Alkyl, -N(C₁₋₅-Alkyl)₂, -NH-Phenyl, -N(C₁₋₅-Alkyl)-Phenyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N[C(=O)-C₁₋₅-Alkyl]-Phenyl, -NH-Phenyl, -N(C₁₋₅-Alkyl)-Phenyl, -(CH₂)-Pyridinyl, Pyridinyl, -0-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann,
und, sofern nicht anders angegeben, die vorstehend genannten (hetero)cycloaliphatischen Reste jeweils ggf. 1, 2 oder 3 (weitere) Heteroatom(e) unabhängig voneinander ausgewählt aus der Gruppe bestehend aus Sauerstoff, Stickstoff und Schwefel aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-C₁₋₅-Alkyl, -C(=O)-OH, -C(=O)-O-C₁₋₅-Alkyl, -NH-C₁₋₅-Alkyl, -N(C₁₋₅-Alkyl)₂, -O-Phenyl, -0-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, - S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-S(=O)₂-C₁₋₅-Alkyl, -NH-C(=O)-O-C₁₋₅-Alkyl, -C(=O)-H, -C(=O)-C₁-₅-Alkyl, -C(=O)-NH₂, -C(=O)-NH-C₁₋₅-Alkyl, -C(=O)-N-(C₁₋₅-Alkyl)₂, -O-Phenyl, -0-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und
die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen.

Der Begriff "Heteroalkylen" bezeichnet eine Alkylen-Kette, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkylen-Gruppen können bevorzugt 1, 2 oder 3 Heteroatom(e), besonders bevorzugt ein Heteroatom, unabhängig voneinander ausgewählt aus der Gruppe bestehend aus Sauerstoff, Schwefel und Stickstoff (NH) als Kettenglied(er) aufweisen. Heteroalkylen-Gruppen können bevorzugt 2- bis 6-gliedrig, besonders bevorzugt 2- oder 3-gliedrig, sein.

Beispielhaft seien Heteroalkylen-Gruppen wie -CH₂-CH₂-O-CH₂-, -CH₂-CH(CH₃)-O-CH₂-, -(CH₂)-O-, -(CH₂)₂-O-, -(CH₂)₃-O-, -(CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-, -CH₂-O-CH₂-, -CH₂-S-CH₂-, -CH₂-NH-CH₂-, -CH₂-NH- und -CH₂-CH₂-NH-CH₂-CH₂ genannt.

Sofern einer oder mehrere der vorstehend genannten Substituenten eine lineare oder verzweigte C₁₋₆-Alkylen-Gruppe aufweisen, kann diese bevorzugt ausgewählt werden aus der Gruppe bestehend aus -(CH₂)-, -(CH₂)₂-, -C(H)(CH₃)-, -(CH₂)₃-, - (CH₂)₄-, -(CH₂)₅-, -C(H)(C(H)(CH₃)₂)- und -C(C₂H₅)(H)-.

Gesättigte oder ungesättigte C₁₋₁₀ aliphatischen Reste können für einen C₁₋₁₀-Alkyl-, C₂₋₁₀-Alkenyl- oder C₂₋₁₀-Alkinyl-Rest stehen. C₂₋₁₀-Alkenyl-Reste weisen wenigstens eine, vorzugsweise 1, 2, 3 oder 4 C-C-Doppelbindungen und C₂₋₁₀-Alkinyl-Reste wenigstens eine, vorzugsweise 1, 2, 3 oder 4 C-C-Dreifachbindungen auf.

Bevorzugt sind C₁₋₁₀-Alkyl-Reste ausgewählt aus der Gruppe bestehend aus Methyl, Ethyl, n-Propyl, Isopropyl, n-Butyl, sek-Butyl, Isobutyl, tert-Butyl, n-Pentyl, 3-Methyl-but-1-yl, 2-Pentyl, 3-Pentyl, sec-Pentyl, neo-Pentyl, 4-Methyl-pent-1-yl, (3,3)-Dimethyl-but-1-yl, n-Hexyl, n-Heptyl, 2-Heptyl, 3-Heptyl, 4-Heptyl, n-Octyl, n-Nonyl, 2-Nonyl, 3-Nonyl, 4-Nonyl, 5-Nonyl und (2,6)-Dimethyl-hept-4-yl, die ggf. mit 1, 2, 3, 4, 5, 6, 7, 8 oder 9 Substituenten unabhängig voneinander ausgewählt aus der Gruppe bestehend aus -O-Phenyl, -O-C(=O)-CH₃, -O-C(=O)-C₂H₅, -O-C(=O)-CH(CH₃)₂, -0-C(=O)-C(CH₃)₃, -C(=O)-O-CH₃, -C(=O)-O-C₂H₅, -C(=O)-O-CH(CH₃)₂, -C(=O)-O-C(CH₃)₃, F, Cl, Br, I, -CN, -NO₂, -OH, -NH₂, -SH, -O-CH₃, -O-C₂H₅, -O-CH(CH₃)₂, -0-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, -N(C₂H₅)₂, -N(CH₃)(C₂H₅), -OCF₃ und -SCF₃ substituiert sein können.

Ebenfalls bevorzugt sind C₂₋₁₀-Alkenyl-Reste ausgewählt aus der Gruppe bestehend aus Vinyl, 1-Propenyl, 2-Propenyl, 1-Butenyl, 2-Butenyl, 3-Butenyl, 2-Methyl-propen-1-yl, 3-Methyl-but-2-en-1-yl, (3,3)-Dimethyl-but-1-enyl, 2-Methyl-buten-2-yl, 1-Pentenyl, 2-Pentenyl, 3-Pentenyl, 4-Pentenyl, 1-Hexenyl, 1-Heptenyl und 1-Octenyl, die ggf. mit 1, 2 oder 3 Substituenten unabhängig voneinander ausgewählt aus der Gruppe bestehend aus F, Cl, Br, I, -CN, -NO₂, -OH, -NH₂, -SH, -O-CH₃, -O-C₂H₅, -0-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, -N(C₂H₅)₂, -N(CH₃)(C₂H₅), -OCF₃ und -SCF₃ substituiert sein können.

Weiterhin bevorzugt sind C₂₋₁₀-Alkinyl-Reste ausgewählt aus der Gruppe bestehend aus (3,3)-Dimethyl-but-1-inyl, 4-Methyl-pent-1-inyl, 1-Hexinyl, Ethinyl, 1-Propinyl, 2-Propinyl, 1-Butinyl, 2-Butinyl, 3-Butinyl, 1-Pentinyl, 2-Pentinyl, 3-Pentinyl und 4-Pentinyl, die ggf. mit 1, 2 oder 3 Substituenten unabhängig voneinander ausgewählt aus der Gruppe bestehend aus F, Cl, Br, I, -CN, -NO₂, -OH, -NH₂, -SH, -O-CH₃, -0-C₂H₅, -O-CH(CH₃)₂, -O-C(CH₃)₃, -S-CH₃, -S-C₂H₅, -S-CH(CH₃)₂, -S-C(CH₃)₃, -NH-CH₃, -NH-C₂H₅, -NH-C(CH₃)₃, -N(CH₃)₂, -N(C₂H₅)₂, -N(CH₃)(C₂H₅), -OCF₃ und -SCF₃ substituiert sein können.

Besonders bevorzugte ggf. substituierte C₁₋₁₀ aliphatische Rest sind ausgewählt aus der Gruppe bestehend aus Methyl, -CF₃, -CHF₂, -CH₂F, -CF₂Cl, -CCl₂F, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), Ethyl, -CF₂-CH₃, -CHF-CF₂Cl, -CF₂-CFCl₂, -CFCl-CF₂Cl, -CFCl-CFCl₂, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, - CH₂-CH₂-CH₂-OH, -CH₂-CH₂-CH₂-SH, -CH₂-CH₂-CH₂-NH₂, -CH₂-CH₂-CH₂-NH-CH₃, - CH₂-CH₂-CH_{Z}-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, - CF₂-CF₂-CF₂-CF₃, -CH₂-CH₂-CH₂-CH₂-CN, -CH₂-CH₂-CH₂-CF₃, -CH₂-CH₂-CH₂-CH₂-CF₃, -CH₂-O-C(=O)-CH₃, -CH₂-O-C(=O)-C₂H₅, -CH₂-O-C(=O)-CH(CH₃)₂, -CH₂-O-C(=O)-C(CH₃)₃, -CH₂-C(=O)-O-CH₃, -CH₂-C(=O)-O-C₂H₅, -CH₂-C(=O)-O-C(CH₃)₃, - CH₂-CH₂-O-CH₃, -CH₂-CH₂-O-C₂H₅, -CH₂-CH₂-O-Phenyl, -CH₂-CH₂-CH₂-O-CH₃, sek-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl, 4-Pentenyl, -CF=CF₂, - CCl=CCl₂, -CH₂-CF=CF₂, -CH₂-CCl=CCl₂, -C≡C-I, -C≡C-F und -C≡C-Cl.

Sofern einer oder mehrere der vorstehend genannten Substituenten für einen (hetero)cycloaliphatischen Rest stehen, der ggf. mit einem gesättigten oder ungesättigten, unsubstituierten oder wenigstens einfach substituierten mono- oder polyzyklischen Ringsystem kondensiert sein kann, kann dieser bevorzugt ausgewählt werden aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Oxetanyl, (1,2,3,6)-Tetrahydropyridinyl, Azepanyl, Azocanyl, Diazepanyl, Dithiolanyl, (1,3,4,5)-Tetrahydropyrido[4,3-b]indolyl, (3,4)-Dihydro-1 H-isochinolinyl, (1,3,4,9)-Tetrahydro-[b]-carbolinyl und (1,3)-Thiazolidinyl.

Als geeignete (hetero)cycloaliphatische Reste, die unsubstituiert oder einfach oder mehrfach substituiert sein können, und mit einem mono- bzw. bizyklischem Ringsystem kondensiert sind, seien beispielhaft (4,5,6,7)-Tetrahydroisoxazolo[5,4-c]pyridinyl, (2,3)-Dihydro-1H-indenyl, 3-Aza-bicyclo[3.1.1]heptyl, 3-Aza-bicyclo[3.2.1]octyl, 6-Aza-bicyclo[3.3.1]heptyl, 8-Aza-bicyclo[3.2.1]octyl, Isoindolyl, Indolyl, (1,2,3,4)-Tetrahydrochinolinyl, (1,2,3,4)-Tetrahydroisochinolinyl, (2,3)-Dihydro-1H-isoindolyl, (1,2,3,4)-Tetrahydronaphthyl, (2,3)-Dihydro-benzo[1.4]dioxinyl, Benzo[1.3]dioxolyl, (1,4)-Benzodioxanyl, (2,3)-dihydrothieno[3,4-b][1,4]dioxinyl, (3,4)-Dihydro-2H-benzo[1.4]oxazinyl, Octahydro-1 H-isoindolyl und Octahydro-pyrrolo[3,4-c]pyrrolyl genannt.

(Hetero)cycloaliphatische Reste können im Sinne der vorliegenden Erfindung mit einem weiteren (hetero)cycloaliphatischen Rest über ein gemeinsames Kohlenstoffatom in beiden Ringen einen spirozyklischen Rest bilden.

Als geeignete spirozyklischer Reste seien beispielsweise ein 6-Aza-spiro[2.5]octyl-Rest, 8-Azaspiro[4.5]decyl-Rest und ein 1-Oxa-2,8-diaza-spiro[4.5]dec-2-enyl-Rest genannt.

Besonders bevorzugt können die (hetero)cycloaliphatischen Reste ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, - S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), - CH₂-OH, -CH₂-CH₂-OH, =CH₂, -CH₂-O-CH₂-Oxetanyl, -O-CH₂-Oxetanyl, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, -CH₂-O-CH₃, -CH₂-O-CH₂-CH₃, -NH-Phenyl, -N(CH₃)-Phenyl, - N(C₂H₅)-Phenyl, -N(C₂H₅)-Phenyl, -O-CH₂-CH₂-CH₂-CH₃, (4,5)-Dihydroisoxazolyl, Thiazolyl, (1,2,5)-Thiadiazolyl, Thiophenyl, Phenethyl, Cyclohexyl, Cyclopentyl, Piperidinyl, Pyrrolidinyl, -O-C(=O)-CH₃, -O-C(=O)-C₂H₅, -O-C(=O)-C(CH₃)₃, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N-[C(=O)-C₂H₅]-Phenyl, -N-[C(=O)-CH₃]-Phenyl, -NH-Phenyl, -N(CH₃)-Phenyl, -N(C₂H₅)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und -O-Benzyl substituiert sein kann.

Sofern einer oder mehrere der vorstehend genannten Substituenten für einen ArylRest stehen, kann dieser bevorzugt ausgewählt werden aus der Gruppe bestehend aus Phenyl und Naphthyl (1-Naphthyl und 2-Naphthyl).

Sofern einer oder mehrere der vorstehend genannten Substituenten für einen Heteroaryl-Rest stehen, kann dieser bevorzugt ausgewählt werden aus der Gruppe bestehend aus Tetrazolyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzoxazolyl, Benzisoxazolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl.

Als geeignete Aryl- und Heteroaryl-Reste, die unsubstituiert oder einfach oder mehrfach substituiert sein können, und mit einem mono- bzw. bizyklischem Ringsystem kondensiert sind, seien beispielhaft Isoindolyl, Indolyl, (1,2,3,4)-Tetrahydrochinolinyl, (1,2,3,4)-Tetrahydroisochinolinyl, (2,3)-Dihydro-1H-isoindolyl, (1,2,3,4)-Tetrahydronaphthyl, (2,3)-Dihydro-benzo[1.4]dioxinyl, (2,3)-Dihydrothieno[3,4-b][1,4]dioxinyl, Benzo[1.3]dioxolyl und (1,4)-Benzodioxanyl genannt.

Besonders bevorzugt können die Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -0-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), - NH-S(=O)₂-CH₃, -NH-S(=O₂)-C₂H₅, -NH-S(=O)₂-CH(CH₃)₂, -NH-C(=OF_{O}-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -0-CH(CH₃)₂, -O-C(CH₃)₃, -O-CF₃, -S-CF₃, Phenyl und -O-Benzyl substituiert sein kann.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt oder ungesättigt sein. Bevorzugt ist ein polyzyklisches Ringsystem ein bizyklisches Ringsystem.

Beispielhaft für Aryl-Reste, die mit einem mono- bzw. polyzyklischen Ringsystem kondensiert sind, seien (1,3)-Benzodioxolyl und (1,4)-Benzodioxanyl genannt. Sofern einer oder mehrere der vorstehend genannten Substituenten ein mono- oder polyzyklisches Ringsystem aufweisen, kann dieses bevorzugt mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -0-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, - N(CH₃)(C₂H₅), -NH-C(=O)-O-CH₃, -NH-C(=O)-O-C₂H₅, -NH-C(=O)-O-C(CH₃)₃, - C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -0-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, - CF₃, -SF₅, -CN, -NO₂, Methyl, Ethyl, n-Propyl, Isopropyl, n-Butyl, sek-Butyl, Isobutyl, tert-Butyl, -O-CH₃, -O-C₂H₅, -O-CH(CH₃)₂, -O-C(CH₃)₃, -O-CF₃, -S-CF₃, Phenyl und - O-Benzyl substituiert sein kann.

Bevorzugt sind C₂₋₆-Alkenylen-Gruppen wie -CH=CH- und -CH₂-CH=CH-.

Bevorzugt sind C₂₋₃-Alkinylen-Gruppen wie -C≡C- und -CH₂-C≡C-.

Bevorzugt sind Verbindungen der vorstehend angegebenen allgemeinen Formel I, worin
X für O steht;
n für 0, 1 oder 2 steht;
R¹, R², R³ und R⁴, unabhängig voneinander, jeweils für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH; - C(=O)-H; -S(=O)₂-OH; -NHR¹⁰; -NR¹¹R¹²; -OR¹³; -SR¹⁴; -S(=O)-R²³; -S(=O)₂-R²⁴ oder für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl_{3,} -CH₂-CBr₃, -CHF-CF₂Cl, -CF₂-CF₂Cl, -CFCl-CF₂Cl, n-Propyl, -CF₂-CF₂-CF₃, -CF(CF₃)₂, Isopropyl, n-Butyl, sek-Butyl, Isobutyl und tert-Butyl stehen;
R⁵ für -NH₂; -NHR²⁵; -NR²⁶R²⁷; für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
R⁶ für -C(=O)-R²⁸ oder für einen Rest ausgewählt aus der Gruppe bestehend aus Methyl, -CF₃, -CCl₃, -CBr₃, -CHF₂, -CH₂F, -CF₂Cl, -CCl₂F, -CH₂-CN, -CH₂-O-CH₃, - CH₂-O-CF₃, -CH₂-SF₃, Ethyl, -CF₂-CH₃, -CH₂-CF₃, -C₂F₅, -CH₂-CCl₃, -CH₂-CBr₃, - CHF-CF₂Cl, -CF₂-CF₂Cl, -CFCl-CF₂Cl, -CH₂-CH₂-CN, n-Propyl, -CF₂-CF₂-CF₃, - CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃, -CH₂-CH₂-CH₂-CH₂-CN, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-CH₂-O-CH₃, Ethenyl, Propenyl, 2-Butenyl, 3-Butenyl, 2-Pentenyl und 3-Pentenyl steht;
R⁷ und R⁸, unabhängig voneinander, jeweils für einen Wasserstoff-Rest;
für einen Alkyl-Rest ausgewählt aus der Gruppe bestehend aus -CH₂-OH, -CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine -(CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -0-CH₃, -O-C₂H₅, -O-CH(CH₃)₂, -O-C(CH₃)₃, -NH₂, -NO₂, -O-CF₃, -S-CF₃, -SH, -S-CH₃, - S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Cyclopropyl, Cyclobutyl, Cyclopentyl, Cyclohexyl und Cycloheptyl stehen;
mit der Maßgabe, dass R⁷ und R⁸ nicht jeweils für einen Wasserstoff-Rest stehen; oder
R⁷ und R⁸ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
T für C-R²⁹ und U für C-R³⁰ und V für N und W für C-R³²
oder
T für C-R²⁹ und U für N und V für C-R³¹ und W für C-R³²
oder
T für N und U für C-R³⁰ und V für C-R³¹ und W für C-R³²
oder
T für N und U für N und V für C-R³¹ und W für C-R³²
oder
T für N und U für C-R³⁰ und V für N und W für C-R³²
oder
T für C-R²⁹ und U für N und V für N und W für C-R³²
steht;
R⁹ für F; Cl; Br; I; -SF₅; -OR¹³; -SR¹⁴; -S(=O)-R²³; -S(=O)₂-R²⁴; für einen Rest ausgewählt aus der Gruppe bestehend aus Methyl, -CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, Ethyl, -CF₂-CH₃, - CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-Propyl, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃, -CH₂-CH₂-CH₂-CH₂-CN, n-Butyl, sek-Butyl, Isobutyl, - C(CH₃)₂(CH₂OH) und tert-Butyl;
oder für einen Rest ausgewählt aus der Gruppe bestehend aus Cyclopropyl, Cyclobutyl, Cyclopentyl, Cyclohexyl, Cycloheptyl, Cyclopentenyl und Cyclohexenyl steht, der ggf. jeweils mit 1, 2, 3, 4 oder 5 Substituenten unabhängig voneinander ausgewählt aus der Gruppe bestehend aus Oxo (=O), Thioxo (=S), -OH, -O-CH₃, -0-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
R¹⁰, R¹¹, R¹², R¹³, R¹⁴, R²¹, R²³ und R²⁴, unabhängig voneinander, jeweils
für einen Rest ausgewählt aus der Gruppe bestehend aus Methyl, -CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, Ethyl, - CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-Propyl, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-Butyl, -CF₂-CF₂-CF₂-CF₃, -CH₂-CH₂-CH₂-CH₂-CN, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1 H-indenyl, Cyclopropyl, Oxetanyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über eine -CH₂-O-, -CH₂-CH₂-O-, -CH₂-CH₂-O-CH₂-, -CH₂-CH(CH₃)-O-CH₂-, -(CH₂)-, - (CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, - C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Thiazolyl, Oxazolyl und Isoxazolyl, wobei der Rest jeweils über eine - (CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -0-CH₃, -O-C₂H₅, -O-CH(CH₃)₂, -O-C(CH₃)₃, -NH₂, -NO₂, -O-CF₃, -S-CF₃, -SH, -S-CH₃, - S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, - C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, - C(=O)-NH₂, -C(=O)-NH-CH₃, -C(=O)-NH-C₂H₅, -C(=O)-N(CH₃)₂, -C(=O)-N(C₂H₅)₂, - O-Phenyl, -O-Benzyl, Phenyl und Benzyl substituiert sein kann, stehen;
R²⁵, R²⁶ und R²⁷, unabhängig voneinander, jeweils für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl stehen;
R²⁸ für einen Rest ausgewählt aus der Gruppe bestehend aus Methyl, -CF₃, -CCl₃, -CBr₃, -CHF₂, -CH₂F, -CF₂Cl, -CH₂-CN, -CH₂-O-CH₃, -CH₂-O-CF₃, -CH₂-SF₃, Ethyl, -CF₂-CH₃, -CH₂-CF₃, -C₂F₅, -CH₂-CCl₃, -CH₂-CBr₃, -CHF-CF₂Cl, -CF₂-CF₂Cl, - CFCl-CF₂Cl, -CH₂-CH₂-CN, n-Propyl, Isopropyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, Ethenyl, Propenyl, 2-Butenyl, 3-Butenyl, 2-Pentenyl und 3-Pentenyl steht;
R²⁹, R³⁰ und R³¹, unabhängig voneinander, jeweils für H; F; Cl; Br; I; -SF₅; -NO₂; -CN; -NH₂; -OH; -SH; -C(=O)-NH₂; -S(=O)₂-NH₂; -C(=O)-NH-OH; -C(=O)-OH; - C(=O)-H; -S(=O)₂-OH, -NHR¹⁰; -NR¹¹R¹²; -OR¹³; -SR¹⁴; -C(=O)-OR²¹; -S(=O)-R²³; - S(=O)₂-R²⁴; für einen Rest ausgewählt aus der Gruppe bestehend aus -CH₂-OH, Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr_{3;} -CHF-CF₂Cl, -CF₂-CF₂Cl, -CFCl-CF₂Cl, n-Propyl, -CF₂-CF₂-CF₃, -CF(CF₃)₂, Isopropyl, n-Butyl, sek-Butyl, Isobutyl und tert-Butyl oder für einen Phenyl-Rest stehen, der unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
R³² für H; -SF₅; -NO₂; -CN; -NH₂; -OH; -SH; -C(=O)-NH₂; -S(=O)₂-NH₂; -C(=O)-NH-OH; -C(=O)-OH; -C(=O)-H: -S(=O)₂-OH; -NHR³³; -NR³⁴R³⁵; -OR³⁶; -SR³7; -C(=O)-OR⁴⁴; -S(=O)-R⁴⁶; -S(=O)₂-R⁴⁷; -C(=NH)-NH₂; -C(=NH)-NH-R⁴⁸; -N=C(NH₂)₂; - N=C(NHR⁴⁹)(NHR⁵⁰);
für einen Rest ausgewählt aus der Gruppe bestehend aus Cyclopropyl, Cyclobutyl, Cyclopentyl, Cyclohexyl, Cyclohexenyl, lmidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C=C- oder -C=C-CH₂-Gruppe an das Grundgerüst gebunden ist und unsubstituiert oder ggf. jeweils mit 1, 2, 3, 4 oder 5 Substituenten unabhängig voneinander ausgewählt aus der Gruppe bestehend aus-CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃)-Phenyl, Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, - S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂ und - C(=O)-O-C(CH₃)₃ substituiert sein kann;
oder für einen Rest ausgewählt aus der Gruppe bestehend aus (1,3)-Benzodioxolyl, (1,4)-Benzodioxanyl, Tetrazolyl, (2,3)-Dihydrothieno[3,4-b][1,4]dioxinyl, Benzo[b]furanyl, Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine -(CH=CH)-, -C=C-, -(CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -0-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅),-NH-C(=O)-O-CH₃, -NH-S(=O)₂-CH₃, -NH-S(=O₂)C₂H₅, -NH-S(=O)₂-CH(CH₃)₂, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, steht;
R³³, R³⁴, R³⁵, R³⁶, R³⁷, R⁴⁴, R⁴⁶, R⁴⁷, R⁴⁸, R⁴⁹ und R⁵⁰, unabhängig voneinander, jeweils
für einen Rest ausgewählt aus der Gruppe bestehend aus Methyl, -CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, Ethyl, - CF₂-CH₃, -CH₂-CF₃, -C₂F₅, -CH₂-CCl₃, -CH₂-CBr₃, -CHF-CF₂Cl, -CF₂-CF₂Cl, -CFCI-CF₂Cl, -CH₂-CH₂-CN, n-Propyl, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-Butyl, -CF₂-CF₂-CF₂-CF₃, -CH₂-CH₂-CH₂-CH₂-CN, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-CH₂-O-CH₃, Ethenyl, Propenyl, 2-Butenyl, 3-Butenyl, 2-Pentenyl und 3-Pentenyl;
für einen Rest ausgewählt aus der Gruppe bestehend aus 2,3-Dihydro-1 H-indenyl, Cyclopropyl, Oxetanyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über eine -CH₂-O-, -CH₂-CH₂-O-, -CH₂-CH₂-O-CH₂-, -CH₂-CH(CH₃)-O-CH₂-, -(CH₂)-, - (CH- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -0-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, - C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Thiazolyl, Oxazolyl und Isoxazolyl, wobei der Rest jeweils über eine - (CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -0-CH₃, -O-C₂H₅, -O-CH(CH₃)₂, -O-C(CH₃)₃, -NH₂, -NO₂, -O-CF₃, -S-CF₃, -SH, -S-CH₃, - S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, - C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, - C(=O)-NH₂, -C(=O)-NH-CH₃, -C(=O)-NH-C₂H₅, -C(=O)-N(CH₃)₂, -C(=O)-N(C₂H₅)₂, - O-Phenyl, -O-Benzyl, Phenyl und Benzyl substituiert sein kann, stehen;
oder
R³⁴ und R³⁵ jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl,
(1,2,3,6)-Tetrahydropyridinyl, (4,5,6,7)-Tetrahydroisoxazolo[5,4-c]pyridinyl, Pyrrolidinyl, Piperidinyl, Piperazinyl, Morpholinyl, Azepanyl, Diazepanyl und Thiomorpholinyl bilden, dessen heterocycloaliphatischer Anteil jeweils unsubstituiert oder mit 1, 2, 3, 4 oder 5 Resten R⁵¹ substituiert sein kann;
R⁵¹ für -NHR⁵², -NR⁵³R⁵⁴ oder für einen Alkyl-Rest
ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
R⁵², R⁵³ und R⁵⁴, unabhängig voneinander, jeweils für -C(=O)-R⁵⁵; für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Thiazolyl, Oxazolyl und Isoxazolyl, wobei der Rest jeweils über eine - (CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -0-CH₃, -O-C₂H₅, -O-CH(CH₃)₂, -O-C(CH₃)₃, -NH₂, -NO₂, -O-CF₃, -S-CF₃, -SH, -S-CH₃, - S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, - C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, - C(=O)-NH₂, -C(=O)-NH-CH₃, -C(=O)-NH-C₂H₅, -C(=O)-N(CH₃)₂, -C(=O)-N(C₂H₅)₂, - O-Phenyl, -O-Benzyl, Phenyl und Benzyl substituiert sein kann, stehen;
und
R⁵⁵ für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel Ia, 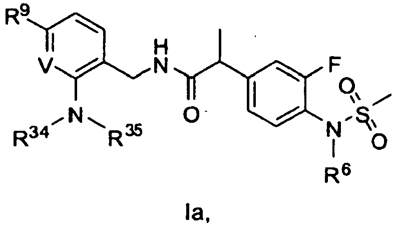 worin
V für N steht;
R⁶ für -C(=O)-R²⁸ oder für einen Rest ausgewählt aus der Gruppe bestehend aus Methyl, -CH₂-CN, Ethyl, -CH₂-CH₂-CN, n-Propyl, -CH₂-CH₂-CH₂-CN, n-Butyl, -CH₂-CH₂-CH₂-CHᵣCN, sek-Butyl, Isobutyl, tert-Butyl, n-Pentyl, 3-Pentyl, n-Heptyl, Ethenyl, Propenyl, 2-Butenyl, 3-Butenyl, 2-Pentenyl und 3-Pentenyl steht;
R⁹ für F; Cl; Br, I; -SF₅; -O-CF₃; -O-CCl₃; -O-CBr₃, -O-CHF₂; -O-CH₂F; -O-CF₂CI; - O-CCl₂F; -O-CF₂-CH₃, -S-CF₃; -S-CCl₃; -S-CBr₃; -S-CHF₂, -S-CH₂F; -S-CF₂Cl; -S-CCl₂F; -S-CF₂-CH₃; oder für einen Rest ausgewählt aus der Gruppe bestehend aus Methyl, -CF₃, -CHF₂, -CH₂F, Ethyl, n-Propyl, Isopropyl, n-Butyl, sek-Butyl, Isobutyl, -C(CH₃)₂(CH₂OH) und tert-Butyl steht;
R²⁸ für einen Rest ausgewählt aus der Gruppe bestehend aus Methyl, Ethyl, n-Propyl, Isopropyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, Ethenyl, Propenyl, 2-Butenyl, 3-Butenyl, 2-Pentenyl und 3-Pentenyl steht;
R³⁴ und R³⁵, unabhängig voneinander, jeweils
für einen Rest ausgewählt aus der Gruppe bestehend aus Methyl, -CH₂-O-CH₃, Ethyl, n-Propyl, n-Butyl, sek-Butyl, Isobutyl, tert-Butyl, n-Pentyl, 3-Pentyl, n-Hexyl, (3,3)-Dimethylbutyl, -CH₂-CH₂-O-CH₃, -CH₂-CH₂-O-C₂H₅ und -CH₂-CH₂-CH₂-O-CH₃ stehen;
für einen Rest ausgewählt aus der Gruppe bestehend aus 2,3-Dihydro-1 H-indenyl, Cyclopropyl, Oxetanyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, Isobutyl, tert-Butyl, n-Pentyl substituiert sein kann, stehen;
oder
R³⁴ und R³⁵ jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, Piperazinyl, Morpholinyl, Azepanyl, Diazepanyl und Thiomorpholinyl bilden, dessen heterocycloaliphatischer Anteil jeweils unsubstituiert oder mit 1, 2, 3, 4 oder 5 Resten R⁵¹ substituiert sein kann;
R⁵¹ für -NHR⁵², -NR⁵³R⁵⁴ oder für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
R⁵², R⁵³ und R⁵⁴, unabhängig voneinander, jeweils für -C(=O)-R⁵⁵; für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl;
oder für einen Rest ausgewählt aus der Gruppe bestehend aus Phenyl und Naphthyl, wobei der Rest jeweils über eine -(CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -O-CH(CH₃)₂, -O-C(CH₃)₃, Methyl, Ethyl, n-Propyl, Isopropyl, n-Butyl, sek-Butyl, Isobutyl, tert-Butyl und n-Pentyl substituiert sein kann, stehen;
und
R⁵⁵ für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.
Ganz besonders bevorzugt sind Verbindungen der vorstehend angegebenen allgemeinen Formel Ia,
R⁶ für -C(=O)-R²⁸ oder für einen Rest ausgewählt aus der Gruppe bestehend aus Methyl, -CH₂-CN, Ethyl, -CH₂-CH₂-CN, n-Propyl, -CH₂-CH₂-CH₂-CN, n-Butyl, -CH₂-CH₂-CH₂-CH₂-CN, sek-Butyl, Isobutyl, tert-Butyl und n-Pentyl steht;
R⁹ für -SF₅; -O-CF₃; -CF₃; -CHF₂; -CH₂F; -C(CH₃)₂(CH₂OH) oder tert-Butyl steht;
R²⁸ für einen Rest ausgewählt aus der Gruppe bestehend aus Methyl, Ethyl, n-Propyl, Isopropyl, sek-Butyl, Isobutyl, tert-Butyl, Ethenyl und Propenyl steht;
R³⁴ und R³⁵ jeweils zusammen mit dem sie verbindenden Stickstoffatom als Ringglied einen Rest ausgewählt aus der Gruppe bestehend Pyrrolidinyl, Piperidinyl, Piperazinyl, Morpholinyl und Azepanyl bilden, dessen heterocycloaliphatischer Anteil jeweils unsubstituiert oder mit 1, 2, 3, 4 oder 5 Resten R⁵¹ substituiert sein kann;
R⁵¹ für -NHR⁵², -NR⁵³R⁵⁴ oder für einen Alkyl-Rest ausgewählt aus der Gruppe bestehend aus Methyl, Ethyl, n-Propyl, Isopropyl, tert-Butyl, n-Butyl, sek-Butyl und Isobutyl steht;
R⁵², R⁵³ und R⁵⁴, unabhängig voneinander, jeweils für -C(=O)-R⁵⁵; für einen Alkyl-Rest ausgewählt aus der Gruppe bestehend aus Methyl, Ethyl, n-Propyl, Isopropyl, tert-Butyl, n-Butyl, sek-Butyl und Isobutyl;
oder für Phenyl-Rest stehen, wobei der Rest mit 1, 2, 3, 4 oder 5 Substituenten unabhängig voneinander ausgewählt aus der Gruppe bestehend aus F, Cl, Br, Methyl, Ethyl, n-Propyl und Isopropyl sein kann, stehen;
und R⁵⁵ für einen Alkyl-Rest ausgewählt aus der Gruppe bestehend aus Methyl, Ethyl, n-Propyl, Isopropyl, tert-Butyl, n-Butyl, sek-Butyl und Isobutyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel Ib, 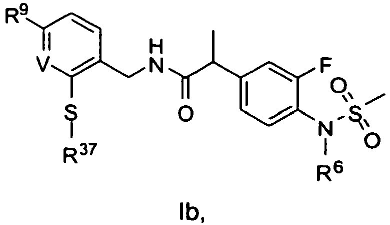 worin
R⁶ für -C(=O)-R²⁸ oder für einen Rest ausgewählt aus der Gruppe bestehend aus Methyl, -CH₂-CN, Ethyl, -CH₂-CH₂-CN, n-Propyl, -CH₂-CH₂-CH₂-CN, n-Butyl, - CH₂-CH₂-CH₂-CH₂-CN, sek-Butyl, Isobutyl, tert-Butyl, n-Pentyl, 3-Pentyl, n-Heptyl, Ethenyl, Propenyl, 2-Butenyl, 3-Butenyl, 2-Pentenyl und 3-Pentenyl steht;
R⁹ für F; Cl; Br; I; -SF₅; -O-CF₃; -O-CCl₃; -O-CBr₃; -O-CHF₂; -O-CH₂F; -O-CF₂Cl; - O-CCl₂F; -O-CF₂-CH₃; -S-CF₃; -S-CCl₃; -S-CBr₃; -S-CHF₂; -S-CH₂F; -S-CF₂Cl; -S-CCl₂F; -S-CF₂-CH₃; oder für einen Rest ausgewählt aus der Gruppe bestehend aus Methyl, -CF₃, -CHF₂, -CH₂F, Ethyl, n-Propyl, Isopropyl, n-Butyl, sek-Butyl, Isobutyl, -C(CH₃)₂(CH₂OH) und tert-Butyl steht;
R²⁸ für einen Rest ausgewählt aus der Gruppe bestehend aus Methyl, Ethyl, n-Propyl, Isopropyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, Ethenyl, Propenyl, 2-Butenyl, 3-Butenyl, 2-Pentenyl und 3-Pentenyl steht;
und
R³⁷ für einen Rest ausgewählt aus der Gruppe bestehend aus Methyl, -CH₂-O-CH₃, Ethyl, n-Propyl, n-Butyl, sek-Butyl, Isobutyl, tert-Butyl, n-Pentyl, 3-Pentyl, n-Hexyl, (3,3)-Dimethylbutyl, -CH₂-CH₂-O-CH₃, -CH₂-CH₂-O-C₂H₅ und -CH₂-CH₂-CH₂-O-CH₃ stehen;
oder für einen Rest ausgewählt aus der Gruppe bestehend aus 2,3-Dihydro-1H-indenyl, Cyclopropyl, Oxetanyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, Isobutyl, tert-Butyl, n-Pentyl substituiert sein kann, steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind Verbindungen der vorstehend angegebenen allgemeinen Formel Ib,
worin
R⁶ für-C(=O)-R²⁸ oder für einen Rest ausgewählt aus der Gruppe bestehend aus Methyl, -CH₂-CN, Ethyl, -CH₂-CH₂-CN, n-Propyl, -CH₂-CH₂-CH₂-CN, n-Butyl, - CH₂-CH₂-CH₂-CH₂-CN, sek-Butyl, Isobutyl, tert-Butyl und n-Pentyl steht;
R⁹ für -SF₅; -O-CF₃; -CF₃; -CHF₂; -CH₂F; -C(CH₃)₂(CH₂OH) oder tert-Butyl steht;
R²⁸ für einen Rest ausgewählt aus der Gruppe bestehend aus Methyl, Ethyl, n-Propyl, Isopropyl, sek-Butyl, Isobutyl, tert-Butyl, Ethenyl und Propenyl steht;
und
R³⁷ für einen Rest ausgewählt aus der Gruppe bestehend aus Cyclopropyl, Cyclobutyl, Cyclopentyl, Cyclohexyl und Cycloheptyl, der jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, Isobutyl, tert-Butyl, n-Pentyl substituiert sein kann, steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer bevorzugter Gegenstand der vorliegenden Erfindung sind Verbindungen der allgemeinen Formel I' und I'' 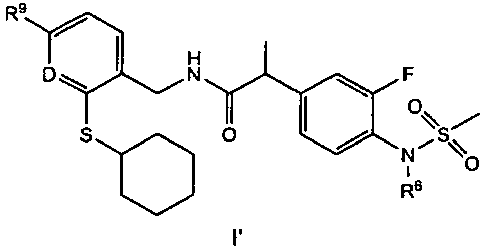 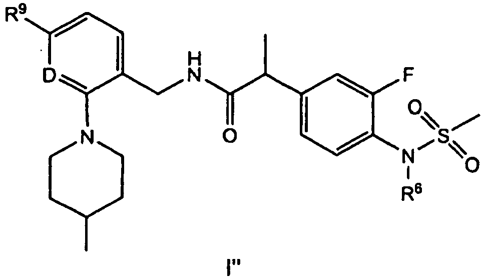 worin jeweils
D für N steht
R⁹ für CF₃ oder für tert-Butyl steht
R⁶ für Methyl, Ethyl, -C(=O)-CH₃ oder -C(=O)-CH₂CH₃ steht,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Noch weiter bevorzugt sind Verbindungen der allgemeinen Formeln I, la, Ib, I' und I'' ausgewählt aus der Gruppe bestehend aus
[1] N-(2-Fluor-4-(1-oxo-1-((2-(4-(N-phenylpropionamido)piperidin-1-yl)-6-(trifluormethyl)pyridin-3-yl)methylamino)propan-2-yl)phenyl)-N-(methylsulfonyl)propionamid,
[2] 2-(3-Fluor-4-(N-(methylsulfonyl)acetamido)phenyl)-N-((2-(4-methylpiperidin-1-yl)-6-(trifluormethyl)pyridin-3-yl)methyl)propanamid,
[3] 2-(3-Fluor-4-(N-methylmethylsulfonamido)phenyl)-N-((2-(4-methylpiperidin-1-yl)-6-(trifluormethyl)pyridin-3-yl)methyl)propanamid,
[7] N-((2-(Cyclohexylthio)-6-(trifluormethyl)pyridin-3-yl)methyl)-2-(3-fluor-4-(N-methylmethylsulfonamido)phenyl)propanamid,
[8] N-((2-(Cyclohexylthio)-6-(trifluormethyl)pyridin-3-yl)methyl)-2-(4-(N-ethylmethylsulfonamido)-3-fluorphenyl)propanamid,
[9] 2-(4-(N-Ethylmethylsulfonamido)-3-fluorphenyl)-N-((2-(4-methylpiperidin-1-yl)-6-(trifluormethyl)pyridin-3-yl)methyl)propanamid,
[10] N-((6-tert-butyl-2-(4-methylpiperidin-1-yl)pyridin-3-yl)methyl)-2-(3-fluor-4-(N-methylmethylsulfonamido)phenyl)propanamid,
[11] N-((6-tert-Butyl-2-(cyclohexylthio)pyridin-3-yl)methyl)-2-(3-fluor-4-(N-methylmethylsulfonamido)phenyl)propanamid,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin können erfindungsgemäße Verbindungen der allgemeinen Formeln I, la, Ib, I' und I'' bevorzugt sein, die im FLIPR-Assay mit CHO K1-Zellen, die mit dem menschlichen VR1-Gen transfiziert wurden, in einer Konzentration kleiner 2000 nM, bevorzugt kleiner 1000 nM, besonders bevorzugt kleiner 300 nM, ganz besonders bevorzugt kleiner 100 nM, noch weiter bevorzugt kleiner 75 nM, darüber hinaus bevorzugt kleiner 50 nM, am weitesten bevorzugt kleiner 10 nM, eine 50-prozentige Verdrängung von Capsaicin bewirken, dass in einer Konzentration von 100 nM vorliegt.

Dabei wird Im FLIPR-Assay der Ca²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegende Erfindung ist ein Verfahren zur Herstellung von Verbindungen der vorstehend angegebenen allgemeinen Formeln I, Ia, Ib, I' und I" gemäß dem wenigstens eine Verbindung der allgemeinen Formel II, 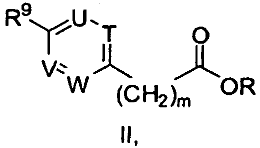 worin R⁹, U, T, V und W die vorstehend genannte Bedeutung haben, m für 0, 1, 2 oder 3 steht und R für Wasserstoff oder für einen linearen oder verzweigten C₁₋₆-Alkyl-Rest steht, in einem Reaktionsmedium, in Gegenwart wenigstens eines Reduktionsmittels, vorzugsweise in Gegenwart wenigstens eines Reduktionsmittels ausgewählt aus der Gruppe bestehend aus Natriumhydrid, Natrium, Kaliumhydrid, Lithiumaluminiumhydrid, Natriumborhydrid und Di(isobutyl)aluminiumhydrid
zu wenigstens einer Verbindung der allgemeinen Formel III, 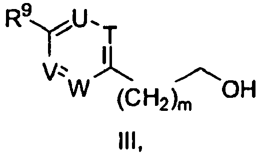 worin R⁹, U, T, V und W die vorstehend genannte Bedeutung haben und m für 0, 1, 2 oder 3 steht, umgesetzt wird und diese ggf. gereinigt und/oder isoliert wird,
und wenigstens eine Verbindung der allgemeinen Formel III in einem Reaktionsmedium in Gegenwart von Diphenylphosphorylazid oder in Gegenwart von HN₃ zu wenigstens einer Verbindung der allgemeinen Formel IV, 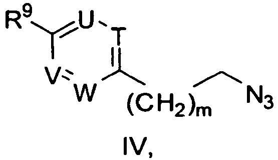 worin R⁹, U, T, V und W die vorstehend genannte Bedeutung haben und m für 0, 1, 2 oder 3 steht, umgesetzt wird und diese ggf. gereinigt und/oder isoliert wird,
und wenigstens eine Verbindung der allgemeinen Formel IV in einem Reaktionsmedium in Gegenwart wenigstens eines Reduktionsmittels, vorzugsweise in Gegenwart wenigstens eines Reduktionsmittels ausgewählt aus der Gruppe bestehend aus Natriumhydrid, Kaliumhydrid, Lithiumaluminiumhydrid, Natriumborhydrid und Di(isobutyl)aluminiumhydrid
oder in einem Reaktionsmedium in Gegenwart eines Katalysators, vorzugsweise in Gegenwart eines Katalysators basierend auf Platin oder Palladium, besonders bevorzugt in Gegenwart von Palladium auf Kohle, und in Gegenwart von Wasserstoff oder in Gegenwart von Hydrazin
oder in einem Reaktionsmedium in Gegenwart von Triphenylphosphin zu wenigstens einer Verbindung der allgemeinen Formel V, 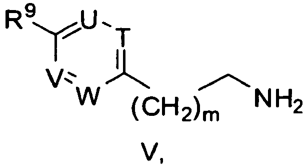 worin R⁹, U, T, V und W die vorstehend genannte Bedeutung haben und m für 0, 1, 2 oder 3 steht, umgesetzt wird und diese ggf. gereinigt und/oder isoliert wird,
oder wenigstens eine Verbindung der allgemeinen Formel VI, 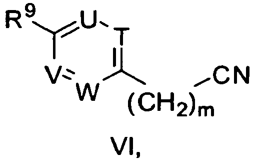 worin R⁹, U, T, V und W die vorstehend genannte Bedeutung haben und m für 0, 1, 2 oder 3 steht, in einem Reaktionsmedium
in Gegenwart wenigstens eines Katalysators, vorzugsweise in Gegenwart wenigstens eines Katalysators basierend auf Palladium oder Platin, besonders bevorzugt in Gegenwart von Palladium auf Kohle, unter einer Wasserstoffatmosphäre, ggf. in Gegenwart wenigstens einer Säure, vorzugsweise in Gegenwart von Salzsäure,
oder in Gegenwart wenigstens eines Reduktionsmittels ausgewählt aus der Gruppe bestehend aus BH₃·S(CH₃)₂, Lithiumaluminiumhydrid und Natriumborhydrid, ggf. in Gegenwart von NiCl₂,
zu wenigstens einer Verbindung der allgemeinen Formel V, ggf. in Form eines entsprechenden Salzes, vorzugsweise in Form eines entsprechenden Hydrochlorids, umgesetzt wird und diese ggf. gereinigt und/oder isoliert wird,
und wenigstens eine Verbindung der allgemeinen Formel V mit wenigstens einer Verbindung der allgemeinen Formel VII, 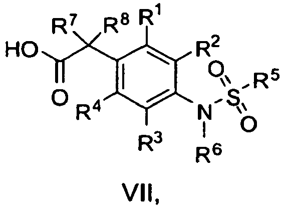 worin R¹, R², R³, R⁴, R⁵, R⁶, R⁷ und R⁸ die vorstehend genannte Bedeutung haben und R⁶ zusätzlich für einen Wasserstoff-Rest stehen kann, in einem Reaktionsmedium, ggf. in Gegenwart wenigstens eines geeigneten Kopplungsmittels, ggf. in Gegenwart wenigstens einer Base,
oder mit wenigstens einer Verbindung der allgemeinen Formel VIII, 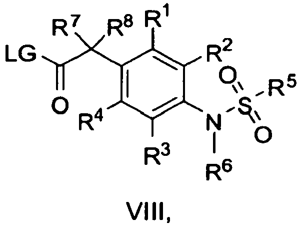 worin R¹, R², R³, R⁴, R⁵, R⁶, R⁷ und R⁸ die vorstehend genannte Bedeutung haben, R⁶ zusätzlich für einen Wasserstoff-Rest stehen kann und LG für eine Abgangsgruppe, vorzugsweise für ein Chlor- oder Bromatom steht, in einem Reaktionsmedium, ggf. in Gegenwart wenigstens einer Base, zu wenigstens einer Verbindung der allgemeinen Formel Ih, 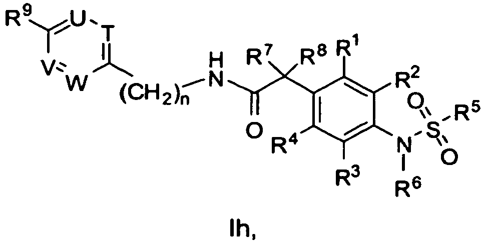 worin T, U, V, W, R¹, R², R³, R⁴, R⁵, R⁶, R⁷, R⁸ und R⁹, die vorstehend genannte Bedeutung haben, R⁶ zusätzlich für einen Wasserstoff-Rest stehen kann und n für 1, 2, 3 oder 4 steht, umgesetzt wird und diese ggf. gereinigt und/oder isoliert wird,
und ggf. wenigstens eine Verbindung der allgemeinen Formel Ih in einem Reaktionsmedium mit wenigstens einer Verbindung der allgemeinen Formel IX, 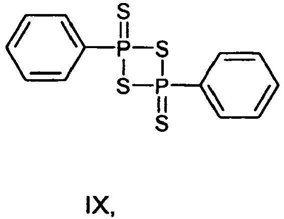 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel Ik, 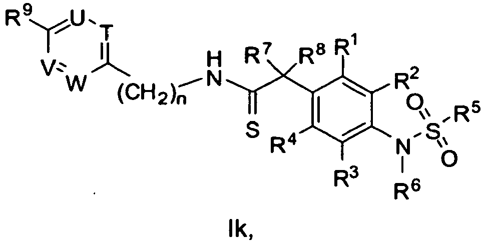 worin T, U, V, W, R¹, R², R³, R⁴, R⁵, R⁶, R⁷, R⁸ und R⁹, die vorstehend genannte Bedeutung haben, R⁶ zusätzlich für einen Wasserstoff-Rest stehen kann und n für 1, 2, 3 oder 4 steht, umgesetzt wird und diese ggf. gereinigt und/oder isoliert wird;
und ggf. wenigstens eine Verbindung der allgemeinen Formel Io, 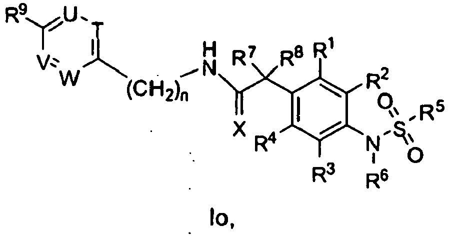 worin X, T, U, V, W, R¹, R², R³, R⁴, R⁵, R⁷, R⁸ und R⁹, die vorstehend genannte Bedeutung haben, R⁶ für einen Wasserstoff-Rest steht und n für 1, 2, 3 oder 4 steht, in einem Reaktionsmedium in Gegenwart wenigstens einer Verbindung der allgemeinen Formel R²⁸-C(=O)-O-C(=O)-R²⁸, worin R²⁸ die vorstehend genannte Bedeutung hat, zu wenigstens einer Verbindung der allgemeinen Formel I, worin X, T, U, V, W, R¹, R², R³, R⁴, R⁵, R⁷, R⁸ und R⁹ die vorstehend genannte Bedeutung haben, R⁶ für -C(=O)-R²⁸ und n für 1, 2, 3 oder 4 steht, umgesetzt wird und diese ggf. gereinigt und/oder isoliert wird.

Ein weiterer Gegenstand der vorliegenden Erfindung ist ein Verfahren zur Herstellung von Verbindungen der vorstehend angegebenen allgemeinen Formeln I, la, Ib, I' und I" gemäß dem wenigstens eine Verbindung der allgemeinen Formel X, 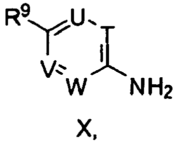 worin R⁹, U, T, V und W die vorstehend genannte Bedeutung haben, mit wenigstens einer Verbindung der allgemeinen Formel VII, 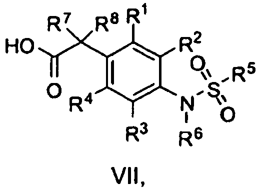 worin R¹, R², R³, R⁴, R⁵, R⁶, R⁷ und R⁸, die vorstehend genannte Bedeutung haben und R⁶ zusätzlich für einen Wasserstoff-Rest stehen kann, in einem Reaktionsmedium, ggf. in Gegenwart wenigstens eines geeigneten Kopplungsmittels, ggf. in Gegenwart wenigstens einer Base,
oder mit wenigstens einer Verbindung der allgemeinen Formel VIII, 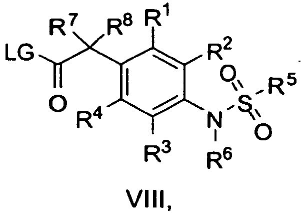 worin R¹, R², R³, R⁴, R⁵, R⁶, R⁷ und R⁸ die vorstehend genannte Bedeutung haben, R⁶ zusätzlich für einen Wasserstoff-Rest stehen kann und LG für eine Abgangsgruppe, vorzugsweise für ein Chlor- oder Bromatom steht, in einem Reaktionsmedium, ggf. in Gegenwart wenigstens einer Base, zu wenigstens einer Verbindung der allgemeinen Formel Im, 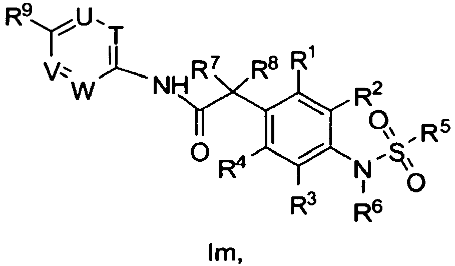 worin T, U, V, W, R¹, R², R³, R⁴, R⁵, R⁷, R⁸ und R⁹ die vorstehend genannte Bedeutung haben, umgesetzt wird und diese ggf. gereinigt und/oder isoliert wird,
und ggf. wenigstens eine Verbindung der allgemeinen Formel Im in einem Reaktionsmedium mit wenigstens einer Verbindung der allgemeinen Formel IX, 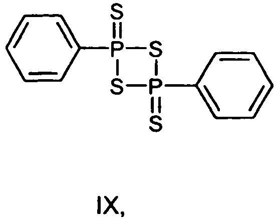 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel In, 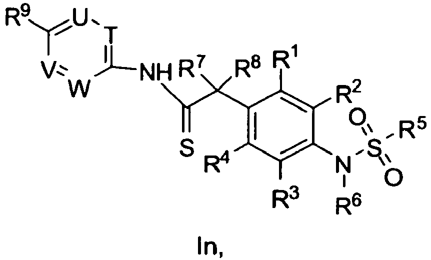 worin T, U, V, W, R¹, R², R³, R⁴, R⁵, R⁶, R⁷, R⁸ und R⁹ die vorstehend genannte Bedeutung haben und R⁶ zusätzlich für einen Wasserstoff-Rest stehen kann, umgesetzt wird und diese ggf. gereinigt und/oder isoliert wird;
und ggf. wenigstens eine Verbindung der allgemeinen Formel Ip, 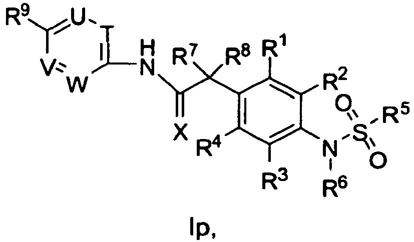 worin X, T, U, V, W, R¹, R², R³, R⁴, R⁵, R⁷, R⁸ und R⁹, die vorstehend genannte Bedeutung haben und R⁶ für einen Wasserstoff-Rest steht, in einem Reaktionsmedium in Gegenwart wenigstens einer Verbindung der allgemeinen Formel R²⁸-C(=O)-O-C(=O)-R²⁸, worin R²⁸ die vorstehend genannte Bedeutung hat, zu wenigstens einer Verbindung der allgemeinen Formel I, worin X, T, U, V, W, R¹, R², R³, R⁴, R⁵, R⁷, R⁸ und R⁹ die vorstehend genannte Bedeutung haben und R⁶ für -C(=O)-R²⁸ steht, umgesetzt wird und diese ggf. gereinigt und/oder isoliert wird.

Die Umsetzung von Verbindungen der vorstehend angegebenen allgemeinen Formeln V bzw. X mit Carbonsäuren der vorstehend angegebenen allgemeinen Formel VII zu Verbindungen der vorstehend angegebenen allgemeinen Formeln Ih bzw. Im erfolgt vorzugsweise in einem Reaktionsmedium ausgewählt aus der Gruppe bestehend aus Diethylether, Tetrahydrofuran, Acetonitril, Methanol, Ethanol, (1,2)-Dichlorethan, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorphosphat (BOP), Dicyclohexylcarbodiimid (DCC), N'-(3-Dimethylaminopropyl)-N-ethylcarbodiimid (EDCI), Diisoproylcarbodiimid, 1,1'-Carbonyl-diimidazol (CDI), N-[(Dimethyamino)-1 H-1, 2, 3-triazolo[4, 5-b]pyridino-1-ylmethylen]-N-methylmethanaminium hexafluorphosphat N-oxid (HATU), 0-(Benzotriazol-1-yl)-N,N,N',N'-tetramethyluroniom hexafluorphosphat (HBTU), 0-(Benzotriazol-1-yl)-N,N,N',N'-tetramethyluronium-tetrafluorborat (TBTU) und 1-Hydroxy-7-azabenzotriazol (HOAt), ggf. in Gegenwart wenigstens einer organischen Base, vorzugsweise ausgewählt aus der Gruppe bestehend aus Triethylamin, Pyridin, Dimethylaminopyridin, N-Methylmorpholin und Diisopropylethylamin, vorzugsweise bei Temperaturen von -70°C bis 100°C.

Alternativ erfolgt die Umsetzung von Verbindungen vorstehend angegebenen allgemeinen Formeln V bzw. X mit Carbonsäurederivaten der vorstehend angegebenen allgemeinen Formel VIII, worin LG für eine Abgangsgruppe, bevorzugt für ein Chlor- oder Bromatom, steht, zu Verbindungen der vorstehend angegebenen allgemeinen Formeln Ih bzw. Im in einem Reaktionsmedium vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart einer organischen oder anorganischen Base, vorzugsweise ausgewählt aus der Gruppe bestehend aus Triethylamin, Dimethylaminopyridin, Pyridin und Diisopropylamin, bei Temperaturen von -70°C bis 100°C.

Die Umsetzung von Verbindungen der allgemeinen Formeln Ih bzw. Im zu Verbindungen der allgemeinen Formeln Ik bzw. In erfolgt vorzugsweise in einem Reaktionsmedium ausgewählt aus der Gruppe bestehend aus Toluol, para-Xylol, ortho-Xylol, meta-Xylol, Acetonitril, Dichlormethan, Dimethylformamid und Mischungen der vorstehend genannten Reaktionsmedien, unter Zusatz eines Dithiaphosphetans, besonders bevorzugt unter Zusatz von 2,4-Bis(4-methoxyphenyl)-1,3,2,4-dithiadiphosphetan-2,4-disulfide (Lawesson-Reagenz), oder unter Zusatz von Phosphorpentasulfid, bei Temperaturen von 50 bis 150 °C.

Die Verbindungen der vorstehend angegebenen Formeln II, III, IV, V, VI, VIII, IX und X sind jeweils am Markt käuflich erhältlich und können auch nach üblichen, dem Fachmann bekannten Verfahren hergestellt werden.

Die Synthese von Verbindungen der allgemeinen Formel VII kann der Druckschrift "4-(Methylsulfonylamino)phenyl analogues as vanilloid antagonist showing excellent analgesic activity and the pharmaceutical compositions comprising the same" von J. W. Lee et al. [WO 2005/003084-A1] entnommen werden. Die entsprechenden Teile der Referenz gelten hiermit als Teil der Offenbarung.

Die vorstehend beschriebenen Umsetzungen können jeweils unter den üblichen dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie. Sämtliche der vorstehend beschriebenen Verfahrensschritte sowie jeweils auch die Reinigung und/oder Isolierung von Zwischen- oder Endprodukten können teilweise oder vollständig unter einer Inertgasatmosphäre, vorzugsweise unter Stickstoffatmosphäre, durchgeführt werden.

Bevorzugte Verbindungen der allgemeinen Formeln I, la, Ib, I' und I" können jeweils als (S)-Enantiomer vorliegen. Beispielhaft ist im Folgenden das (S)-Enantiomer von Verbindungen der allgemeinen Formel Ia angeben.

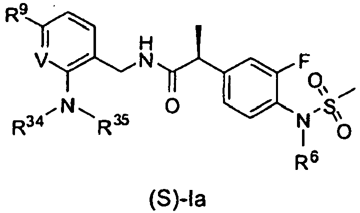

Die erfindungsgemäßen substituierten Verbindungen der vorstehend genannten allgemeinen Formeln I, Ia, Ib, I' und I" - im Folgenden nur als Verbindungen der allgemeinen Formel I - bezeichnet, sowie entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen erfindungsgemäßen substituierten Verbindungen der vorstehend genannten allgemeinen Formel I sowie entsprechender Stereoisomere; können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden. Die freien Basen der jeweiligen substituierten Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-Alkyl-Rest steht, genannt.

Die erfindungsgemäßen substituierten Verbindungen der vorstehend genannten allgemeinen Formel I und entsprechender Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1(VR1/TRPV1)-Stimulation.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Schmerz ausgewählt aus der Gruppe bestehend aus akutem Schmerz, chronischem Schmerz, neuropathischem Schmerz und visceralem Schmerz; Gelenkschmerz; Hyperalgesie; Allodynie; Kausalgie; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma, Bronchitis und Lungenentzündung; Husten; Haminkontinenz; einer überaktiven Blase (overactive bladder, OAB); Erkrankungen und/oder Verletzungen des Magen-Darm-Trakts; Zwölffingerdarmgeschwüren; Magengeschwüren; Reizdarmsyndrom; Schlaganfällen; Augenreizungen; Hautreizungen; neurotischen Hauterkrankungen; allergischen Hautkrankheiten; Psiorasis; Vitiligo; Herpes simplex; Entzündungen, vorzugsweise Entzündungen des Darmes, der Augen, der Blase, der Haut oder der Nasenschleimhaut; Diarrhöe; Pruritus; Osteoporose; Arthritis; Osteoarthritis; rheumatischen Erkrankungen; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Behandlung von Wunden und/oder Verbrennungen; zur Behandlung von durchtrennten Nerven; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Entzündungen, vorzugsweise Entzündungen des Darmes, der Augen, der Blase, der Haut oder der Nasenschleimhaut; Haminkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens einer erfindungsgemäßen Verbindung sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1(VR1/TRPV1)-Stimulation.

Bevorzugt ist die Verwendung wenigstens einer erfindungsgemäßen substituierten Verbindung sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 vermittelt werden.

Besonders bevorzugt ist die Verwendung wenigstens einer erfindungsgemäßen Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz und Gelenkschmerz.

Besonders bevorzugt ist die Verwendung wenigstens einer erfindungsgemäßen Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Hyperalgesie; Allodynie; Kausalgie; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma, Bronchitis und Lungenentzündung; Husten; Haminkontinenz; einer überaktiven Blase (overactive bladder, OAB); Erkrankungen und/oder Verletzungen des Magen-Darm-Trakts; Zwölffingerdarmgeschwüren; Magengeschwüren; Reizdarmsyndrom; Schlaganfällen; Augenreizungen; Hautreizungen; neurotischen Hauterkrankungen; allergischen Hautkrankheiten; Psiorasis; Vitiligo; Herpes simplex; Entzündungen, vorzugsweise Entzündungen des Darmes, der Augen, der Blase, der Haut oder der Nasenschleimhaut; Diarrhöe; Pruritus; Osteoporose; Arthritis; Osteoarthritis; rheumatischen Erkrankungen; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Behandlung von Wunden und/oder Verbrennungen; zur Behandlung von durchtrennten Nerven; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Ganz besonders bevorzugt ist die Verwendung wenigstens einer erfindungsgemäßen substituierten Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Entzündungen, vorzugsweise Entzündungen des Darmes, der Augen, der Blase, der Haut oder der Nasenschleimhaut; Haminkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Noch weiter bevorzugt ist die Verwendung wenigstens einer erfindungsgemäßen substituierten Verbindung sowie ggf. eines oder mehrerer pharmazeutisch verträglicher Hilfsstoffe zur Herstellung eines Arzneimittels zur Behandlung und/oder Prophylaxe von Schmerz, vorzugsweise ausgewählt aus der Gruppe bestehend aus akutem Schmerz, chronischem Schmerz, neuropathischem Schmerz und visceralem Schmerz, und/oder Harninkontinenz.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpresst, in Kapseln abgefüllt oder in einer Flüssigkeit suspendiert, vorliegen und als solche auch verabreicht werden.

Neben wenigstens einer substituierten Verbindung der vorstehend angegebenen allgemeinen Formel I, ggf. in Form eines ihrer reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die beispielsweise ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Die in dem erfindungsgemäßen Arzneimittel zum Einsatz kommenden erfindungsgemäßen substituierten Verbindungen in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die jeweilige erfindungsgemäße substituierte Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung. Die an den Patienten zu verabreichende Menge der jeweiligen erfindungsgemäßen substituierten Verbindungen der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0.001 bis 100 mg/kg, vorzugsweise 0.05 bis 75 mg/kg, besonders bevorzugt 0.05 bis 50 mg/kg, Körpergewicht des Patienten wenigstens einer solchen erfindungsgemäßen Verbindung appliziert.

### Pharmakologische Methoden

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Komplett-Medium: 50 mL HAMS F12 Nutrient Mixture (Gibco Invitrogen GmbH, Karlsruhe, Deutschland) mit
10 Vol-% FCS (fetal calf serum, Gibco Invitragen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100, µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20 °C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelganglien (DRGs; dorsal root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig ab pipettiert und die zurückgebliebenen DRGs werden jeweils mit 500 µL Komplett-Medium versetzt. Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0.01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca²⁺-Messung im FLIPR-Assay eingesetzt. Die Ca²⁺-abhängige Fluoreszenz wird dabei vor und nach Zugabe von Substanzen gemessen (λex = 488 nm, λem = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Verbindungen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

Ausgehend von der prozentualen Verdrängung durch unterschiedliche Konzentrationen der zu prüfenden Verbindungen der allgemeinen Formel wurden IC₅₀ Hemmkonzentrationen berechnet, die eine 50-prozentige Verdrängung von Capsaicin bewirken. Durch Umrechnung mittels der Cheng-Prusoff-Beziehung wurden Kᵢ-Werte für die Prüfsubstanzen erhalten (Cheng, Prusoff; Biochem. Pharmacol. 22, 3099-3108, 1973).

### II. Funktionelle Untersuchungen am Vanilloid Rezeptor (VR1)

Die agonistische bzw. antagonistische Wirkung der zu untersuchenden Substanzen auf Vanilloid Rezeptor (VR1) kann auch mit dem folgenden Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes, Europe BV, Leiden, Niederlande) im Fluorescent Imaging Plate Reader (FLIPR, Molecular Devices, Sunnyvale, USA) quantifiziert.

### Methode:

Chinese Hamster Ovary Zellen (CHO K1-Zellen, European Collection of Cell Cultures (ECACC) Großbritannien) werden stabil mit dem VR1-Gen transfiziert. Für funktionelle Untersuchungen werden diese Zellen auf Poly-D-Lysin-beschichtete, schwarze 96-Loch-Platten mit klarem Boden (BD Biosciences, Heidelberg, Deutschland) in einer Dichte von 25.000 Zellen/Loch ausplattiert. Über Nacht werden die Zellen bei 37 °C und 5 % CO₂ in einem Kulturmedium (Nutrient Mixture 'am's F12, 10 Vol-% FCS (Fetal calf serum), 18 µg/ml L-Prolin) inkubiert. Am folgenden Tag werden die Zellen mit Fluo-4 (Fluo-4 2 µM, Pluronic F127 0.01 Vol-%, Molecular Probes in HBSS (Hank's buffered saline solution), Gibco Invitrogen GmbH, Karlsruhe, Deutschland) für 30 Minuten bei 37 °C inkubiert. Anschließend werden die Platten 3 mal mit HBSS-Puffer gewaschen und nach einer weiteren Inkubation von 15 Minuten bei Raumtemperatur zur Ca²⁺-Messung im FLIPR eingesetzt. Die Ca²⁺-abhängige Fluoreszenz wird dabei vor und nach Zugabe der zu untersuchenden Substanzen gemessen (Wellenlänge λₑₓ=488 nm, λₑₘ= 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Substanzen (10µM) auf die Zellen pipettiert und der Ca²⁺-Einstrom mit der Kontrolle (Capsaicin 10 µM) verglichen (% Aktivierung bezogen auf das Ca²⁺-Signal nach Zugabe von 10 µM Capsaicin). Nach 5 Minuten Inkubation werden 100 nM Capsaicin appliziert und ebenfalls der Einstrom von Ca²⁺ ermittelt.

Desensitisierende Agonisten und Antagonisten führten zu einer Unterdrückung des Ca²⁺-Einstroms. Es werden % Inhibierung im Vergleich zu der maximal erreichbaren Inhibierung mit 10 µM Capsaicin berechnet.

Ausgehend von der prozentualen Verdrängung durch unterschiedliche Konzentrationen der zu prüfenden Verbindungen der allgemeinen Formel wurden IC₅₀ Hemmkonzentrationen berechnet, die eine 50-prozentige Verdrängung von Capsaicin bewirken. Durch Umrechnung mittels der Cheng-Prusoff-Beziehung wurden Kᵢ-Werte für die Prüfsubstanzen erhalten (Cheng, Prusoff; Biochem. Pharmacol. 22, 3099-3108, 1973).

### III. Formalin-Test an der Maus

Die Untersuchung zur Bestimmung der antinociceptiven Wirkung der erfindungsgemäßen Verbindungen wird im Formalin-Test an männlichen Mäusen (NMRI, 20 bis 30 g Körpergewicht, Iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-Injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen Verbindungen werden in der zweiten Phase des Formalin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen Verbindungen vor der Formalin-Injektion gewählt. Die intravenöse Applikation von 10 mg/kg Körpergewicht der Testsubstanzen erfolgt 5 Minuten vor der Formalin-Injektion. Diese erfolgt durch eine einmalige subkutane Formalin-Injektion (20 µL, 1 %-ige wässrige Lösung) in die dorsale Seite der rechten Hinterpfote, so dass bei frei beweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfasst. Die Quantifizierung des Schmerzverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wässrige Natriumchlorid-Lsg.) vor Formalinapplikation erhalten. Basierend auf der Quantifizierung des Schmerzverhaltens wird die Substanzwirkung im Formalin-Test als Änderung gegen die entsprechende Kontrolle in Prozent ermittelt.

Nach Injektion von Substanzen, die im Formalin-Test antinociceptiv wirksam sind, werden die beschriebenen Verhaltensweisen der Tiere, d. h. Lecken und Beißen, reduziert bzw. aufgehoben.

### IV. Untersuchung auf analgetische Wirksamkeit im Writhing-Test

Die Untersuchung der erfindungsgemäßen Verbindungen der allgemeinen Formel I auf analgetische Wirksamkeit wurde im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Verbindungsdosis erhielten 10 Minuten nach intravenöser Gabe der zu untersuchenden Verbindungen 0,3 ml/Maus einer 0.02%igen wässrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere wurden einzeln in Beobachtungskäfige gesetzt. Mit Hilfe eines Drucktastenzählers wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mit geführt, die nur physiologische Kochsalzlösung erhalten hatten. Alle Verbindungen wurden in der Standarddosierung von 10 mg/kg getestet.

### V. Hypothermie Assay an der Maus

### Methodenbeschreibung:

Der Hypothermie Assay wird in männlichen NMRI Mäusen (Gewicht 25-35 Gramm, Züchter IFFA CREDO, Brüssel, Belgien) durchgeführt. Die Tiere wurden unter standardisierten Bedingungen gehalten: Licht/Dunkel Rhythmus (6:00 bis 18:00 Uhr Licht-; 18:00 bis 6:00 Uhr Dunkelphase), Raumtemperatur 19-22°C, relative Luftfeuchte 35-70%, 15 Raumluftwechsel pro Stunde, Luftbewegung < 0.2 m/sec. Die Tiere erhielten Standardfutter (ssniff R/M-Haltung, ssniff Spezialdiäten GmbH, Soest, Germany) und Leitungswasser. Wasser und Futter wurden während des Versuches entzogen. Alle Tiere wurden nur einmal im Versuch eingesetzt. Die Tiere hatten eine Eingewöhnungsphase von mindestens 5 Tagen.

Die akute Applikation von Capsaicin (VR-1 Agonist) führt zu einem Abfall der Körperkemtemperatur in Ratten und Mäusen über eine Stimulation von Wärmesensoren. Nur spezifisch wirkende VR-1-rezeptor Antagonisten können die Capsaicin induzierte Hypothermie antagonisieren. Eine durch Morphin induzierte Hypothermie dagegen wird nicht von VR-1 Antagonisten antagonisiert. Dieses Modell ist daher geeignet, Substanzen mit VR-1 antagonistischen Eigenschaften über die Wirkung auf die Körpertemperatur zu identifizieren.

Für die Messung der Körperkemtemperatur wurde ein digitales Thermometer (Thermalert TH-5, physitemp, Clifton NJ, USA) benutzt. Der Messfühler wird dabei in das Rektum der Tiere eingeführt.

Als individueller Basiswert wird bei jedem Tier 2 mal im Abstand von ca. einer halben Stunde die Körpertemperatur gemessen. Anschließend erhält eine Gruppe von Tieren (n= 6 bis 10) eine intraperitoneale (i.p.) Applikation von Capsaicin 3mg/kg und Vehikel (Kontrollgruppe). Eine andere Gruppe von Tieren erhält die zu testende Substanz (i.v. oder p.o.) und zusätzlich Capsaicin (3mg/kg) i.p. Die Applikation der Testsubstanz erfolgt i.v. 10 min bzw. p.o. 15 Minuten vor Capsaicin. Anschließend wird 7,5/15 und 30 min nach Capsaicin( i.v. + i.p.) bzw. 15 / 30 / 60 /90 /120 min (p.o. + i.p.) nach Capsaicin die Körpertemperatur gemessen. Zusätzlich wird eine Tiergruppe nur mit der Testsubstanz behandelt sowie eine Gruppe nur mit Vehikel. Die Auswertung bzw. Darstellung der Messwerte erfolgt als Mittelwert +/- S.E.M. der absoluten Werte als Grafik. Die antagonistische Wirkung wird berechnet als Prozent Reduktion der Capsaicin-induzierten Hypothermie.

### VI. Neuropathischer Schmerz an der Maus

Die Untersuchung auf Wirksamkeit im neuropathischen Schmerz wurde im Bennett-Modell (chronic constriction injury; Bennett und Xie, 1988, Pain 33: 87-107) untersucht.

NMRI Mäuse mit einem Gewicht von 16-18g werden unter Ketavet-Rompun-Narkose mit drei losen Ligaturen des rechten nervus ischiaticus versehen. Die Tiere entwickeln an der vom Geschädigten Nerv innervierten Pfote eine Überempfindlichkeit, die nach einer Erholungsphase von einer Woche über etwa drei Wochen mittels einer 4°C kalten Metallplatte quantifiziert wird (Kälte-Allodynie). Die Tiere werden für einen Zeitraum von 2 min. auf dieser Platte beobachtet und die Anzahl der Wegziehreaktionen der geschädigten Pfote wird gemessen. Bezogen auf den Vorwert vor Substanzapplikation wird die Substanzwirkung über einen bestimmten Zeitraum an verschiedenen Zeitpunkten (z.B. 15, 30, 45, 60 min. nach Applikation) bestimmt und die resultierend Fläche unter der Kurve (AUC) und/oder die Hemmung der Kälte-Allodynie zu den einzelnen Messpunkten in Prozent Wirkung zur Vehikelkontrolle (AUC) bzw. zum Ausgangswert (Einzelmesspunkte) ausgedrückt. Die Gruppengröße beträgt n=10, die Signifikanz einer antiallodynischen Wirkung (*=p<0.05) wird anhand einer Varianzanalyse mit wiederholter Messung und einer post hoc Analyse nach Bonferroni bestimmt.

Im Folgenden wird die Erfindung mit Hilfe einiger Beispiele erläutert. Diese Erläuterungen sind lediglich beispielhaft und schränken den allgemeinen Erfindungsgedanken nicht ein.

### Beispiele:

Die Ausbeuten der hergestellten Verbindungen wurden nicht optimiert.

Alle Temperaturen sind unkorrigiert.

Die Angabe "Äquivalente" bedeutet Stoffmengenäquivalente, "RT" Raumtemperatur, "M" und "N" sind Konzentrationsangaben in mol/l, "aq." wäßrig, "ges." gesättigt, "Lsg." Lösung,

Weitere Abkürzungen:

| | |
|---|---|
| DMF | N,N-Dimethylformamid |
| EDCI | N-Ethyl-N'-(3-dimethylaminopropyl)-carbodiimid Hydrochlorid |
| EE | Essigsäureethylester |
| H₂O | Wasser |
| MeOH | Methanol |

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, Oakwood etc.) bezogen oder nach den üblichen dem Fachmann bekannten Methoden synthetisiert.

Als stationäre Phase für die Säulenchromathographie wurde Kieselgel 60 (0.0-0 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik erfolgte durch Massenspektroskopie und NMR.

### 1. Allgemeine Vorschrift zur Darstellung von Aminen der allgemeinen Formel V-A

Die Darstellung von Aminen der allgemeinen Formel V-A erfolgt wie im nachfolgenden Schema 1 dargestellt.

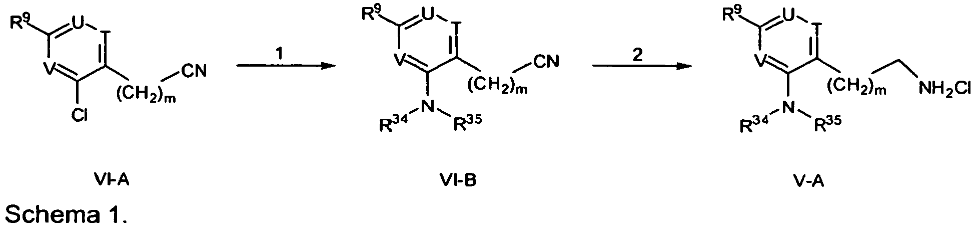

### Stufe 1: Darstellung von Nitrilen der allgemeinen Formel VI-B

### Methode A:

Verbindungen der allgemeinen Formel VI-A (1 Äquivalent), worin R⁹, U, T und V die vorstehend genannte Bedeutung haben und m für 0, 1, 2 oder 3 steht, werden mit einem Amin der allgemeinen Formel HNR³⁴R³⁵ (6 Äquivalente) 48 Stunden bei RT gerührt. Die Reaktionsmischung wird mit 1 N Salzsäure versetzt und mehrfach mit EE extrahiert. Die wässrige Phase wird mit NaCl gesättigt und anschließend wiederum mit EE extrahiert. Die vereinigten organischen Phasen werden mit 1 N Salzsäure und mit ges. aq. NaCl-Lsg. gewaschen, über MgSO₄ getrocknet und das Lösungsmittel wurde im Vakuum entfernt.

### Methode B:

Verbindungen der allgemeinen Formel VI-A (1 Äquivalent), worin R⁹, U, T und V die vorstehend genannte Bedeutung haben und m für 0, 1, 2 oder 3 steht, werden mit einem Amin der allgemeinen Formel HNR³⁴R³⁵ (2 Äquivalente) und DBU [1,8-Diaza-bicyclo[5.4.0]undec-7-en] (2 Äquivalente) in Acetonitril (7 ml pro mmol der Verbindung der Formel VI-A) 12 Stunden bei RT gerührt. Die Reaktionsmischung wird mehrfach mit EE extrahiert. Die vereinigten organischen Phasen werden mit ges. aq. NaCl-Lsg. gewaschen, über MgSO₄ getrocknet und das Lösungsmittel wurde im Vakuum entfernt. Der Rückstand wird jeweils durch Säulenchromatographie (SiO₂, verschiedene Mischungen Hexan/EE) gereinigt.

Die folgenden Verbindungen wurden gemäß Methode B. hergestellt.

### 6-(Trifluormethyl)-2-(4-methylpiperidin-1-yl)pyridin-3-carbonitril

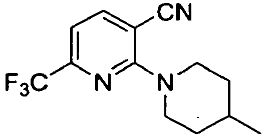

¹H NMR (300 MHz, CDCl₃) δ 7.87 (d, 1 H, J = 7.8 Hz), 6.95 (d, 1 H, *J* = 7.8 Hz), 4.53 (m, 2H), 3.05 (m, 2 H), 1.78 (m, 2 H), 1.64 (m, 1 H), 1.29 (m, 2 H), 1.00 (d, 3 H, J = 6.6 Hz); IR (pur) 2926, 2852, 2218, 1590, 1497, 1456, 1324, 1237, 1186, 1147, 1082, 963 cm⁻¹; MS (FAB) *m*/*z* 270 (M+H)

### N-(3'-Cyano-6'-trifluormethyl-3,4,5,6-tetrahydro-2H-[1,2']bipyridinyl-4-yl)-N-phenyl-propionamid

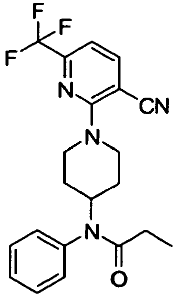

¹H NMR (300 MHz, CDCl₃) δ 7.85 (d, 1 H, J = 7.8 Hz), 7.41 (m, 3 H), 7.11 (m, 2 H), 6.95 (d, 2 H, J = 7.8 Hz), 4.96 (m, 1 H), 4.61 (m, 2 H), 3.14 (m, 2 H), 1.96 (m, 4 H), 1.46 (m, 2 H), 1.03 (t, 3 H, J = 7.5 Hz); MS (FAB) m/z 403(M+H)

### Stufe 2:

### Methode 1

Verbindungen der allgemeinen Formel VI-B (5 mmol), worin R⁹, R³⁴, R³⁵, U, T und V die vorstehend genannte Bedeutung haben und m für 0, 1, 2 oder 3 steht, Palladium auf Kohle (10 %, 500 mg) und konzentrierte Salzsäure (3 mL) werden in MeOH (30 mL) gelöst und 6 Stunden bei RT einer Wasserstoffatmosphäre ausgesetzt. Die Reaktionsmischung wird über Celite filtriert und das Filtrat wird im Vakuum eingeengt. Der Rückstand wird mittels Flashchromatographie (SiO₂, EE) gereinigt.

### Methode 2:

Verbindungen der allgemeinen Formel-VI-B (2 mmol), worin R⁹, R³⁴, R³⁵, U, T und V die vorstehend genannte Bedeutung haben und m für 0, 1, 2 oder 3 steht, werden in THF (10 mL, 10 mL) gelöst und BH₃·S(CH₃)₂ [2.0 M in THF, 3 mL, 3 Äquivalent] wird hinzu gegeben. Die Reaktionsmischung wird 8 Stunden zum Rückfluss erhitzt, aq. HCl (2 N) wird hinzu gegeben und die Reaktionsmischung wird erneut für 30 Minuten zum Rückfluss erhitzt. Die Reaktionsmischung wird mit aq. Natriumhydroxid-Lösung (2N) versetzt und mit EE gewaschen. Die vereinigten organischen Phasen werden mit ges. aq. NaCl-Lösung gewaschen und über Magnesiumsulfat getrocknet. Das Lösungsmittel wird im Vakuum entfernt und der Rückstand säulenchromatographisch (SiO₂, verschiedene Mischung aus Dichlormethan und Methanol als Laufmittel) gereinigt.

Die folgenden Verbindungen wurden gemäß Methode 2. erhalten.

### (6-(Trifluormethyl)-2-(4-methylpiperidin-1-yl)pyridin-3-yl)methanamin

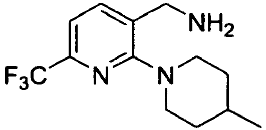

¹H NMR (300 MHz, CDCl₃) δ 7.89 (d, 1 H, J = 7.8 Hz), 7.33 (d, 1 H, J = 7.8 Hz), 3.88 (s, 2H), 3.39 (m, 2 H), 2.83 (m, 2 H), 1.75 (m, 2 H), 1.55 (m, 1 H), 1.38 (m, 2 H), 1.00 (d, 3 H, J = 6.6 Hz); MS (FAB) *m*/*z* 274(M+H)

### 3'-Aminomethyl-4-phenyl-6'-trifluormethyl-3,4,5,6-tetrahydro-2H-[1,2']bipyridinyl-4-carbonsäureethylester

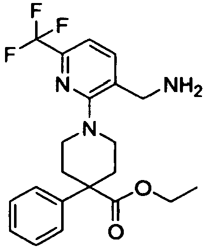

¹H NMR (300 MHz, CDCl₃) δ 7.83 (d, 1 H, J = 7.5 Hz), 7.45 (m, 2 H), 7.35 (m, 3 H), 7.26 (d, 1 H, J = 8.1 Hz), 4.15 (q, 2 H, J= 7.2 Hz), 4.03 (s, 2 H), 3.47 (m, 2 H), 3.08 (m, 2 H), 2.69 (m, 2 H), 2.10 (m, 2 H), 1.21 (t, 3 H, J = 7.2 Hz); MS (FAB) m/z 408 (M+H)

### 2. Allgemeine Vorschrift zur Darstellung von Aminen der allgemeinen Formel V-E

Die Darstellung von Aminen der allgemeinen Formel V-E erfolgt wie im nachfolgenden Schema 2 dargestellt.

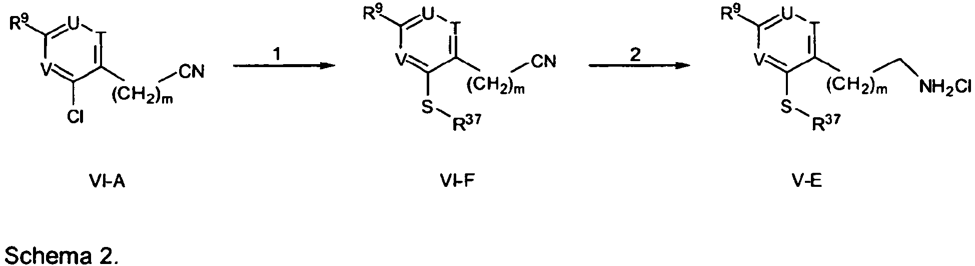

### Stufe 1:

### Synthese von 2-(Cyclohexylthio)-6-(trifluormethyl)nicotinnitril

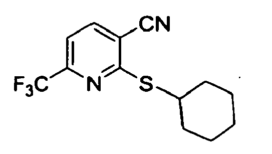

1.3 Äquivalente NaH (4.9 g, 0.124 mol) wurden unter Stickstoff-Atmosphäre in 50 ml DMF gelöst. Nach Zugabe von 1.2 Äquivalenten Cyclohexanthiol (14.2 ml, 0.116 mol) wurde bei Raumtemperatur 1,5 h gerührt. Die entstandene Suspension wurde auf 10 °C abgekühlt und tropfenweise 1 Äquivalent 2-Chlor-6-(trifluormethyl)nicotinnitril (20 g, 0.096 mol) in 50 ml DMF zugefügt und 2 h bei Raumtemperatur gerührt. Das Reaktionsgemisch wurde mit ges. aq. NH₄Cl-Lsg, gequencht, mit 1 I Wasser verdünnt und mehrfach mit EE (3 x 200 ml) extrahiert. Die vereinigten organischen Phasen wurden mit ges. aq. NaCl-Lsg. gewaschen, über MgSO₄ getrocknet und im Vakuum eingeengt. Die säulenchromatographische Reinigung (Slilicagel. 100-200 mesh, Eluent: 2 % EE in Hexan) erbrachte 26 g (93.8 %) Produkt.
¹H NMR (300 MHz, CDCl₃) δ 7.94 (d ,1 H, *J* = 7.9 Hz), 7.34 (d, 1 H, *J* = 7.9 Hz), 4.00 (m, 1 H),1.90-2.14 (m, 2 H), 1.42-1.88 (m, 8 H)
IR (neat) 2930, 2854, 2232, 1643, 1573, 1447, 1334, 1245, 1186, 1149, 1107, 851 cm⁻¹
MS (FAB) *m*/*z* 287 (M+H)

### Stufe 2:

### Synthese von (2-(Cyclohexylthio)-6-(trifluormethyl)pyridin-3-yl)methanamin dihydrochlorid

Das Nitril (26 g, 0.091 mol) wurde unter Stickstoff-Atmosphäre in 600 ml THF gelöst und auf 5 °C abgekühlt. Tropfenweise wurde BH₃-DMS (13.78 g, 0.182 mol) zugefügt und 20 h unter Rückfluß gekocht. Nach Abkühlung auf 5 °C wurde der Reaktionsansatz mit 100 ml MeOH gequencht und 15 Minuten bei Raumtemperatur gerührt. Dann wurde Di-tert.Butyl-dicarbonat (29.7 g, 0.136 mol) zugegeben und 30 min bei Raumtemperatur gerührt. Nach Entfernen des Lösungsmittels im Vakuum wurde das Rohprodukt säulenchromatographisch (Silicagel. 100-200 mesh, Eluent: 10 % EE in Hexan) gereinigt und 23.4 g (66 %) Produkt erhalten.

Das Rohprodukt wurde in 120 ml ges. HCl-Dioxan-Lsg. gelöst und 6 h bei Raumtemperatur gerührt. Nach Entfernen des Lösungsmittels im Vakuum wurde der Feststoff mit 10 % EE in Hexan (2 x 100 ml) gewaschen und abfiltriert.
Ausbeute: 17 g (88.8 %)
¹H NMR (DMSO-d₆, 400 MHz): δ 8.8 (s,2H), 8.05(d,1H), 7.76 (d,1H), 4.01 (s, 1H), 3.86-3.93 (m,1 H), 2.02-2.08 (m,2H), 1.71-1.74 (m,2H), 1.40-1.60 (m,6H).

### 3. Allgemeine Vorschrift zur Darstellung von Aminen der allgemeinen Formel V-B

Die Darstellung von Aminen der allgemeinen Formel V-B erfolgt wie im nachfolgenden Schema 3 dargestellt.

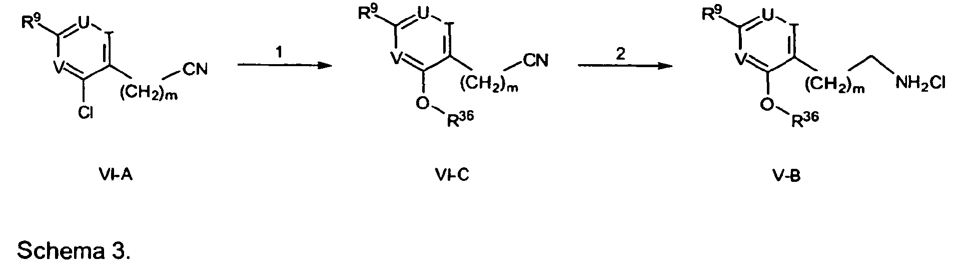

### Stufe 1: Darstellung von Nitrilen der allgemeinen Formel VI-C

Verbindungen der allgemeinen Formel VI-A (1 Äquivalent), worin R⁹, U, T und V die vorstehend genannte Bedeutung haben und m für 0, 1, 2 oder 3 steht, werden mit einem Alkohol der allgemeinen Formel HO-R³⁶ (3.5 Äquivalente) und DBU [1,8-Diaza-bicyclo[5.4.0]undec-7-en] (3.5 Äquivalente) in Acetonitril (7 ml pro mmol der Verbindung der Formel VI-A) 12 Stunden bei RT gerührt. Die Reaktionsmischung wird mehrfach mit EE extrahiert. Die vereinigten organischen Phasen werden mit ges. aq. NaCl-Lsg. gewaschen, über MgSO₄ getrocknet und das Lösungsmittel wurde im Vakuum entfernt. Der Rückstand wird jeweils durch Säulenchromatographie (SiO₂, verschiedene Mischungen Hexan/EE) gereinigt.

### Methode 2:

Verbindungen der allgemeinen Formel VI-C (2 mmol), worin R⁹, R³⁶, U, T und V die vorstehend genannte Bedeutung haben und m für 0, 1, 2 oder 3 steht, werden in THF (10 mL, 10 mL) gelöst und BH₃·S(CH₃)₂ [2.0 M in THF, 3 mL, 3 Äquivalent] wird hinzu gegeben. Die Reaktionsmischung wird 8 Stunden zum Rückfluss erhitzt, aq. HCl (2 N) wird hinzu gegeben und die Reaktionsmischung wird erneut für 30 Minuten zum Rückfluss erhitzt. Die Reaktionsmischung wird mit aq. Natriumhydroxid-Lösung (2N) versetzt und mit EE gewaschen. Die vereinigten organischen Phasen werden mit ges. aq. NaCl-Lösung gewaschen und über Magnesiumsulfat getrocknet. Das Lösungsmittel wird im Vakuum entfernt und der Rückstand säulenchromatographisch (SiO₂, verschiedene Mischung aus Dichlormethan und Methanol als Laufmittel) gereinigt.

### Methode 3:

Verbindungen der allgemeinen Formel VI-C (1.5 mmol), worin R⁹, R³⁶, U, T und V die vorstehend genannte Bedeutung haben und m für 0, 1, 2 oder 3 steht, werden in Diethylether (3 mL) gelöst und bei 0 °C wird eine Suspension von Lithiumaluminiumhydrid (3 mmol) in Ether (5 mL) langsam zu getropft. Die Reaktionsmischung wird 4 Stunden zum Rückfluss erhitzt und bei 0 °C wird langsam Methanol und anschließend 1 N aq. NaOH-Lösung zu getropft. Die Reaktionsmischung wird mit Methanol verdünnt und über Celite filtriert. Das Lösungsmittel wird im Vakuum entfernt und der Rückstand säulenchromatographisch (SiO₂, verschiedene Mischung aus Dichlormethan und Methanol als Laufmittel) gereinigt.

### 4. Allgemeine Vorschrift zur Darstellung von Aminen der allgemeinen Formel V-C

Die Darstellung von Aminen der allgemeinen Formel V-C erfolgt wie im nachfolgenden Schema 4 dargestellt.

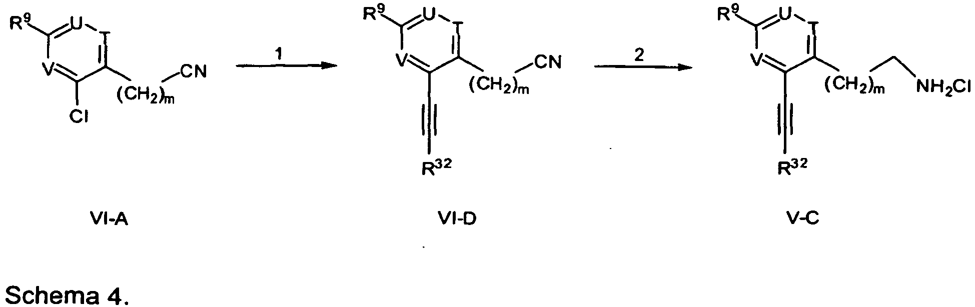

### Stufe 1: Darstellung von Nitrilen der allgemeinen Formel VI-D

Verbindungen der allgemeinen Formel VI-A (1 Äquivalent), worin R⁹, U, T und V die vorstehend genannte Bedeutung haben und m für 0, 1, 2 oder 3 steht, werden mit Bis(triphenylphosphin)palladium-dichlorid (7 Mol-%) und Kupfer(I)iodid (14 Mol-%) in 1-Methyl-2-pyrrolidinon (7 mL pro mmol Verbindung der allgemeinen Formel VI-A) gelöst. Nach 10 Minuten werden das Alkin der allgemeinen Formel HC≡C-R³² (3.5 Äquivalente) und N,N-Diisopropylethylamin (2 Äquivalente) zugegeben und die Reaktionsmischung wird 12 h bei einer Temperatur zwischen 90 und 110 °C gerührt. Die Reaktionsmischung wird über Celite filtriert und mehrfach mit EE extrahiert. Die vereinigten organischen Phasen werden mit ges. aq. NaCl-Lsg. gewaschen, über MgSO₄ getrocknet und das Lösungsmittel wurde im Vakuum entfernt. Der Rückstand wird jeweils durch Säulenchromatographie (SiO₂, verschiedene Mischungen Hexan/EE) gereinigt.

### 5. Allgemeine Vorschrift zur Darstellung von Aminen der allgemeinen Formel V-D

Die Darstellung von Aminen der allgemeinen Formel V-D erfolgt wie im nachfolgenden Schema 5 dargestellt.

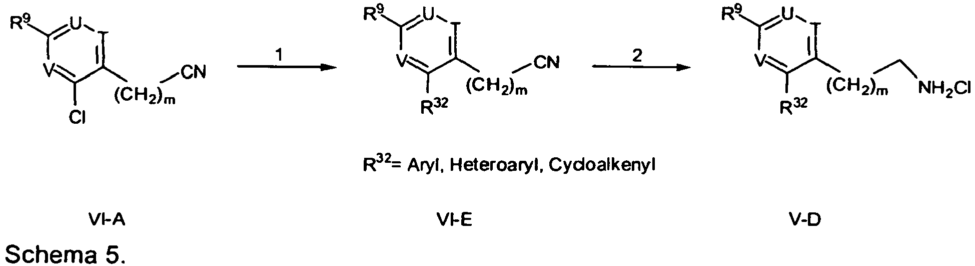

### Stufe 1: Darstellung von Nitrilen der allgemeinen Formel VI-E

Verbindungen der allgemeinen Formel VI-A (1 Äquivalent), worin R⁹, U, T und V die vorstehend genannte Bedeutung haben und m für 0, 1, 2 oder 3 steht, werden mit Palladiumdichlorid (5 mol-%) und einer Verbindung der allgemeinen Formel R³²-B(OH)₂ (2 Äquivalente), worin R³² für Aryl, Heteroaryl oder Cycloalkenyl steht, in einem Lösungsmittelgemisch aus Toluol/Dioxan/ 2 N aq. Natriumcarbonat-Lösung (20 ml pro 1 mmol Verbindungen der allgemeinen Formel VI-A) gerührt. Die Reaktionsmischung wird 12 h unter Rückfluss erhitzt und über Celite filtriert. Die vereinigten organischen Phasen werden über Magnesiumsulfat getrocknet und das Lösungsmittel im Vakuum entfernt. Der Rückstand wird säulenchromatographisch (SiO₂, verschiedene Lösungsmittelgemische von Hexan und EE) gereinigt.

### Stufe 2:

### Methode 1

Verbindungen der allgemeinen Formel VI-E (5 mmol), worin R⁹, R³², U, T und V die vorstehend genannte Bedeutung haben und m für 0, 1, 2 oder 3 steht, Palladium auf Kohle (10 %, 500 mg) und konzentrierte Salzsäure (3 mL) werden in MeOH (30 mL) gelöst und 6 Stunden bei RT einer Wasserstoffatmosphäre ausgesetzt. Die Reaktionsmischung wird über Celite filtriert und das Filtrat wird im Vakuum eingeengt. Der Rückstand wird mittels Flashchromatographie (SiO₂, EE) gereinigt.

### Methode 2:

Verbindungen der allgemeinen Formel VI-E (2 mmol), worin R⁹, R³², U, T und V die vorstehend genannte Bedeutung haben und m für 0, 1, 2 oder 3 steht, werden in THF (10 mL, 10 mL) gelöst und BH₃·S(CH₃)₂ [2.0 M in THF, 3 mL, 3 Äquivalent] wird hinzu gegeben. Die Reaktionsmischung wird 8 Stunden zum Rückfluss erhitzt, aq. HCl (2 N) wird hinzu gegeben und die Reaktionsmischung wird erneut für 30 Minuten zum Rückfluss erhitzt. Die Reaktionsmischung wird mit aq. Natriumhydroxid-Lösung (2N) versetzt und mit EE gewaschen. Die vereinigten organischen Phasen werden mit ges. aq. NaCl-Lösung gewaschen und über Magnesiumsulfat getrocknet. Das Lösungsmittel wird im Vakuum entfernt und der Rückstand säulenchromatographisch (SiO₂, verschiedene Mischung aus Dichlormethan und Methanol als Laufmittel) gereinigt.

### 6. Allgemeine Vorschrift zur Darstellung von Carbonsäuren der allgemeinen Formel VIIa

Die Darstellung von Carbonsäuren der allgemeinen Formel Vlla erfolgt wie im nachfolgenden Schema 6 dargestellt.

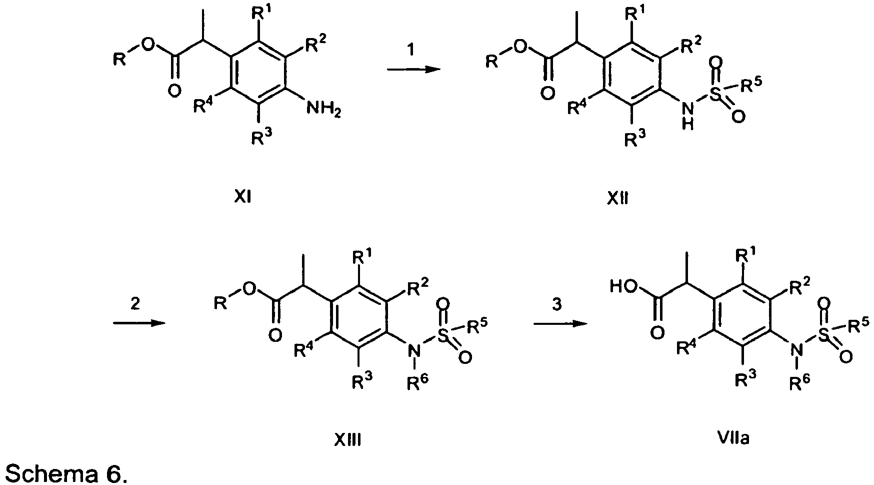

### Stufe 1:

Verbindungen der allgemeinen Formel XI (7 mmol), worin R¹, R², R³ und R⁴ die vorstehend genannte Bedeutung haben und R für einen linearen oder verzweigten C₁₋₆-Alkyl-Rest steht, werden mit Verbindungen der allgemeinen Formel Cl-S(=O)₂-R⁵ (8 mmol), worin R⁵ die vorstehend genannte Bedeutung hat, 10 min bei 0 °C und anschließend 3 Stunden bei Raumtemperatur in Pyridin (10 mL) gerührt. Die Reaktionsmischung wird in Dichlormethan und aq. HCl (1 N) aufgenommen, die organische Phase wird abgetrennt und das Lösungsmittel im Vakuum entfernt. Der Rückstand wird jeweils aus Dichlormethan/Hexan-Mischungen kristallisiert.

### Stufe 2:

Zu einer Suspension von 1.25 Äquivalenten NaH (60-%ig) in DMF wurde 1 Äquivalent der Verbindungen der allgemeinen Formel XII, worin R¹, R², R³, R⁴ und R⁵ die vorstehend genannte Bedeutung haben und R für einen linearen oder verzweigten C₁₋₆-Alkyl-Rest steht, zugegeben und die Suspension wurde 30 Minuten bei Raumtemperatur gerührt. Zu dieser Reaktionsmischung wurden 3.5 Äquivalente einer Verbindung der allgemeinen Formel R⁶-I, worin R⁶ für einen linearen oder verzweigten C₁₋₆-Alkyl-Rest steht, portionsweise zugegeben und die Suspension wurde 1.5 h bei 100 °C gerührt und langsam auf Raumtemperatur abgekühlt. Nach Zugabe von Wasser wurde das Reaktionsgemisch zweifach mit EE extrahiert, die vereinigten organischen Phasen mehrfach mit ges. aq. NaCl-Lsg. gewaschen, über MgSO₄ getrocknet und eingeengt. Das Rohprodukt wurde direkt im nächsten Schritt weiter verarbeitet.

### Stufe 3:

Verbindungen der allgemeinen Formel XIII (5 mmol), worin R¹, R², R³, R⁴, R⁵ und R⁶ die vorstehend genannte Bedeutung haben und R für einen linearen oder verzweigten C₁₋₆-Alkyl-Rest steht, werden mit Lithiumhydroxid Monohydrat (15 mmol) in einem Lösungsmittel-Gemisch aus Wasser und Tetrahydrofuran (1:2, 24 mL) 4 Stunden bei 40 °C gerührt. Die Reaktionsmischung wird in Dichlormethan und Wasser aufgenommen, mit aq. Salzsäure (1 N) versetzt und mehrfach mit Dichlormethan extrahiert. Die vereinigten organischen Phasen werden mit ges. aq. NaCl-Lösung gewaschen und über Natriumsulfat getrocknet. Das Lösungsmittel wird im Vakuum entfernt und der Rückstand aus Ethylacetat/Hexan-Mischungen kristallisiert.

### 7. Allgemeine Vorschrift zur Umsetzung von Aminen der allgemeinen Formeln V oder X mit Carbonsäuren der allgemeinen Formel VII

Die Säure der allgemeinen Formel VII (1 Äquivalent), das Amin der allgemeinen Formeln V oder X (1.2 Äquivalente) und EDCI (1.2 Äquivalente) werden in DMF (10 mmol Säure in 20 mL) 12 Stunden bei RT gerührt und anschließend Wasser dazugegeben. Die Reaktionsmischung wird mehrfach mit EE extrahiert, die wässrige Phase wird mit NaCl gesättigt und anschließend wieder mit EE extrahiert. Die vereinigten organischen Phasen werden mit 1 N Salzsäure und ges. aq. NaCl-Lsg. gewaschen, über MgSO₄ getrocknet und das Lösungsmittel wird im Vakuum entfernt. Der Rückstand wird mittels Flashchromatographie (SiO₂, EE/Hexan 1:2) gereinigt.

Die folgenden Beispielverbindungen wurden gemäß der vorstehend genannten allgemeinen Vorschrift erhalten.

### Beispielverbindung 3:

### 2-(3-Fluor-4-(N-methylmethylsulfonamido)phenyl)-N-((2-(4-methylpiperidin-1-yl)-6-(trifluormethyl)pyridin-3-yl)methyl)propanamid

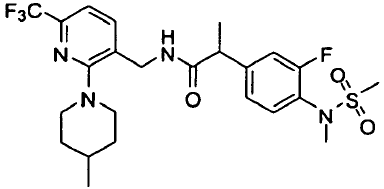

¹H NMR (300 MHz, CDCl₃) δ 7.47 (d, 1 H, J = 7.8 Hz), 7.38 (dd, 1 H, J = 8.1, 8.1 Hz), 7.21 (d, 1 H, J = 7.8 Hz), 7.07-7.14 (m. 2 H), 6.31 (bt, 1 H), 4.47 (d, 2 H, J = 5.7 Hz), 3.57 (q, 1 H, J = 6.9 Hz), 3.23-3.55 (m, 5 H), 3.95 (s, 3 H), 2.81 (m, 2 H), 1.73 (m, 2 H) 1.52 (d, 3 H, J = 6.9 Hz), 1.17-1.34 (m, 3 H), 0.98 (d, 3 H, J = 6.3 Hz) IR (KBr) 3245, 2923, 1651, 1508, 1443, 1330, 1222, 1159, 1111 cm⁻¹
MS (FAB) *m*/*z* 531 (M+H)

### 8. Allgemeine Vorschrift zur Darstellung von Verbindungen der allgemeinen Formel I, worin R⁶ für -C(=O)-R²⁸ steht

1 Äquivalent einer Verbindung der allgemeinen Formel I, worin R⁶ für Wasserstoff steht, und 1 Äquivalent des entsprechenden frisch destillierten Carbonsäureanhydrids der allgemeinen Formel R²⁸-C(=O)-O-C(=O)-R²⁸ wurden in wenig Dichlormethan gelöst und mit einigen Tropfen H₂SO₄ versetzt und 2 h bei 100 °C gerührt. Nach dem Abkühlen auf Raumtemperatur wurde das Reaktionsgemisch auf Eiswasser gegossen und die wässrige Phase zweimal mit Dichlormethan extrahiert. Die vereinigten organischen Phasen wurden mit Sodalösung entsäuert, mit Wasser gewaschen und über MgSO₄ getrocknet. Nach Entfernen des Lösungsmittels im Vakuum wurde das Rohprodukt säulenchromatographisch (Silicagel: mesh 100-200, Eluent: 15 % EE in Hexan) gereinigt.

### Beispielverbindung 1:

### N-(2-Fluor-4-(1-oxo-1-((2-(4-(N-phenylpropionamido)piperidin-1-yl)-6-(trifluormethyl)pyridin-3-yl)methylamino)propan-2-yl)phenyl)-N-(methylsulfonyl)propionamid

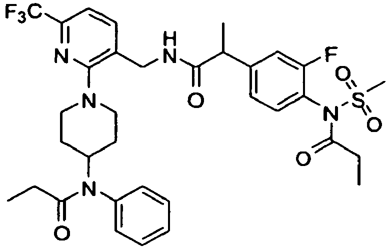

¹H NMR (300 MHz, CDCl₃) δ 7.29-7.44 (m, 5 H), 7.10-7.19 (m, 5 H), 6.24 (bt, 1 H), 4.78 (m, 1 H), 4.38 (d, 2 H, J = 5.1 Hz), 3.50 (q, 1 H, J = 6.9 Hz), 3.46(s, 3 H), 3.35-3.39 (m, 2 H), 2.97 -3.04 (m, 2 H), 1.89-1.96 (m, 4 H) 1.45-1.65(m, 9 H), 0.99-1.10 (m, 6 H)
IR (KBr) 2934, 1716, 1644, 1592, 1504, 1415, 1361, 1277, 1160, 963, 915,732cm⁻¹ MS (FAB) *m*/*z* 706(M+H)

### Beispielverbindung 2:

### 2-(3-Fluor-4-(N-(methylsulfonyl)acetamido)phenyl)-N-((2-(4-methylpiperidin-1-yl)-6-(trifluormethyl)pyridin-3-yl)methyl)propanamid

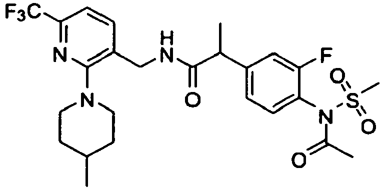

¹H NMR (300 MHz, CDCl₃) δ 7.42 (d, 1 H, J = 7.8 Hz), 7.31 (dd, 1 H, J = 8.1, 8.1 Hz), 7.17-7.25 (m, 3 H), 6.37 (bt, 1 H), 4.46 (m, 2 H), 3.57 (q, 1 H, J = 6.9 Hz), 3.43(s, 3 H), 3.31 (m, 2 H), 2.82 (m, 2 H), 1.98 (s, 3 H), 1.72 (m, 2 H) 1.54 (d, 3 H, J = 7.2 Hz), 1.21-1.29 (m, 3 H), 0.96 (d, 3 H, J = 6.6 Hz)
IR (KBr) 3265, 2933, 1652, 1519, 1433, 1327, 1223, 1161, 1105 cm⁻¹
MS (FAB) *m*/*z* 559(M+H)

Die weiteren Beispielverbindungen wurden nach den vorstehend beschriebenen Methoden erhalten.

| | |
|---|---|
| [4] * | N-(4-tert-Butylbenzyl)-2-(3-fluor-4-(N-methylmethylsulfonamido)phenyl)propanamid, |
| [5] * | (S)-N-(4-tert-Butylbenzyl)-2-(3-fluor-4-(N-methylmethylsulfonamido)phenyl)propanamid, |
| [6] * | (S)-N-(4-tert-Butytbenzyl)-2-(4-(N-ethylmethylsulfonamido)-3-fluorphenyl)propanamid, |
| [7] | N-((2-(Cyclohexylthio)-6-(trifluormethyl)pyridin-3-yl)methyl)-2-(3-fluor-4-(N-methylmethylsulfonamido)phenyl)propanamid, |
| [8] | N-((2-(Cyclohexylthio)-6-(trifluormethyl)pyridin-3-yl)methyl)-2-(4-(N-ethylmethylsulfonamido)-3-fluorphenyl)propanamid, |
| [9] | 2-(4-(N-Ethylmethylsulfonamido)-3-fluorphenyl)-N-((2-(4-methylpiperidin-1-yl)-6-(trifluormethyl)pyridin-3-yl)methyl)propanamid, |
| [10] | N-((6-tert-butyl-2-(4-methylpiperidin-1-yl)pyridin-3-yl)methyl)-2-(3-fluor-4-(N-methylmethylsulfonamido)phenyl)propanamid, |
| [11] | N-((6-tert-Butyl-2-(cyclohexylthio)pyridin-3-yl)methyl)-2-(3-fluor-4-(N-methylmethylsulfonamido)phenyl)propanamid, |
| [12] * | 2-(3-Fluor-4-(N-methylmethylsulfonamido)phenyl)-N-(2-(4-methylpiperidin-1-yl)-4-(trifluormethyl)benzyl)propanamid, |
| [13] * | N-(2-(Cyclohexylthio)-4-(trifluormethyl)benzyl)-2-(3-fluor-4-(N-methylmethylsulfonamido)phenyl)propanamid, |
| [14] * | N-(4-tert-Butyl-2-(4-methylpiperidin-1-yl)benzyl)-2-(3-fluor-4-(N-methylmethylsulfonamido)phenyl)propanamid, |
| [15] * | N-(4-tert-Butyl-2-(cyclohexylthio)benzyl)-2-(3-fluor-4-(N-methylmethylsulfonamido)phenyl)propanamid, |

| | |
|---|---|
| *Referenzbeispiele | |

In der nachfolgenden Tabelle 1. sind die gemessenen Werte aus der Massenspektrometrie für die weiteren Beispielverbindungen angegeben.

**Tabelle 1**

| **Beispielverbindung** | **[M+H]** |
|---|---|
| 4 * | 421 |
| 5 * | 421 |
| 6 * | 435 |
| 7 | 548 |
| 8 | 562 |
| 9 | 545 |
| 10 | 519 |
| 11 | 536 |
| 12 * | 530 |
| 13 * | 547 |
| 14 * | 518 |
| 15 * | 535 |

| | |
|---|---|
| *Referenzbeispiele | |

### Pharmakologische Daten

Die Affinität der erfindungsgemäßen Verbindungen für den Vanilloid-Rezeptor 1 (VR1/TRPV1-Rezeptor) wurde wie vorstehend beschrieben bestimmt (Pharmakologische Methoden I bzw. II).

Die erfindungsgemäßen Verbindungen der vorstehend angegebenen Formel I weisen eine ausgezeichnet Affinität zum VR1/TRPV1-Rezeptor auf (Tabelle 2.).

**Tabelle 2.**

| **Verbindung gemäß Beispiel** | **Kᵢ (Ratte) Capsaicin [nM]** | **Kᵢ (Mensch) Capsaicin [nM]** | **IC₅₀ (Mensch) [nM] nach pH-Stimulus** |
|---|---|---|---|
| 1 | 19,5 | 69.1 | 524.5 |
| 2 | 2,2 | 11.9 | 947 |
| 3 | 20 | 55.5 | 16% @ 10µM; 8% @ 5 µm |
| 4 * | 50,4 | ne | 22% @ 10 µM; 0% @ 5 µM |
| 6 * | 66,8 | ne | 21 @ 10 µM; 0% @ 5 µM |
| 7 | 30.8 | 48.1 | 20% @ 10 µM; 0% @ 5 µM |
| 9 | 63.8 | 22.7 | 46% @ 10 µM; 11% @ 5 µM |
| 10 | 2,8 | 27,7 | 11% @ 10 µM; 0% @ 5 µM |
| 11 | 16,8 | 56% @ 5 µM; 33% @ 1 µM; 0% @ 0,1 µM | ne |
| 12 * | 5,0 | 7,8 | 43% @ 10 µM; 0% @ 5 µM |
| 13 * | 5,2 | 33,1 | ne |
| 14 * | 3 | 13,1 | 43% @ 10 µM; 9% @ 5 µM |
| 15 * | 7,6 | 18,3 | 30% @ 10 µM; 6% @ 5 µM; 0% @ 1 µM |

| | | | |
|---|---|---|---|
| *Referenzbeispiele ne bedeutet jeweils "no effect", d. h. es wurde keine Reaktion beobachtet. | | | |

Der Wert nach dem Zeichen "@" gibt die Konzentration an, bei der die Hemmung (in Prozent) jeweils bestimmt wurde.

## Patentansprüche

1. Substituierte Verbindungen der allgemeinen Formel I, 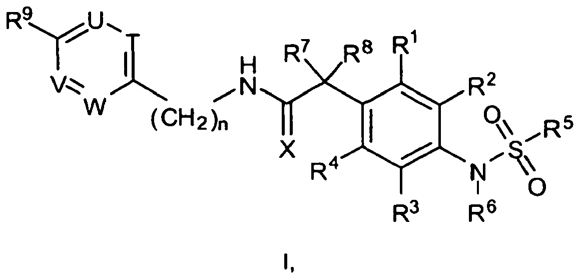 worin
X für O, S oder N-C≡N steht;
n für 0, 1, 2, 3 oder 4 steht;
R¹, R², R³ und R⁴, unabhängig voneinander, jeweils für H; F; Cl; Br; I; -SF₅; -NO₂; -CN; -NH₂, -OH; -SH; -C(=O)-NH₂; -S(=O)₂-NH₂; -C(=O)-NH-OH; -C(=O)-OH; -C(=O)-H; -S(=O)₂-OH; -NHR¹⁰; -NR¹¹R¹²; -OR¹³; -SR¹⁴; -C(=O)-NHR¹⁵; - C(=O)-NR¹⁶R¹⁷; -S(=O)₂-NHR¹⁸; -S(=O)₂-NR¹⁹R²⁰; -C(=O)-OR²¹; -C(=O)-R²²; -S(=O)-R²³; -S(=O)₂-R²⁴ oder für einen linearen oder verzweigten, gesättigten oder ungesättigten, unsubstituierten oder wenigstens einfach substituierten aliphatischen C₁₋₁₀ Rest stehen;
R⁵ für -NH₂; -NHR²⁵; -NR²⁶R²⁷ oder für einen linearen oder verzweigten, gesättigten oder ungesättigten, unsubstituierten oder wenigstens einfach substituierten aliphatischen C₁₋₁₀ Rest steht;
R⁶ für -C(=O)-R²⁸ oder für einen linearen oder verzweigten, gesättigten oder ungesättigten, unsubstituierten oder wenigstens einfach substituierten aliphatischen C₁₋₁₀ Rest steht;
R⁷ und R⁸, unabhängig voneinander, jeweils für einen Wasserstoff-Rest;
für einen linearen oder verzweigten, gesättigten oder ungesättigten, unsubstituierten oder wenigstens einfach substituierten aliphatischen C₁₋₁₀ Rest;
für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest stehen;
mit der Maßgabe, dass R⁷ und R⁸ nicht beide jeweils für einen Wasserstoff-Rest stehen;
oder
R⁷ und R⁸ jeweils zusammen mit dem sie verbindenden Kohlenstoffatom als Ringglied einen gesättigten oder ungesättigten, unsubstituierten oder wenigstens einfach substituierten 3-, 4-, 5- oder 6-gliedrigen cycloaliphatischen Rest bilden;
T für C-R²⁹ und U für C-R³⁰ und V für N und W für C-R³²
oder
T für C-R²⁹ und U für N und V für C-R³¹ und W für C-R³²
oder
T für N und U für C-R³⁰ und V für C-R³¹ und W für C-R³²
oder
T für N und U für N und V für C-R³¹ und W für C-R³²
oder
T für N und U für C-R³⁰ und V für N und W für C-R³²
oder
T für C-R²⁹ und U für N und V für N und W für C-R³²
steht;
R⁹ für F; Cl; Br; I; -SF₅; -NO₂; -CF₃; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹⁰; -NR¹¹R¹²; - OR¹³; -SR¹⁴; -C(=O)-NHR¹⁵; -C(=O)-NR¹⁶R¹⁷; -S(=O)₂-NHR¹⁸; -S(=O)₂-NR¹⁹R²⁰; -C(=O)-OR²¹; -C(=O)-R²²; -S(=O)-R²³; -S(=O)₂-R²⁴;
für einen linearen oder verzweigten, gesättigten oder ungesättigten, unsubstituierten oder wenigstens einfach substituierten aliphatischen C₁₋₁₀ Rest;
oder für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest steht;
R¹⁰, R¹¹, R¹², R¹³, R¹⁴, R¹⁵, R¹⁶, R¹⁷, R¹⁸, R¹⁹, R²⁰, R²¹, R²², R²³ und R²⁴, unabhängig voneinander, jeweils
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder 2- bis 6-gliedrige Heteroalkylen-Gruppe gebunden sein kann, stehen;
R²⁵, R²⁶ und R²⁷, unabhängig voneinander, jeweils für einen linearen oder verzweigten, gesättigten oder ungesättigten, unsubstituierten oder wenigstens einfach substituierten aliphatischen C₁₋₁₀ Rest stehen;
R²⁸ für einen linearen oder verzweigten, gesättigten oder ungesättigten, unsubstituierten oder wenigstens einfach substituierten aliphatischen C₁₋₁₀ Rest steht;
R²⁹, R³⁰ und R³¹, unabhängig voneinander, jeweils für H; F; Cl; Br; I; -SF₅; -NO₂; -CF₃; -CN; -NH₂; -OH; -SH; -C(=O)-NH₂; -S(=O)₂-NH₂; -C(=O)-NH-OH; - C(=O)-OH; -C(=O)-H; -S(=O)₂-OH; -NHR¹⁰; -NR¹¹R¹²; -OR¹³; -SR¹⁴; -C(=O)-NHR¹⁵; -C(=O)-NR¹⁶R¹⁷; -S(=O)₂-NHR¹⁸; -S(=O)₂-NR¹⁹R²⁰; -C(=O)-OR²¹; - C(=O)-R²²; -S(=O)-R²³; -S(=O)-R²⁴;
für einen linearen oder verzweigten, gesättigten oder ungesättigten, unsubstituierten oder wenigstens einfach substituierten aliphatischen C₁₋₁₀ Rest;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann, steht;
R³² für H; F; Cl; Br; I; -SF₅; -NO₂; -CF₃; -CF₂Cl; -CN; -NH_{2:} -OH; -SH; -C(=O)-NH₂; -S(=O)₂-NH₂; -C(=O)-NH-OH; -C(=O)-OH; -C(=O)-H; -S(=O)₂-OH; -NHR³³;-NR³⁴R³⁵; -OR³⁶; -SR³⁷; -C(=O)-NHR³⁸; -C(=O)-NR³⁹R⁴⁰; -S(=O)₂-NHR⁴¹; - S(=O)₂-NR⁴²R⁴³; -C(=O)-OR⁴⁴; -C(=O)-R⁴⁵; -S(=O)-R⁴⁶; -S(=O)₂-R⁴⁷; -C(=NH)-NH₂; -C(=NH)-NH-R⁴⁸; -N=C(NH₂)₂; -N=C(NHR⁴⁹)(NHR⁵⁰);
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der jeweils über ein Kohlenstoffatom im Ring des cycloaliphatischen Restes an das Grundgerüst gebunden ist und mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann;
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann, steht;
R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁴⁸, R⁴⁹ und R⁵⁰, unabhängig voneinander, jeweils
für einen linearen oder verzweigten, gesättigten oder ungesättigten, unsubstituierten oder wenigstens einfach substituierten aliphatischen C₁₋₁₀ Rest;
für einen ungesättigten oder gesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der mit einem gesättigten oder ungesättigten, unsubstituierten oder wenigstens einfach substituierten mono- oder polyzyklischen Ringsystem kondensiert und/oder über eine lineare oder verzweigte, unsubstituierte oder wenigstens einfach substituierte C₁₋₆-Alkylen-Gruppe oder 2- bis 6-gliedrige Heteroalkylen-Gruppe gebunden sein kann:
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder 2- bis 6-gliedrige Heteroalkylen-Gruppe gebunden sein kann, stehen;
oder
R³⁴ und R³⁵ jeweils zusammen mit dem sie verbindenden Stickstoffatom als Ringglied einen gesättigten oder ungesättigten, unsubstituierten oder mit 1, 2, 3, 4 oder 5 Resten R⁵¹ substituierten 4-, 5-, 6-, 7-, 8- oder 9-gliedrigen, ggf. wenigstens ein weiteres Heteroatom als Ringglied aufweisenden heterocycloaliphatischen Rest bilden, der mit einem gesättigten oder ungesättigten, unsubstituierten oder wenigstens einfach substituierten mono-oder polyzyklischen Ringsystem kondensiert sein kann;
R⁵¹ für -NHR⁵², -NR⁵³R⁵⁴ oder für einen linearen oder verzweigten, gesättigten oder ungesättigten, unsubstituierten oder wenigstens einfach substituierten aliphatischen C₁₋₁₀ Rest;
R⁵², R⁵³ und R⁵⁴, unabhängig voneinander, jeweils für-C(=O)-R⁵⁵; für einen linearen oder verzweigten, gesättigten oder ungesättigten, unsubstituierten oder wenigstens einfach substituierten aliphatischen C₁₋₁₀ Rest
oder für einen unsubstituierten oder wenigstens einfach substituierten 5- bis 14-gliedrigen Aryl- oder Heteroaryl-Rest, der mit einem gesättigten oder ungesättigten, unsubstituierten oder wenigstens einfach substituierten mono-oder polyzyklischen Ringsystem kondensiert und/oder über eine lineare oder verzweigte, unsubstituierte oder wenigstens einfach substituierte C₁₋₆-AlkylenGruppe oder C-₂₋₆-Alkenylen-Gruppe oder C₂₋₆-Alkinylen-Gruppe gebunden sein kann, stehen;
und
R⁵⁵ für einen linearen oder verzweigten, gesättigten oder ungesättigten, unsubstituierten oder wenigstens einfach substituierten aliphatischen C₁₋₁₀ Rest steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate;
wobei
die vorstehend genannten aliphatischen C₁₋₁₀ Reste ggf. jeweils mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), - NH(C₁₋₅-Alkyl), -N(C₁₋₅-Alkyl)(C₁₋₅-Alkyl), -C(=O)-O-C₁₋₅-Alkyl, -O-C(=O)-C₁₋₅-Alkyl, -0-Phenyl, Phenyl, -OCF₃ und -SCF₃ substituiert sein können;
die vorstehend genannten 2- bis 6-gliedrigen Heteroalkylen-Gruppen, C₁₋₆-AlkylenGruppen und C₂₋₆-Alkenylen-Gruppen und C₂₋₆-Alkinylen-Gruppen ggf. jeweils mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert sein können;
die vorstehend genannten Heteroalkylen-Gruppen jeweils ggf. 1, 2 oder 3 Heteroatom(e) unabhängig voneinander ausgewählt aus der Gruppe bestehend aus Sauerstoff, Schwefel und Stickstoff (NH) als Kettenglied(er) aufweisen;
die vorstehend genannten (hetero)cycloaliphatischen Reste ggf. jeweils mit 1, 2, 3, 4 oder 5 Substituenten unabhängig voneinander ausgewählt aus der Gruppe bestehend aus -C₁₋₆-Alkylen-OH, =CH₂, -O-C₁₋₅-Alkylen-Oxetanyl, -C₁₋₅-Alkylen-O-C₁₋₅-Alkylen-Oxetanyl,-CH₂-NH-C₁₋₅-Alkyl, -CH₂-N(C₁₋₅-Alkyl)₂, -N[C(=O)-C₁₋₅-Alkyl]-Phenyl, -CH₂-O-C₁₋₅-Alkyl, Oxo (=O), Thioxo (=S), F, Cl, Br, I, -CN, -CF₃, -SF₅, -OH, -O-C_{I}-₅-Alkyl, -O-C(=O)-C₁₋₅-Alkyl, -NH₂, -NO₂, -O-CF₃, -S-CF₃, -SH, -S-C₁₋₅-Alkyl, -C₁₋₅-Alkyl, -C(=O)-C₁₋₅-Alkyl, -C(=O)-OH, -C(=O)-O-C₁₋₅-Alkyl, -NH-C₁₋₅-Alkyl, -N(C₁₋₅-Alkyl)₂, - NH-Phenyl, -N(C₁₋₅-Alkyl)-Phenyl, Cyclohexyl, Cyclopentyl, (4,5)-Dihydroisoxazolyl, Thiazolyl, (1,2,5)-Thiadiazolyl, Thiophenyl, Phenethyl, Piperidinyl, Pyrrolidinyl, -(CH₂)-Pyridinyl, Pyridinyl, -O-Phenyl, -O-Benzyl, Phenyl und Benzyl substituiert sein, wobei jeweils der zyklische Teil der Reste Oxetanyl, (4,5)-Dihydroisoxazolyl, Thiazolyl, (1,2,5)-Thiadiazolyl, Thiophenyl, Phenethyl, -N[C(=O)-C₁₋₅-Alkyl]-Phenyl, -NH-Phenyl, -N(C₁₋₅-Alkyl)-Phenyl, -(CH₂)-Pyridinyl, Pyridinyl, -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und, sofern nicht anders angegeben, die vorstehend genannten (hetero)cycloaliphatischen Reste jeweils ggf. 1, 2 oder 3 (weitere) Heteroatom(e) unabhängig voneinander ausgewählt aus der Gruppe bestehend aus Sauerstoff, Stickstoff und Schwefel aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, - O-C₁₋₅-Alkyl, -NH₂, -NO₂, -O-CF₃, -S-CF₃, -SH, -S-C₁₋₅-Alkyl, -C₁₋₅-Alkyl, -C(=O)-C₁₋₅-Alkyl, -C(=O)-OH, -C(=O)-O-C₁₋₅-Alkyl, -NH-C₁₋₅-Alkyl, -N(C₁₋₅-Alkyl)₂, -O-Phenyl, -0-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH. -C(=O)-O-C₁₋₅-Alkyl, -NH-C₁₋₅-Alkyl, -N(C₁₋₅-Alkyl)₂, -NH-S(=O)₂-C₁₋₅-Alkyl, -NH-C(=O)-O-C₁₋₅-Alkyl, -C(=O)-H, -C(=O)-C₁₋₅-Alkyl, - C(=O)-NH₂, -C(=O)-NH-C₁₋₅-Alkyl, -C(=O)-N-(C₁₋₅-Alkyl)₂, -O-Phenyl, -O-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, - CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und
die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen.

2. Verbindungen gemäß Anspruch 1, **dadurch gekennzeichnet, dass**
X für O steht;
n für 0, 1 oder 2 steht;
R¹, R², R³ und R⁴, unabhängig voneinander, jeweils für H; F; Cl, Br; I; -SF₅; -NO₂; -CN; -NH₂; -OH; -SH; -C(=O)-NH₂; -S(=O)₂-NH₂; -C(=O)-NH-OH; - C(=O)-OH; -C(=O)-H; -S(=O)₂-OH; -NHR¹⁰; -NR¹¹R¹²; -OR¹³; -SR¹⁴; -S(=O)-R²³; -S(=O)₂-R²⁴ oder für einen Rest ausgewählt aus der Gruppe bestehend aus Methyl, -CF₃, -CCl₃, -CBr₃, -CHF₂, -CH₂F, -CF₂Cl, -CCl₂F, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCI-CF₂Cl, n-Propyl, -CF₂-CF₂-CF₃, -CF(CF₃)₂, Isopropyl, n-Butyl, sek-Butyl, Isobutyl und tert-Butyl stehen;
R⁵ für -NH₂; -NHR²⁵; -NR²⁶R²⁷; für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
R⁶ für -C(=O)-R²⁸ oder für einen Rest ausgewählt aus der Gruppe bestehend aus Methyl, -CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, Ethyl, =CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, -CH₂-CH₂-CN, n-Propyl, - CF₂-CF₂-CF₃, -CF(CF₃)₂, Isopropyl, -CH₂-CH₂-CH₂-CN, -CH₂-O-CH₂-CH₃, - CH₂-CH₂-SF₃, -CH₂-CH₂-OCF₃, -CH(CH₃)(O-CH₃), -CH(CH₃)(S-CH₃), n-Butyl, - CF₂-CF₂-CF₂-CF₃, -CH₂-CH₂-CH₂-CH₂-CN, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-CH₂-O-CH₃, Ethenyl, Propenyl, 2-Butenyl, 3-Butenyl, 2-Pentenyl und 3-Pentenyl steht;
R⁷ und R⁸, unabhängig voneinander, jeweils für einen Wasserstoff-Rest;
für einen Alkyl-Rest ausgewählt aus der Gruppe bestehend aus -CH₂-OH, - CH₂-CH₂-OH, -CH₂-CH₂-CH₂-C)H, -CH₂-CH₂-CH₂-CH₂-OH, Isopropyl, n-Butyl, sek-Butyl, Isobutyl, Methyl, Ethyl und n-Propyl;
für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine -(CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, - NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
oder für einen Rest ausgewählt aus der Gruppe bestehend aus Cyclopropyl, Cyclobutyl, Cyclopentyl, Cyclohexyl und Cycloheptyl stehen;
mit der Maßgabe, dass R⁷ und R⁸ nicht jeweils für einen Wasserstoff-Rest stehen;
oder
R⁷ und R⁸ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
T für C-R²⁹ und U für C-R³⁰ und V für N und W für C-R³²
oder
T für C-R²⁹ und U für N und V für C-R³¹ und W für C-R³²
oder
T für N und U für C-R³⁰ und V für C-R³¹ und W für C-R³²
oder
T für N und U für N und V für C-R³¹ und W für C-R³²
oder
T für N und U für C-R³⁰ und V für N und W für C-R³²
oder
T für C-R²⁹ und U für N und V für N und W für C-R³²
steht;
R⁹ für F; Cl; Br; I; -SF₅; -OR¹³; -SR¹⁴; -S(=O)-R²³; -S(=O)₂-R²⁴;
für einen Rest ausgewählt aus der Gruppe bestehend aus Methyl, -CF₃, - CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-Propyl, -CF₂-CF₂-CF₃, - CF(CF₃)₂, Isopropyl, -CH₂-CH₂-CH₂-CN, -CH₂-O-CH₂-CH₃, -CH₂-CH₂-SF₃, - CH₂-CH₂-OCF₃, -CH(CH₃)(O-CH₃), -CH(CH₃)(S-CH₃), n-Butyl, -CF₂-CF₂-CF₂-CF₃, -CH₂-CH₂-CH₂-CH₂-CN, n-Butyl, sek-Butyl, Isobutyl, -C(CH₃)₂(CH₂OH) und tert-Butyl;
oder für einen Rest ausgewählt aus der Gruppe bestehend aus Cyclopropyl, Cyclobutyl, Cyclopentyl, Cyclohexyl, Cycloheptyl, Cyclopentenyl und Cyclohexenyl steht, der ggf. jeweils mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-C(CH₃)₃, -O-CF₃, - S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
R¹⁰, R¹¹, R¹², R¹³, R¹⁴, R²¹, R²³ und R²⁴, unabhängig voneinander, jeweils für einen Rest ausgewählt aus der Gruppe bestehend aus Methyl, -CF₃, -CCl₃, -CBr₃, -CHF₂, - CH₂F, -CF₂Cl, -CCl₂F, -CH₂-CN, -CH₂-O-CH₃, -CH₂-O-CF₃, -CH₂-SF₃, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-Propyl, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃, -CH₂-CH₂-CH₂-CH₂-CN, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-CH₂-O-CH₃, Ethenyl, Propenyl, 2-Butenyl, 3-Butenyl, 2-Pentenyl und 3-Pentenyl stehen;
für einen Rest ausgewählt aus der Gruppe bestehend aus 2,3-Dihydro-1 H-indenyl, Cyclopropyl, Oxetanyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über eine -CH₂-O-, -CH₂-CH₂-O-, -CH₂-CH₂-O-CH₂-, -CH₂-CH(CH₃)-O-CH₂-, -(CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, - O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Thiazolyl, Oxazolyl und Isoxazolyl, wobei der Rest jeweils über eine -(CH, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -0-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, - C(=O)-O-CH(CH₃)₂, -C(=O)-O-C(CH₃)₃, -NH-CH₃, -NH-C₂H₅, -NH-C(CH₃)₃, - N(CH₃)₂, -N(C₂H₅)₂, -N(CH₃)(C₂H₅), -NH-C(=O)-O-CH₃, -NH-C(=O)-O-C₂H₅, - NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, stehen;
R²⁵, R²⁶ und R²⁷, unabhängig voneinander, jeweils für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl stehen;
R²⁸ für einen Rest ausgewählt aus der Gruppe bestehend aus Methyl, -CF₃, -CCl₃, -CBr₃, -CHF₂, -CH₂F, -CF₂Cl, -CH₂-CN, -CH₂-O-CH₃, -CH₂-O-CF₃, -CH₂-SF₃, Ethyl, -CF₂-CH₃, -CH₂-CF₃, -C₂F₅, -CH₂-CCl₃, -CH₂-CBr₃, -CHF-CF₂Cl, - CF₂-CF₂Cl, -CFCl-CF₂Cl, -CH₂-CH₂-CN, n-Propyl, Isopropyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, Ethenyl, Propenyl, 2-Butenyl, 3-Butenyl, 2-Pentenyl und 3-Pentenyl steht;
R²⁹, R³⁰ und R³¹, unabhängig voneinander, jeweils für H; F; Cl; Br; I; -SF₅; -NO₂; -CN; -NH₂; -OH; -SH; -C(=O)-NH₂; -S(=O)₂-NH₂; -C(=O)-NH-OH; - C(=O)-OH; -C(=O)-H; -S(=O)₂-OH; -NHR¹⁰; -NR¹¹R¹²; -OR¹³; -SR¹⁴; -C(=O)-OR²¹ ; -S(=O)-R²³; -S(=O)₂-R²⁴; für einen Rest ausgewählt aus der Gruppe bestehend aus -CH₂-OH, Methyl, -CF₃, -CCl₃, -CBr₃, -CHF₂, -CH₂F, -CF₂Cl, - CCl₂F, Ethyl, -CF₂-CH₃, -CH₂-CF₃, -C₂F₅, -CH₂-CCl₃, -CH₂-CBr₃, -CHF-CF₂Cl, - CF₂-CF₂Cl, -CFCl-CF₂Cl, n-Propyl, -CF₂-CF₂-CF₃, -CF(CF₃)₂, Isopropyl, n-Butyl, sek-Butyl, Isobutyl und tert-Butyl oder für einen Phenyl-Rest stehen, der unsubstituiert oder ggf. mit 1, 2, 3, 4 oder 5 Substituenten unabhängig voneinander ausgewählt aus der Gruppe bestehend aus F, Cl, Br, I, -CN, - CF₃, -SF₅, -OH, -O-CH₃, -O-C₂H₅, -O-CH(CH₃)₂, -O-C(CH₃)₃, -NH₂, -NO₂, -0-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl und n-Pentyl substituiert sein kann;
R³² für H; -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR³³; -NR³⁴R³⁵; -OR³⁸; -SR³⁷; - C(=O)-OR⁴⁴; -S(=O)-R⁴⁶; -S(=O)₂-R⁴⁷; -C(=NH)-NH₂; -C(=NH)-NH-R⁴⁸; - N=C(NH₂)₂; -N=C(NHR⁴⁹)(NHR⁵⁰);
für einen Rest ausgewählt aus der Gruppe bestehend aus Cyclopropyl, Cyclobutyl, Cyclopentyl, Cyclohexyl, Cyclohexenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste oder über eine -(CH=CH)-, -C≡C- oder -C≡C-CH₂-Gruppe an das Grundgerüst gebunden ist und unsubstituiert oder ggf. jeweils mit 1, 2, 3, 4 oder 5 Substituenten unabhängig voneinander ausgewählt aus der Gruppe bestehend aus-CN, -CH₂-N(CH₃)₂, -CH₂-N(C₂H₅)₂, -CH₂-NH-CH₃, -CH₂-NH-C₂H₅, -N-[C(=O)-C₂H₅]-Phenyl, -N-[C(=O)-CH₃]-Phenyl, Oxo (=O), Thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, - C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus (1,3)-Benzodioxolyl, (1,4)-Benzodioxanyl, Tetrazolyl, (2,3)-Dihydrothieno[3,4-b][1,4]dioxinyl, Benzo[b]furanyl, Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Thiazolyl, Oxazolyl, Isoxazolyl, Pyridazinyl, Pyrazinyl, Pyrimidinyl, Indazolyl, Chinoxalinyl, Chinolinyl und Isochinolinyl, der jeweils über eine - (CH=CH)-, -C≡C-, -(CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, - NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, - C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, - N(CH₃)(C₂H₅), -NH-C(=O)-O-CH₃, -NH-S(=O)₂-CH₃, -NH-S(=O₂)-C₂H₅, -NH-S(=O)₂-CH(CH₃)₂, -NH-C(=O)-O-C₂H₅, -NH-C(=O)-O-C(CH₃)₃, -C(=O)-H, - C(=O)-CH₃, -C(=O)-C₂H₅, -C(=O)-CH(CH₃)₂, -C(=O)-C(CH₃)₃, -C(=O)-NH₂, - C(=O)-NH-CH₃, -C(=O)-NH-C₂H₅, -C(=O)-N(CH₃)₂, -C(=O)-N(C₂H₅)₂, -0-Phenyl, -O-Benzyl, Phenyl und Benzyl substituiert sein kann, steht;
R³³, R³⁴, R³⁵, R³⁶, R³⁷, R⁴⁴, R⁴⁶, R⁴⁷, R⁴⁸, R⁴⁹ und R⁵⁰, unabhängig voneinander, jeweils
für einen Rest ausgewählt aus der Gruppe bestehend aus Methyl, -CF₃, -CCl₃, -CBr₃, -CHF₂, -CH₂F, -CF₂Cl, -CCl₂F, -CH₂-CN, -CH₂-O-CH₃, -CH₂-O-CF₃, - CH₂-SF₃, Ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-Propyl, -CF₂-CF₂-CF₃, - CF(CP₃)₂, Isopropyl, -CH₂-CH₂-CH₂-CN, -CH₂-O-CH₂-CH₃, -CH₂-CH₂-SF₃, - CH₂-CH₂-OCF₃, -CH(CH₃)(O-CH₃), -CH(CH₃)(S-CH₃), n-Butyl, -CF₂-CF₂-CF₂-CF₃, -CH₂-CH₂-CH₂-CH₂-CN, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, -CH₂-CH₂-O-CH₃, -CH₂-CH₂-O-C₂H₅, -CH₂-CH₂-CH₂-O-CH₃, Ethenyl, Propenyl, 2-Butenyl, 3-Butenyl, 2-Pentenyl und 3-Pentenyl;
für einen Rest ausgewählt aus der Gruppe bestehend aus 2,3-Dihydro-1 H-indenyl, Cyclopropyl, Oxetanyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über eine -CH₂-O-, -CH₂-CH₂-O-, -CH₂-CH₂-O-CH₂-, -CH₂-CH(CH₃)-O-CH₂-, -(CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, - O-C(CH₃)₃, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂ und -C(=O)-O-C(CH₃)₃ substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Thiazolyl, Oxazolyl und Isoxazolyl, wobei der Rest jeweils über eine -(CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -0-CH(CH₃)₂, -O-C(CH₃)₃, -NH₂, -NO₂, -O-CF₃, -S-CF₃, -SH, -S-CH₃; -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, - C(=O)-O-CH(CH₃)₂, -C(=O)-O-C(CH₃)₃, -NH-CH₃, -NH-C₂H₅, -NH-C(CH₃)₃, - N(CH₃)₂, -N(C₂H₅)₂, -N(CH₃)(C₂H₅), -NH-C(=O)-O-CH₃, -NH-C(=O)-O-C₂H₅, - NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, stehen;
oder
R³⁴ und R³⁵ jeweils zusammen mit dem sie verbindenden Stickstoffatom als Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Azabicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl, (1,2,3,6)-Tetrahydropyridinyl, (4,5,6,7)-Tetrahydroisoxazolo[5,4-c]pyridinyl, Pyrrolidinyl, Piperidinyl, Piperazinyl, Morpholinyl, Azepanyl, Diazepanyl und Thiomorpholinyl bilden, dessen heterocycloaliphatischer Anteil jeweils unsubstituiert oder mit 1, 2, 3, 4 oder 5 Resten R⁵¹ substituiert sein kann;
R⁵¹ für -NHR⁵², -NR⁵³R⁵⁴ oder für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
R⁵² R⁵³ und R⁵⁴, unabhängig voneinander, jeweils für -C(=O)-R⁵⁵; für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Thiazolyl, Oxazolyl und Isoxazolyl, wobei der Rest jeweils über eine -(CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, Methyl, Ethyl, n-Propyl, Isopropyl, n-Butyl, sek-Butyl, Isobutyl, tert-Butyl, n-Pentyl, -C(=O)-OH, -C(=O)-O-CH₃, -C(=O)-O-C₂H₅, - C(=O)-O-CH(CH₃)₂, -C(=O)-O-C(CH₃)₃, -NH-CH₃, -NH-C₂H₅, -NH-C(CH₃)₃, - N(CH₃)₂, -N(C₂H₅)₂, -N(CH₃)(C₂H₅), -NH-C(=O)-O-CH₃, -NH-C(=O)O-C₂H₅, - NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-Phenyl, -O-Benzyl, Phenyl und Benzyl substituiert sein kann, stehen;
und
R⁵⁵ für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

3. Verbindungen gemäß Anspruch 1 oder 2, **dadurch gekennzeichnet, dass**
X für O steht;
n für 1 steht;
R¹, R³ und R⁴, unabhängig voneinander, jeweils für H; F; Cl; Br; I; -NHR¹⁰; -NR¹¹R¹² ; -OR¹³; -SR¹⁴ oder für einen Rest ausgewählt aus der Gruppe bestehend aus Methyl, -CF₃, -CHF₂, -CH₂F, Ethyl, n-Propyl, -CF₂-CF₂-CF₃, - CF(CF₃)₂, Isopropyl, n-Butyl, sek-Butyl, Isobutyl und tert-Butyl stehen;
R² für H; F; Cl; Br; I oder Methyl steht;
R⁵ für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
R⁶ für -C(=O)-R²⁸ oder für einen Rest ausgewählt aus der Gruppe bestehend aus Methyl, -CH₂-CN, Ethyl, -CH₂-CH₂-CN, n-Propyl, -CH₂-CH₂-CH₂-CN, n-Butyl, - CH₂-CH₂-CH₂-CH₂-CN, sek-Butyl, Isobutyl, tert-Butyl, n-Pentyl, 3-Pentyl, n-Heptyl, Ethenyl, Propenyl, 2-Butenyl, 3-Butenyl, 2-Pentenyl und 3-Pentenyl steht;
R⁷ für einen Alkyl-Rest ausgewählt aus der Gruppe bestehend aus -CH₂-OH, - CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl;
oder für einen Rest ausgewählt aus der Gruppe bestehend aus Cyclopropyl, Cyclobutyl, Cyclopentyl, Cyclohexyl und Cycloheptyl steht;
R⁸ für einen Wasserstoff-Rest steht;
oder
R⁷ und R⁸ jeweils zusammen mit dem sie verbindenden Kohlenstoffatom als Ringglied einen Rest ausgewählt aus der Gruppe bestehend aus Cyclopropyl, Cyclobutyl, Cyclopentyl, Cyclohexyl und Cycloheptyl bilden;
T für C-R²⁹ und U für C-R³⁰ und V für N und W für C-R³²
steht;
R⁹ für F; Cl; Br; I; -SF₅; -O-CF₃; -O-CCl₃; -O-CBr₃; -O-CHF₂; -O-CH₂F; -O-CF₂Cl; - O-CCl₂F; -O-CF₂-CH₃; -S-CF₃; -S-CCl₃; -S-CBr₃; -S-CHF₂; -S-CH₂F; -S-CF₂Cl; -S-CCl₂F; -S-CF₂-CH₃-, oder für einen Rest ausgewählt aus der Gruppe bestehend aus Methyl, -CF₃, -CHF₂, -CH₂F, Ethyl, n-Propyl, Isopropyl, n-Butyl, sek-Butyl, Isobutyl, -C(CH₃)₂(CH₂OH) und tert-Butyl steht;
R¹⁰, R¹¹, R¹², R¹³ und R¹⁴, unabhängig voneinander, jeweils
für einen Rest ausgewählt aus der Gruppe bestehend aus Methyl, -CF₃, - CHF₂, -CH₂F, Ethyl, n-Propyl, Isopropyl, n-Butyl, sek-Butyl, Isobutyl, tert-Butyl und n-Pentyl stehen;
R²⁸ für einen Rest ausgewählt aus der Gruppe bestehend aus Methyl, Ethyl, n-Propyl, Isopropyl, sek-Butyl, Isobutyl, tert-Butyl, n-Pentyl, 3-Pentyl, n-Heptyl, 4-Heptyl, n-Octyl, n-Nonyl, 5-Nonyl, (2,6)-Dimethyl-hept-4-yl, 3-Methyl-butyl, n-Hexyl, (3,3)-Dimethylbutyl, Ethenyl, Propenyl, 2-Butenyl, 3-Butenyl, 2-Pentenyl und 3-Pentenyl steht;
R²⁹ und R³⁰, unabhängig voneinander, jeweils für H; F; Cl; Br; I; -SF₅; -NO₂; -CN; -NH₂; -OH; -SH; -NHR¹⁰; -NR¹¹R¹²; -OR¹³; -SR¹⁴; oder für einen Rest ausgewählt aus der Gruppe bestehend aus -CH₂-OH, Methyl, -CF₃, Ethyl, n-Propyl, Isopropyl, n-Butyl, sek-Butyl, Isobutyl und tert-Butyl stehen;
R³² für H; -SF₅; -NO₂; -CN; -NH₂; -OH; -NHR³³; -NR³⁴R³⁵; -OR³⁶; -SR³⁷;
für einen Rest ausgewählt aus der Gruppe bestehend aus Cyclopropyl, Cyclobutyl, Cyclopentyl, Cyclohexyl, Cyclohexenyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils über ein Kohlenstoffatom der Ringe der vorstehend genannten Reste an das Grundgerüst gebunden ist und unsubstituiert oder ggf. jeweils mit 1, 2, 3, 4 oder 5 Substituenten unabhängig voneinander ausgewählt aus der Gruppe bestehend aus -CH₂-N(CH₃)₂, -CH₂-N(C₂H₅)₂, -CH₂-NH-CH₃, -CH₂-NH-C₂H₅, - N-[C(=O)-C₂H₅]-Phenyl, -N-[C(=O)-CH₃]-Phenyl, Methyl, Ethyl, n-Propyl, Isopropyl, n-Butyl, sek-Butyl, Isobutyl und tert-Butyl substituiert sein kann;
oder für einen Rest ausgewählt aus der Gruppe bestehend aus Phenyl, Thiophenyl, Furanyl, Pyrrolyl, Pyrazolyl, Pyrazinyl, Pyranyl, Triazolyl, Pyridinyl, Imidazolyl, Thiazolyl, Oxazolyl, Isoxazolyl, Pyridazinyl, Pyrazinyl und Pyrimidinyl, der jeweils ggf. mit 1, 2, 3, 4 oder 5 Substituenten unabhängig voneinander ausgewählt aus der Gruppe bestehend aus F, Cl, Br, I, -CN, - CF₃, -SF₅, -OH, -O-CH₃, -O-C₂H₅, -O-CH(CH₃)₂, -O-C(CH₃)₃, -O-CF₃, Methyl, Ethyl, n-Propyl, Isopropyl, n-Butyl, sek-Butyl, Isobutyl, tert-Butyl und n-Pentyl substituiert sein kann, steht;
R³³, R³⁴, R³⁵, R³⁶ und R³⁷, unabhängig voneinander, jeweils
für einen Rest ausgewählt aus der Gruppe bestehend aus Methyl, -CH₂-O-CH₃, Ethyl, n-Propyl, n-Butyl, sek-Butyl, Isobutyl, tert-Butyl, n-Pentyl, 3-Pentyl, n-Hexyl, (3,3)-Dimethylbutyl, -CH₂-CH₂-O-CH₃, -CH₂-CH₂-O-C₂H₅ und -CH₂-CH₂-CH₂-O-CH₃ stehen;
für einen Rest ausgewählt aus der Gruppe bestehend aus 2,3-Dihydro-1H-indenyl, Cyclopropyl, Oxetanyl, Cyclobutyl, Cyclopentyl, Cyclohexyl, Imidazolidinyl, Tetrahydrofuranyl, Tetrahydrothiophenyl, Pyrrolidinyl, Piperidinyl, Morpholinyl, Piperazinyl, Azepanyl, Diazepanyl, Azocanyl und Thiomorpholinyl, der jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, Isobutyl, tert-Butyl, n-Pentyl substituiert sein kann, stehen;
oder
R³⁴ und R³⁵ jeweils zusammen mit dem sie verbindenden Stickstoffatom als
Ringglied einen Rest ausgewählt aus der Gruppe bestehend aus 3-Aza-bicyclo[3.1.1]heptyl, 6-Aza-spiro[2.5]octyl, 3-Aza-bicyclo[3.2.1]octyl, 6-Aza-bicyclo[3.3.1]heptyl, 8-Aza-bicyclo[3.2.1]octyl, 1-Oxa-2,8-diaza-spiro[4.5]dec-2-enyl, Azocanyl, Isoindolyl, Indolyl,
(1,2,3,6)-Tetrahydropyridinyl, (4,5,6,7)-Tetrahydroisoxazolo[5,4-c]pyridinyl, Pyrrolidinyl, Piperidinyl, Piperazinyl, Morpholinyl, Azepanyl, Diazepanyl und Thiomorpholinyl bilden, dessen heterocycloaliphatischer Anteil jeweils unsubstituiert oder mit 1, 2, 3, 4 oder 5 Resten R⁵¹ substituiert sein kann;
R⁵¹ für -NHR⁵², -NR⁵³R⁵⁴ oder für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
R⁵², R⁵³ und R⁵⁴, unabhängig voneinander, jeweils für -C(=O)-R⁵⁵; für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl;
oder für einen Rest ausgewählt aus der Gruppe bestehend aus Phenyl und Naphthyl, wobei der Rest jeweils über eine -(CH₂)-, -(CH₂)₂- oder -(CH₂)₃-Gruppe gebunden sein kann und/oder jeweils unsubstituiert oder ggf. mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -O-CH(CH₃)₂, -O-C(CH₃)₃, Methyl, Ethyl, n-Propyl, Isopropyl, n-Butyl, sek-Butyl, Isobutyl, tert-Butyl und n-Pentyl substituiert sein kann, stehen;
und
R⁵⁵ für einen Alkyl-Rest ausgewählt aus der Gruppe bestehend aus -CF₃, -CH₂-CF₃, Methyl, Ethyl, n-Propyl, Isopropyl, tert-Butyl, n-Butyl, sek-Butyl und Isobutyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

4. Verbindungen gemäß einem oder mehreren der Ansprüche 1 bis 3, **dadurch gekennzeichnet, dass**
X für O steht;
n für 1 steht;
R¹, R³ und R⁴ jeweils für H stehen;
R² für F; Cl oder Br steht;
R⁵ für einen Alkyl-Rest ausgewählt aus der Gruppe bestehend aus Methyl, Ethyl, n-Propyl und Isopropyl steht;
R⁶ für -C(=O)-R²⁸ oder für einen Rest ausgewählt aus der Gruppe bestehend aus Methyl, -CH₂-CN, Ethyl, -CH₂-CH₂-CN, n-Propyl, -CH₂-CH₂-CH₂-CN, n-Butyl, - CH₂-CH₂-CH₂-CH₂-CN, sek-Butyl, Isobutyl, tert-Butyl und n-Pentyl steht;
R⁷ für einen Alkyl-Rest ausgewählt aus der Gruppe bestehend aus -CH₂-OH, - CH₂-CH₂-OH, -CH₂-CH₂-CH₂-OH, -CH₂-CH₂-CH₂-CH₂-OH, Isopropyl, n-Butyl, sek-Butyl, Isobutyl, Methyl, Ethyl und n-Propyl steht;
R⁸ für einen Wasserstoff-Rest steht;
T für C-R²⁹ und U für C-R³⁰ und V für N und W für C-R³²
steht;
R⁹ für -SF₅; -O-CF₃, -CF₃; -CHF₂; -CH₂F, -C(CH₃)₂(CH₂OH) oder tert-Butyl steht;
R²⁸ für einen Rest ausgewählt aus der Gruppe bestehend aus Methyl, Ethyl, n-Propyl, Isopropyl, sek-Butyl, Isobutyl, tert-Butyl, Ethenyl und Propenyl steht;
R²⁹ und R³⁰ jeweils für H stehen;
R³² für H; -NHR³³; -NR³⁴R³⁵; -OR³⁶ oder -SR³⁷ steht;
R³³, R³⁶ und R³⁷, unabhängig voneinander, jeweils
für einen Rest ausgewählt aus der Gruppe bestehend aus Cyclopropyl, Cyclobutyl, Cyclopentyl, Cyclohexyl und Cycloheptyl, der jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sek-Butyl, Isobutyl, tert-Butyl, n-Pentyl substituiert sein kann, stehen;
R³⁴ und R³⁵ jeweils zusammen mit dem sie verbindenden Stickstoffatom als Ringglied einen Rest ausgewählt aus der Gruppe bestehend Pyrrolidinyl, Piperidinyl, Piperazinyl, Morpholinyl und Azepanyl bilden, dessen heterocycloaliphatischer Anteil jeweils unsubstituiert oder mit 1, 2, 3, 4 oder 5 Resten R⁵¹ substituiert sein kann;
R⁵¹ für -NHR⁵², -NR⁵³R⁵⁴ oder für einen Alkyl-Rest ausgewählt aus der Gruppe bestehend aus Methyl, Ethyl, n-Propyl, Isopropyl, tert-Butyl, n-Butyl, sek-Butyl und Isobutyl steht;
R⁵², R⁵³ und R⁵⁴, unabhängig voneinander, jeweils für -C(=O)-R⁵⁵; für einen Alkyl-Rest ausgewählt aus der Gruppe bestehend aus Methyl, Ethyl, n-Propyl, Isopropyl, tert-Butyl, n-Butyl, sek-Butyl und Isobutyl;
oder für Phenyl-Rest stehen, wobei der Rest mit 1, 2, 3, 4 oder 5 Substituenten unabhängig voneinander ausgewählt aus der Gruppe bestehend aus F, Cl, Br, Methyl, Ethyl, n-Propyl und Isopropyl sein kann, stehen;
und
R⁵⁵ für einen Alkyl-Rest ausgewählt aus der Gruppe bestehend aus Methyl, Ethyl, n-Propyl, Isopropyl, tert-Butyl, n-Butyl, sek-Butyl und Isobutyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

5. Verbindungen der allgemeinen Formel Ia gemäß einem oder mehreren der Ansprüche 1 bis 4, 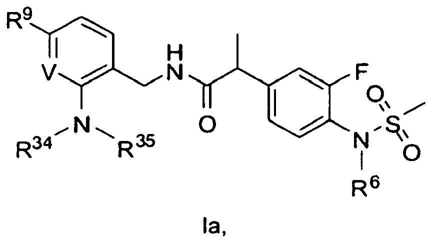 worin
V für N steht;
und R⁶, R⁹, R³⁴ und R³⁵ die Bedeutung gemäß Anspruch 3 haben;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

6. Verbindungen der allgemeinen Formel Ia gemäß einem oder mehreren der Ansprüche 1 bis 5, 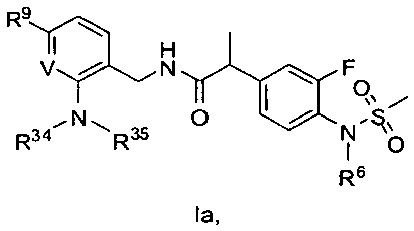 worin
V für N steht;
und R⁶, R⁹, R³⁴ und R³⁵ die Bedeutung gemäß Anspruch 4 haben;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

7. Verbindungen der allgemeinen Formel Ib gemäß einem oder mehreren der Ansprüche 1 bis 4, 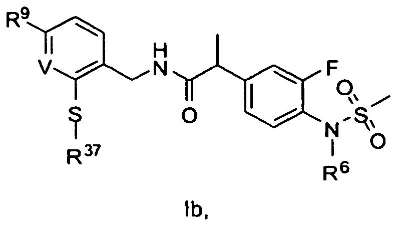 V für N steht;
und R⁶, R⁹ und R³⁷ die Bedeutung gemäß Anspruch 3 haben;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

8. Verbindungen der allgemeinen Formel Ib gemäß einem oder mehreren der Ansprüche 1 bis 7, 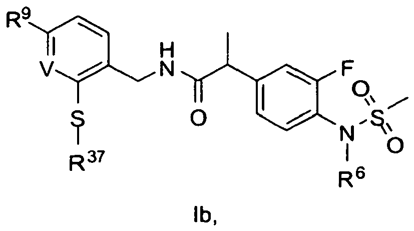 V für N steht;
und R⁶, R⁹ und R³⁷ die Bedeutung gemäß Anspruch 4 haben;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

9. Verbindungen gemäß einem oder mehreren der Ansprüche 1 bis 8 ausgewählt aus der Gruppe bestehend aus
[1] N-(2-Fluor-4-(1-oxo-1-((2-(4-(N-phenylpropionamido)piperidin-1-yl)-6-(trifluormethyl)pyridin-3-yl)methylamino)propan-2-yl)phenyl)-N-(methylsulfonyl)propionamid,
[2] 2-(3-Fluor-4-(N-(methylsulfonyl)acetamido)phenyl)-N-((2-(4-methylpiperidin-1-yl)-6-(trifluormethyl)pyridin-3-yl)methyl)propanamid,
[3] 2-(3-Fluor-4-(N-methylmethylsulfonamido)phenyl)-N-((2-(4-methylpiperidin-1-yl)-6-(trifluormethyl)pyridin-3-yl)methyl)propanamid,
[7] N-((2-(Cyclohexylthio)-6-(trifluormethyl)pyridin-3-yl)methyl)-2-(3-fluor-4-(N-methylmethylsulfonamido)phenyl)propanamid,
[8] N-((2-(Cyclohexylthio)-6-(trifluormethyl)pyridin-3-yl)methyl)-2-(4-(N-ethylmethylsulfonamido)-3-fluorphenyl)propanamid,
[9] 2-(4-(N-Ethylmethylsulfonamido)-3-fluorphenyl)-N-((2-(4-methylpiperidin-1-yl)-6-(trifluormethyl)pyridin-3-yl)methyl)propanamid,
[10] N-((6-tert-Butyl-2-(4-methylpiperidin-1-yl)pyridin-3-yl)methyl)-2-(3-fluor-4-(N-methylmethylsulfonamido)phenyl)propanamid,
[11] N-((6-tert-Butyl-2-(cyclohexylthio)pyridin-3-yl)methyl)-2-(3-fluor-4-(N-methylmethylsulfonamido)phenyl)propanamid,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

10. Verfahren zur Herstellung einer Verbindung gemäß einem oder mehreren der Ansprüche 1 bis 9, **dadurch gekennzeichnet, dass** wenigstens eine Verbindung der allgemeinen Formel II, 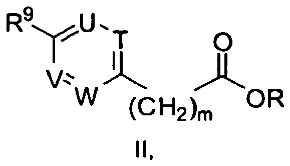 worin R⁹, U, T, V und W die Bedeutung gemäß einem oder mehreren der Ansprüche 1 bis 9 haben, m für 0, 1, 2 oder 3 steht und R für Wasserstoff oder für einen linearen oder verzweigten C₁₋₆-Alkyl-Rest steht, in einem Reaktionsmedium, in Gegenwart wenigstens eines Reduktionsmittels zu wenigstens einer Verbindung der allgemeinen Formel III, 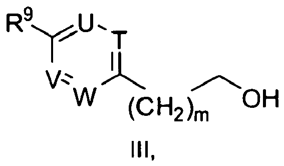 worin R⁹, U, T, V und W die Bedeutung gemäß einem oder mehreren der Ansprüche 1 bis 9 haben und m für 0, 1, 2 oder 3 steht, umgesetzt wird,
und wenigstens eine Verbindung der allgemeinen Formel III in einem Reaktionsmedium in Gegenwart von Diphenylphosphorylazid oder in Gegenwart von HN₃ zu wenigstens einer Verbindung der allgemeinen Formel IV, 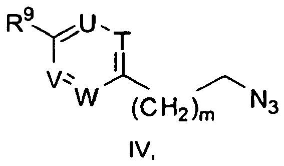 worin R⁹, U, T, V und W die Bedeutung gemäß einem oder mehreren der Ansprüche 1 bis 9 haben und m für 0, 1, 2 oder 3 steht, umgesetzt wird, und wenigstens eine Verbindung der allgemeinen Formel IV in einem Reaktionsmedium in Gegenwart wenigstens eines Reduktionsmittels
oder in einem Reaktionsmedium in Gegenwart eines Katalysators und in Gegenwart von Wasserstoff oder in Gegenwart von Hydrazin
oder in einem Reaktionsmedium in Gegenwart von Triphenylphosphin
zu wenigstens einer Verbindung der allgemeinen Formel V, 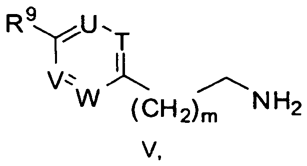 worin R⁹, U, T, V und W die Bedeutung gemäß einem oder mehreren der Ansprüche 1 bis 9 haben und m für 0, 1, 2 oder 3 steht, umgesetzt wird,
oder wenigstens eine Verbindung der allgemeinen Formel VI, 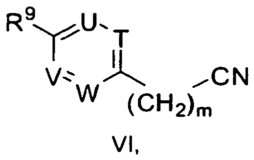 worin R⁹, U, T, V und W die Bedeutung gemäß einem oder mehreren der Ansprüche 1 bis 9 haben und m für 0, 1, 2 oder 3 steht, in einem Reaktionsmedium
in Gegenwart wenigstens eines Katalysators unter einer Wasserstoffatmosphäre,
oder in Gegenwart wenigstens eines Reduktionsmittels ausgewählt aus der Gruppe bestehend aus BH₃•S(CH₃)₂, Lithiumaluminiumhydrid und Natriumborhydrid,
zu wenigstens einer Verbindung der allgemeinen Formel V, ggf. in Form eines entsprechenden Salzes, umgesetzt wird,
und wenigstens eine Verbindung der allgemeinen Formel V mit wenigstens einer Verbindung der allgemeinen Formel VII, 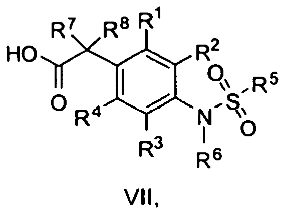 worin R¹, R², R³, R⁴, R⁵, R⁶, R⁷ und R⁸ die Bedeutung gemäß einem oder mehreren der Ansprüche 1 bis 9 haben und R⁶ zusätzlich für einen Wasserstoff-Rest stehen kann, in einem Reaktionsmedium,
oder mit wenigstens einer Verbindung der allgemeinen Formel VIII, 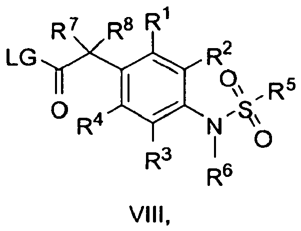 worin R¹, R², R³, R⁴, R⁵, R⁶, R⁷ und R⁸ die Bedeutung gemäß einem oder mehreren der Ansprüche 1 bis 9 haben, R⁶ zusätzlich für einen Wasserstoff-Rest stehen kann und LG für eine Abgangsgruppe steht, in einem Reaktionsmedium zu wenigstens einer Verbindung der allgemeinen Formel Ih, 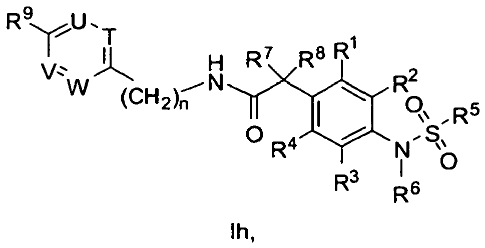 worin T, U, V, W, R¹, R², R³, R⁴, R⁵, R⁶, R⁷, R⁸ und R⁹, die Bedeutung gemäß einem oder mehreren der Ansprüche 1 bis 9 haben, R⁶ zusätzlich für einen Wasserstoff-Rest stehen kann und n für 1, 2, 3 oder 4 steht, umgesetzt wird,
und ggf. wenigstens eine Verbindung der allgemeinen Formel Ih in einem Reaktionsmedium mit wenigstens einer Verbindung der allgemeinen Formel IX, 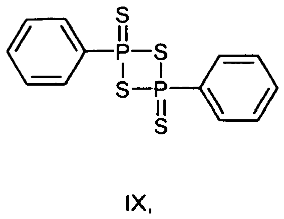 worin die Phenyl-Reste jeweils mit 1 oder 2 Substituenten unabhängig voneinander ausgewählt aus der Gruppe bestehend aus Methoxy, Phenoxy, Cl, Methyl und Br substituiert sind, oder mit Phosphorpentasulfid, zu wenigstens einer Verbindung der allgemeinen Formel Ik, 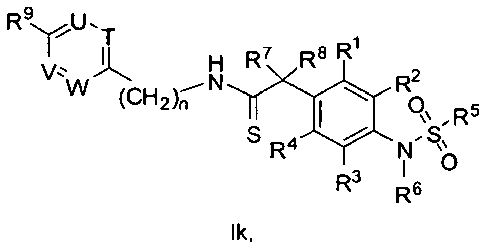 worin T, U, V, W, R¹, R², R³, R⁴, R⁵, R⁶, R⁷, R⁸ und R⁹, die Bedeutung gemäß einem oder mehreren der Ansprüche 1 bis 9 haben, R⁶ zusätzlich für einen Wasserstoff-Rest stehen kann und n für 1, 2, 3 oder 4 steht, umgesetzt wird;
und ggf. wenigstens eine Verbindung der allgemeinen Formel Io, 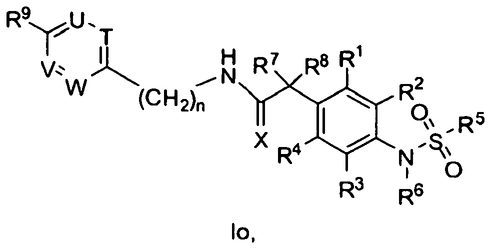 worin X, T, U, V, W, R¹, R², R³, R⁴, R⁵, R⁷, R⁸ und R⁹, die Bedeutung gemäß einem oder mehreren der Ansprüche 1 bis 9 haben, R⁶ für einen Wasserstoff-Rest steht und n für 1, 2, 3 oder 4 steht, in einem Reaktionsmedium in Gegenwart wenigstens einer Verbindung der allgemeinen Formel R²⁸-C(=O)-O-C(=O)-R²⁸, worin R²⁸ die Bedeutung gemäß einem oder mehreren der Ansprüche 1 bis 9 hat, zu wenigstens einer Verbindung der allgemeinen Formel I, worin X, T, U, V, W, R¹, R², R³, R⁴, R⁵, , R⁷, R⁸ und R⁹ die Bedeutung gemäß einem oder mehreren der Ansprüche 1 bis 9 haben, R⁶ für -C(=O)-R²⁸ und n für 1, 2, 3 oder 4 steht, umgesetzt wird.

11. Verfahren zur Herstellung wenigstens einer Verbindung gemäß einem oder mehreren der Ansprüche 1 bis 9, **dadurch gekennzeichnet, dass** wenigstens eine Verbindung der allgemeinen Formel X, 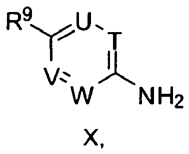 worin R⁹, U, T, V und W die Bedeutung gemäß einem oder mehreren der Ansprüche 1 bis 9 haben, mit wenigstens einer Verbindung der allgemeinen Formel VII, 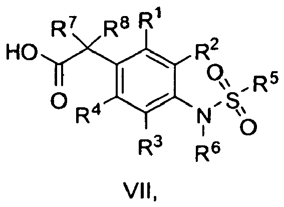 worin R¹, R², R³, R⁴, R⁵, R⁶, R⁷ und R⁸, die Bedeutung gemäß einem oder mehreren der Ansprüche 1 bis 9 haben und R⁶ zusätzlich für einen Wasserstoff-Rest stehen kann, in einem Reaktionsmedium,
oder mit wenigstens einer Verbindung der allgemeinen Formel VIII, 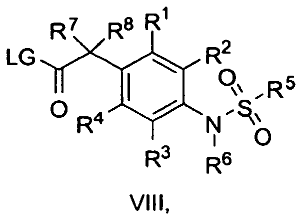 worin R¹, R², R³, R⁴, R⁵, R⁶, R⁷ und R⁸ die Bedeutung gemäß einem oder mehreren der Ansprüche 1 bis 9 haben, R⁶ zusätzlich für einen Wasserstoff-Rest stehen kann und LG für eine Abgangsgruppe steht, in einem Reaktionsmedium zu wenigstens einer Verbindung der allgemeinen Formel Im, 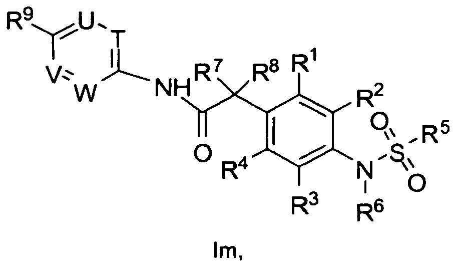 worin T, U, V, W, R¹, R², R³, R⁴, R⁵, R⁷, R⁸ und R⁹ die Bedeutung gemäß einem oder mehreren der Ansprüche 1 bis 9 haben, umgesetzt wird,
und ggf. wenigstens eine Verbindung der allgemeinen Formel Im in einem Reaktionsmedium mit wenigstens einer Verbindung der allgemeinen Formel IX, 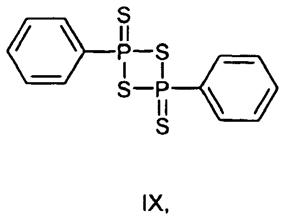 worin die Phenyl-Reste jeweils mit 1 oder 2 Substituenten unabhängig voneinander ausgewählt aus der Gruppe bestehend aus Methoxy, Phenoxy, Cl, Methyl und Br substituiert sind, oder mit Phosphorpentasulfid, zu wenigstens einer Verbindung der allgemeinen Formel In, 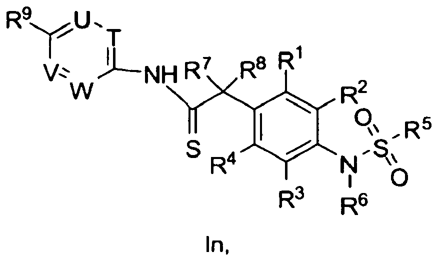 worin T, U, V, W, R¹, R², R³, R⁴, R⁵, R⁶, R⁷, R⁸ und R⁹ die Bedeutung gemäß einem oder mehreren der Ansprüche 1 bis 9 haben und R⁶ zusätzlich für einen Wasserstoff-Rest stehen kann, umgesetzt wird;
und ggf. wenigstens eine Verbindung der allgemeinen Formel Ip, 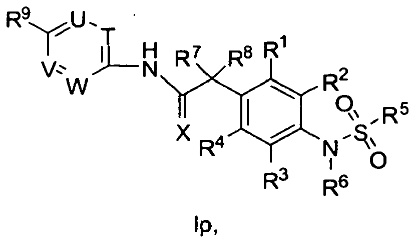 worin X, T, U, V, W, R¹, R², R³, R⁴, R⁵, R⁷, R⁸ und R⁹, die Bedeutung gemäß einem oder mehreren der Ansprüche 1 bis 9 haben und R⁶ für einen Wasserstoff-Rest steht, in einem Reaktionsmedium in Gegenwart wenigstens einer Verbindung der allgemeinen Formel R²⁸-C(=O)-O-C(=O)-R²⁸, worin R²⁸ die Bedeutung gemäß einem oder mehreren der Ansprüche 1 bis 9 hat, zu wenigstens einer Verbindung der allgemeinen Formel I, worin X, T, U, V, W, R¹, R², R³, R⁴, R⁵, R⁷, R⁸ und R⁹ die Bedeutung gemäß einem oder mehreren der Ansprüche 1 bis 9 haben und R⁶ für - C(=O)-R²⁸ steht, umgesetzt wird.

12. Arzneimittel enthaltend wenigstens eine Verbindung gemäß einem oder mehreren der Ansprüche 1 bis 9 und ggf. einen oder mehrere physiologisch verträgliche Hilfsstoffe.

13. Verwendung wenigstens einer Verbindung gemäß einem oder mehreren der Ansprüche 1 bis 9 zur Herstellung eines Arzneimittels zur Behandlung und/oder Prophylaxe von einer oder mehreren Erkrankungen ausgewählt aus der Gruppe bestehend aus Schmerz; Gelenkschmerz; Hyperalgesie; Allodynie; Kausalgie und Migräne.

14. Verwendung wenigstens einer Verbindung gemäß einem oder mehreren der Ansprüche 1 bis 9 zur Herstellung eines Arzneimittels zur Behandlung und/oder Prophylaxe von einer oder mehreren Erkrankungen ausgewählt aus der Gruppe bestehend aus Depressionen; Nervenleiden; Nervenverletzungen; neurodegenerativen Erkrankungen; kognitiven Dysfunktionen; und Epilepsie.

15. Verwendung wenigstens einer Verbindung gemäß einem oder mehreren der Ansprüche 1 bis 9 zur Herstellung eines Arzneimittels zur Behandlung und/oder Prophylaxe von einer oder mehreren Erkrankungen ausgewählt aus der Gruppe bestehend aus Atemwegserkrankungen; Husten; Harninkontinenz; einer überaktiven Blase (overactive bladder, OAB); Erkrankungen und/oder Verletzungen des Magen-Darm-Trakts; Zwölffingerdarmgeschwüren; Magengeschwüren; Reizdarmsyndrom; Schlaganfällen; Augenreizungen; Hautreizungen; neurotischen Hauterkrankungen; allergischen Hautkrankheiten; Psiorasis; Vitiligo; Herpes simplex; Entzündungen, vorzugsweise Entzündungen des Darmes, der Augen, der Blase, der Haut oder der Nasenschleimhaut; Diarrhöe; Pruritus; Osteoporose; Arthritis; Osteoarthritis; rheumatischen Erkrankungen; Störungen der Nahrungsaufnahme; Medikamentenabhängigkeit; Medikamentenmißbrauch; Entzugserscheinungen bei Medikamentenabhängigkeit; Toleranzentwicklung gegenüber Medikamenten; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Behandlung von Wunden und/oder Verbrennungen; zur Behandlung von durchtrennten Nerven; zur Libidosteigerung; zur Modulation der Bewegungsaktivität; zur Anxiolyse; zur Lokalanästhesie und/oder zur Hemmung unerwünschter Nebenwirkungen, ausgelöst durch die Verabreichung von Vanilloid-Rezeptor 1 (VR1/TRPV1-Rezeptoren)-Agonisten.

## Claims

1. Substituted compounds of the general formula I, 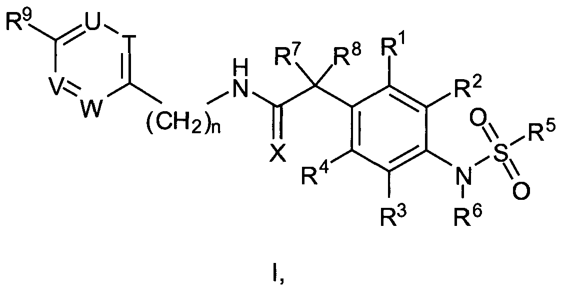 in which
X denotes O, S or N-C≡N;
n denotes 0, 1, 2, 3 or 4;
R¹, R², R³ and R⁴, mutually independently, in each case denote H; F; Cl; Br; I; -SF₅; -NO₂; -CN; -NH₂; -OH; -SH; -C(=O)-NH₂; -S(=O)₂-NH₂; -C(=O)-NH-OH; - C(=O)-OH; -C(=O)-H; -S(=O)₂-OH; -NHR¹⁰; -NR¹¹R¹²; -OR¹³; -SR¹⁴; -C(=O)-NHR¹⁵; -C(=O)-NR¹⁶R¹⁷; -S(=O)₂-NHR¹⁸; -S(=O)₂-NR¹⁹R²⁰; -C(=O)-OR²¹; - C(=O)-R²²; -S(=O)-R²³; -S(=O)₂-R²⁴ or denote a linear or branched, saturated or unsaturated, unsubstituted or at least monosubstituted aliphatic C₁₋₁₀ residue;
R⁵ denotes -NH₂; -NHR²⁵; -NR²⁶R²⁷ or denotes a linear or branched, saturated or unsaturated, unsubstituted or at least monosubstituted aliphatic C₁₋₁₀ residue;
R⁶ denotes -C(=O)-R²⁸ or denotes a linear or branched, saturated or unsaturated, unsubstituted or at least monosubstituted aliphatic C₁₋₁₀ residue;
R⁷ and R⁸, mutually independently, in each case denote a hydrogen residue;
denote a linear or branched, saturated or unsaturated, unsubstituted or at least monosubstituted aliphatic C₁₋₁₀ residue;
denote an unsubstituted or at least monosubstituted 5- to 14-membered aryl or heteroaryl residue, which may be fused with a saturated or unsaturated, unsubstituted or at least monosubstituted mono- or polycyclic ring system and/or be attached via a linear or branched, unsubstituted or at least monosubstituted C₁₋₆ alkylene group or C₂₋₆ alkenylene group or C₂₋₆ alkynylene group;
or denote an unsaturated or saturated, unsubstituted or at least monosubstituted 3-, 4-, 5-, 6-, 7-, 8- or 9-membered cycloaliphatic residue optionally comprising at least one heteroatom as a ring member;
providing that R⁷ and R⁸ do not both in each case denote a hydrogen residue;
or
R⁷ and R⁸ in each case together with the carbon atom joining them together as a ring member form a saturated or unsaturated, unsubstituted or at least monosubstituted 3-, 4-, 5- or 6-membered cycloaliphatic residue;
T denotes C-R²⁹ and U denotes C-R³⁰ and V denotes N and W denotes C-R³² or
T denotes C-R²⁹ and U denotes N and V denotes C-R³¹ and W denotes C-R³² or
T denotes N and U denotes C-R³⁰ and V denotes C-R³¹ and W denotes C-R³² or
T denotes N and U denotes N and V denotes C-R³¹ and W denotes C-R³²
or
T denotes N and U denotes C-R³⁰ and V denotes N and W denotes C-R³²
or
T denotes C-R²⁹ and U denotes N and V denotes N and W denotes C-R³²
R⁹ denotes F; Cl; Br; I; -SF₅; -NO₂; -CF₃; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹⁰; -NR¹¹R¹²; -OR¹³; -SR¹⁴; -C(=O)-NHR¹⁵; -C(=O)-NR¹⁶R¹⁷; - S(=O)₂-NHR¹⁸; -S(=O)₂-NR¹⁹R²⁰; -C(=O)-OR²¹; -C(=O)-R²²; -S(=O)-R²³; -S(=O)₂-R²⁴;
denotes a linear or branched, saturated or unsaturated, unsubstituted or at least monosubstituted aliphatic C₁₋₁₀ residue;
or denotes an unsaturated or saturated, unsubstituted or at least monosubstituted 3-, 4-, 5-, 6-, 7-, 8- or 9-membered cycloaliphatic residue optionally comprising at least one heteroatom as a ring member;
R¹⁰, R¹¹, R¹², R¹³, R¹⁴, R¹⁵, R¹⁶, R¹⁷, R¹⁸, R¹⁹, R²⁰, R²¹, R²², R²³ and R²⁴, mutually independently, in each case
denote a linear or branched, saturated or unsaturated, unsubstituted or at least monosubstituted aliphatic C₁₋₁₀ residue;
denote an unsaturated or saturated, unsubstituted or at least monosubstituted 3-, 4-, 5-, 6-, 7-, 8- or 9-membered cycloaliphatic residue optionally comprising at least one heteroatom as a ring member, which residue may be fused with a saturated or unsaturated, unsubstituted or at least monosubstituted mono- or polycyclic ring system and/or be attached via a linear or branched, unsubstituted or at least monosubstituted C₁₋₆ alkylene group or 2- to 6-membered heteroalkylene group;
or denote an unsubstituted or at least monosubstituted 5- to 14-membered aryl or heteroaryl residue, which may be fused with a saturated or unsaturated, unsubstituted or at least monosubstituted mono- or polycyclic ring system and/or be attached via a linear or branched, unsubstituted or at least monosubstituted C₁₋₆ alkylene group or 2- to 6-membered heteroalkylene group;
R²⁵, R²⁶ and R²⁷, mutually independently, in each case denote a linear or branched, saturated or unsaturated, unsubstituted or at least monosubstituted aliphatic C₁₋₁₀ residue;
R²⁸ denotes a linear or branched, saturated or unsaturated, unsubstituted or at least monosubstituted aliphatic C₁₋₁₀ residue;
R²⁹, R³⁰ and R³¹, mutually independently, in each case denote H; F; Cl; Br; I; - SF₅; -NO₂; -CF₃; -CN; -NH₂; -OH; -SH; -C(=O)-NH₂; -S(=O)₂-NH₂; - C(=O)-NH-OH; -C(=O)-OH; -C(=O)-H; -S(=O)₂-OH; -NHR¹⁰; -NR¹¹R¹²; - OR¹³; -SR¹⁴; -C(=O)-NHR¹⁵; -C(=O)-NR¹⁶R¹⁷; -S(=O)₂-NHR¹⁸; -S(=O)₂-NR¹⁹R²⁰; -C(=O)-OR²¹; -C(=O)-R²²; -S(=O)-R²³; -S(=O)₂-R²⁴;
denote a linear or branched, saturated or unsaturated, unsubstituted or at least monosubstituted aliphatic C₁₋₁₀ residue;
or denote an unsubstituted or at least monosubstituted 5- to 14-membered aryl or heteroaryl residue, which may be fused with a saturated or unsaturated, unsubstituted or at least monosubstituted mono- or polycyclic ring system and/or be attached via a linear or branched, unsubstituted or at least monosubstituted C₁₋₆ alkylene group or C₂₋₆ alkenylene group or C₂₋₆ alkynylene group;
R³² denotes H; F; Cl; Br; I; -SF₅; -NO₂; -CF₃; -CF₂Cl; -CN; -NH₂; -OH; -SH; - C(=O)-NH₂; -S(=O)₂-NH₂; -C(=O)-NH-OH; -C(=O)-OH; -C(=O)-H; - S(=O)₂-OH; -NHR³³; -NR³⁴R³⁵; -OR³⁶; -SR³⁷; -C(=O)-NHR³⁸; -C(=O)-NR³⁹R⁴⁰ ; -S(=O)₂-NHR⁴¹; -S(=O)₂-NR⁴²R⁴³; -C(=O)-OR⁴⁴; -C(=O)-R⁴⁵; - S(=O)-R⁴⁶; -S(=O)₂-R⁴⁷; -C(=NH)-NH₂; -C(=NH)-NH-R⁴⁸; -N=C(NH₂)₂; - N=C(NHR⁴⁹)(NHR⁵⁰);
denotes a linear or branched, saturated or unsaturated, unsubstituted or at least monosubstituted aliphatic C₁₋₁₀ residue;
denotes an unsaturated or saturated, unsubstituted or at least monosubstituted 3-, 4-, 5-, 6-, 7-, 8- or 9-membered cycloaliphatic residue optionally comprising at least one heteroatom as a ring member, which residue is in each case attached to the parent structure via a carbon atom in the ring of the cycloaliphatic residue and may be fused with a saturated or unsaturated, unsubstituted or at least monosubstituted mono- or polycyclic ring system and/or be attached via a linear or branched, unsubstituted or at least monosubstituted C₁₋₆ alkylene group or C₂₋₆ alkenylene group or C₂₋₆ alkynylene group;
or denotes an unsubstituted or at least monosubstituted 5- to 14-membered aryl or heteroaryl residue, which may be fused with a saturated or unsaturated, unsubstituted or at least monosubstituted mono- or polycyclic ring system and/or be attached via a linear or branched, unsubstituted or at least monosubstituted C₁₋₆ alkylene group or C₂₋₆ alkenylene group or C₂₋₆ alkynylene group;
R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁴⁸, R⁴⁹ and R⁵⁰, mutually independently, in each case
denote a linear or branched, saturated or unsaturated, unsubstituted or at least monosubstituted aliphatic C₁₋₁₀ residue;
denote an unsaturated or saturated, unsubstituted or at least monosubstituted 3-, 4-, 5-, 6-, 7-, 8- or 9-membered cycloaliphatic residue optionally comprising at least one heteroatom as a ring member, which residue may be fused with a saturated or unsaturated, unsubstituted or at least monosubstituted mono- or polycyclic ring system and/or be attached via a linear or branched, unsubstituted or at least monosubstituted C₁₋₆ alkylene group or 2- to 6-membered heteroalkylene group;
or denote an unsubstituted or at least monosubstituted 5- to 14-membered aryl or heteroaryl residue, which may be fused with a saturated or unsaturated, unsubstituted or at least monosubstituted mono- or polycyclic ring system and/or be attached via a linear or branched, unsubstituted or at least monosubstituted C₁₋₆ alkylene group or 2- to 6-membered heteroalkylene group;
or
R³⁴ and R³⁵ in each case together with the nitrogen atom joining them together as a ring member form a saturated or unsaturated, 4-, 5-, 6-, 7-, 8- or 9-membered heterocycloaliphatic residue, which may be unsubstituted or substituted with 1, 2, 3, 4 or 5 residues R⁵¹ and optionally comprising at least one further heteroatom as a ring member, which heterocycloaliphatic residue may be fused with a saturated or unsaturated, unsubstituted or at least monosubstituted mono- or polycyclic ring system;
R⁵¹ denotes -NHR⁵², -NR⁵³R⁵⁴ or denotes a linear or branched, saturated or unsaturated, unsubstituted or at least monosubstituted aliphatic C₁₋₁₀ residue;
R⁵², R⁵³ and R⁵⁴, mutually independently, in each case denote -C(=O)-R5⁵⁵; denote a linear or branched, saturated or unsaturated, unsubstituted or at least monosubstituted aliphatic C₁₋₁₀ residue;
or denote an unsubstituted or at least monosubstituted 5- to 14-membered aryl or heteroaryl residue, which may be fused with a saturated or unsaturated, unsubstituted or at least monosubstituted mono- or polycyclic ring system and/or be attached via a linear or branched, unsubstituted or at least monosubstituted C₁₋₆ alkylene group or C₂₋₆ alkenylene group or C₂₋₆ alkynylene group;
and
R⁵⁵ denotes a linear or branched, saturated or unsaturated, unsubstituted or at least monosubstituted aliphatic C₁₋₁₀ residue;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates;
wherein
the above-stated aliphatic C₁₋₁₀ residues may optionally in each case be substituted with 1, 2, 3, 4, 5, 6, 7, 8 or 9 substituents mutually independently selected from the group consisting of F, Cl, Br, I, -CN, -NO₂, -OH, -NH₂, -SH, - O(C₁₋₅-alkyl), -S(C₁₋₅-alkyl), -NH(C₁₋₅-alkyl), -N(C₁₋₅-alkyl)(C₁₋₅-alkyl), -C(=O)-O-C₁₋₅-alkyl, -O-C(=O)-C₁₋₅-alkyl, -O-phenyl, phenyl, -OCF₃ and -SCF₃;
the above-stated 2- to 6-membered heteroalkylene groups, C₁₋₆ alkylene groups and C₂₋₆ alkenylene groups and C₂₋₆ alkynylene groups may optionally in each case be substituted with 1, 2, 3, 4, 5, 6, 7, 8 or 9 substituents mutually independently selected from the group consisting of F, Cl, Br, I, -CN, -NO₂, - OH, -NH₂, -SH, -O(C₁₋₅-alkyl), -S(C₁₋₅-alkyl), -NH(C₁₋₅-alkyl), -N(C₁₋₅-alkyl)(C₁₋₅-alkyl), -OCF₃ and -SCF₃;
the above-stated heteroalkylene groups may in each case optionally comprise 1, 2 or 3 heteroatom(s) mutually independently selected from the group consisting of oxygen, sulfur and nitrogen (NH) as chain link(s);
the above-stated (hetero)cycloaliphatic residues may optionally in each case be substituted with 1, 2, 3, 4 or 5 substituents mutually independently selected from the group consisting of -C₁₋₆-alkylene-OH, =CH₂, -O-C₁₋₅-alkylene-oxetanyl, -C₁₋₅-alkylene-O-C₁₋₅-alkylene-oxetanyl, -CH₂-NH-C₁₋₅-alkyl, -CH₂-N(C₁₋₅-alkyl)₂, -N[-C(=O)-C₁₋₅-alkyl]-phenyl, -CH₂-O-C₁₋₅-alkyl. oxo (=O), thioxo (=S), F, Cl, Br, I, -CN, -CF₃, -SF₅, -OH, -O-C₁₋₅-alkyl, -O-C(=O)-C₁₋₅-alkyl. - NH₂, -NO₂, -O-CF₃, -S-CF₃, -SH, -S-C₁₋₅-alkyl, -C₁₋₅-alkyl, -C(=O)-C₁₋₅-alkyl, - C(=O)-OH, -C(=O)-O-C₁₋₅-alkyl, -NH-C₁₋₅-alkyl, -N(C₁₋₅-alkyl)₂, -NH-phenyl, - N(-C₁₋₅-alkyl)-phenyl, cyclohexyl, cyclopentyl, (4,5)-dihydroisoxazolyl, thiazolyl, (1,2,5)-thiadiazolyl, thiophenyl, phenethyl, piperidinyl, pyrrolidinyl, -(CH₂)-pyridinyl, pyridinyl, -O-phenyl, -O-benzyl, phenyl and benzyl, wherein in each case the cyclic moiety of the residues oxetanyl, (4,5)-dihydroisoxazolyl, thiazolyl, (1,2,5)-thiadiazolyl, thiophenyl, phenethyl, -N[-C(=O)-C₁₋₅-alkyl]-phenyl, -NH-phenyl, -N(-C₁₋₅-alkyl)-phenyl, -(CH₂)-pyridinyl, pyridinyl, -0-phenyl, -O-benzyl, phenyl and benzyl may be substituted with 1, 2, 3, 4 or 5 substituents mutually independently selected from the group consisting of F, Cl, Br, -OH, -CF₃, -SF₅, -CN, -NO₂, -C₁₋₅ alkyl, -O-C₁₋₅-alkyl, -O-CF₃, -S-CF₃, phenyl and -O-benzyl,
and unless otherwise stated the above-stated (hetero)cycloaliphatic residues may in each case optionally comprise 1, 2 or 3 (further) heteroatom(s) mutually independently selected from the group consisting of oxygen, nitrogen and sulfur;
the rings of the above-stated mono- or polycyclic ring systems may in each case optionally be substituted with 1, 2, 3, 4 or 5 substituents mutually independently selected from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅-alkyl, -C(=O)-C₁₋₅-alkyl, -C(=O)-OH, -C(=O)-O-C₁₋₅-alkyl, - NH-C₁₋₅-alkyl, -N(C₁₋₅-alkyl)₂, -O-phenyl, -O-benzyl, phenyl and benzyl, wherein in each case the cyclic moiety of the residues -O-phenyl, -O-benzyl, phenyl and benzyl may be substituted with 1, 2, 3, 4 or 5 substituents mutually independently selected from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
and the rings of the above-stated mono- or polycyclic ring systems are in each case 5-, 6- or 7-membered and may in each case optionally comprise 1, 2, 3, 4 or 5 heteroatom(s) as ring member(s), which are mutually independently selected from the group consisting of oxygen, nitrogen and sulfur;
and the above-stated aryl or heteroaryl residues may optionally in each case be substituted with 1, 2, 3, 4 or 5 substituents mutually independently selected from the group consisting of F, Cl, Br, I, -CN, -CF₃, -SF₅, -OH, -I-C₁₋₅-alkyl, - NH₂, -NO₂, -O-CF₃, -S-CF₃, -SH, -S-C₁₋₅-alkyl, -C₁₋₅ alkyl, -C(=O)-OH, -C(=O)-O-C₁₋₅-alkyl, -NH-C₁₋₅-alkyl, -N(C₁₋₅-alkyl)₂, -NH-S(=O)₂-C₁₋₅-alkyl, -NH-C(=O)-O-C₁₋₅-alkyl, -C(=O)-H, -C(=O)-C₁₋₅-alkyl, -C(=O)-NH₂, -C(=O)-NH-C₁₋₅-alkyl, - C(=O)-N-(-C₁₋₅-alkyl)₂, -O-phenyl, -O-benzyl, phenyl and benzyl, wherein in each case the cyclic moiety of the residues -O-phenyl, -O-benzyl, phenyl and benzyl may be substituted with 1, 2, 3, 4 or 5 substituents mutually independently selected from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
and
the above-stated heteroaryl residues may in each case optionally comprise 1, 2, 3, 4 or 5 heteroatom(s) mutually independently selected from the group consisting of oxygen, nitrogen and sulfur as ring member(s).

2. Compounds according to claim 1, **characterised in that**
X denotes O;
n denotes 0, 1 or 2;
R¹, R², R³ and R⁴, mutually independently, in each case denote H; F; Cl; Br; I; -SF₅; -NO₂; -CN; -NH₂; -OH; -SH; -C(=O)-NH₂; -S(=O)₂-NH₂; -C(=O)-NH-OH; - C(=O)-OH; -C(=O)-H; -S(=O)₂-OH; -NHR¹⁰; -NR¹¹R¹²; -OR¹³; -SR¹⁴; -S(=O)-R²³; -S(=O)₂-R²⁴ or denote a residue selected from the group consisting of methyl, -CF₃, -CCl₃, -CBr₃, -CHF₂, -CH₂F, -CF₂Cl, -CCl₂F, ethyl, -CF₂-CH₃, - CH₂-CF₃, -C₂F₅, -CH₂-CCl₃, -CH₂-CBr₃, -CHF-CF₂Cl, -CF₂-CF₂Cl, -CFCI-CF₂Cl, n-propyl, -CF₂-CF₂-CF₃, -CF(CF₃)₂, isopropyl, n-butyl, sec.-butyl, isobutyl and tert.-butyl;
R⁵ denotes -NH₂; -NHR²⁵; -NR²⁶R²⁷; denotes an alkyl residue selected from the group consisting of -CF₃, -CH₂-CF₃, methyl, ethyl, n-propyl, isopropyl, tert.-butyl, n-butyl, sec.-butyl and isobutyl;
R⁶ denotes -C(=O)-R²⁸ or denotes a residue selected from the group consisting of methyl, -CF₃, -CCl₃, -CBr₃, -CHF₂, -CH₂F, -CF₂Cl, -CCl₂F, - CH₂-CN, -CH₂-O-CH₃, -CH₂-O-CF₃, -CH₂-SF₃, ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCI-CF₂Cl, -CH₂-CH₂-CN, n-propyl, -CF₂-CF₂-CF₃, -CF(CF₃)₂, isopropyl, - CH₂-CH₂-CH₂-CN, -CH₂-O-CH₂-CH₃, -CH₂-CH₂-SF₃, -CH₂-CH₂-OCF₃, - CH(CH₃)(O-CH₃), -CH(CH₃)(S-CH₃), n-butyl, -CF₂-CF₂-CF₂-CF₃, -CH₂-CH₂-CH₂-CH₂-CN, sec.-butyl, isobutyl, tert.-butyl, n-pentyl, 3-pentyl, n-heptyl, 4-heptyl, n-octyl, n-nonyl, 5-nonyl, (2,6)-dimethyl-hept-4-yl, 3-methylbutyl, n-hexyl, (3,3)-dimethylbutyl, -CH₂-CH₂-O-CH₃, -CH₂-CH₂-O-C₂H₅, -CH₂-CH₂-CH₂-O-CH₃, ethenyl, propenyl, 2-butenyl, 3-butenyl, 2-pentenyl and 3-pentenyl;
R⁷ and R⁸, mutually independently, in each case denote a hydrogen residue;
denote an alkyl residue selected from the group consisting of -CH₂-OH, -CH₂-CH₂-OH, -CH₂-CH₂-CH₂-OH, -CH₂-CH₂-CH₂-CH₂-OH, isopropyl, n-butyl, sec.-butyl, isobutyl, methyl, ethyl and n-propyl;
denote a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, thiazolyl, oxazolyl, isoxazolyl, pyridazinyl, pyrazinyl, pyrimidinyl, indazolyl, quinoxalinyl, quinolinyl and isoquinolinyl, which may in each case be attached via a -(CH₂)-, - (CH₂)₂- or-(CH₂)₃ group and/or may in each case be unsubstituted or optionally substituted with 1, 2, 3, 4 or 5 substituents mutually independently selected from the group consisting of F, Cl, Br, I, -CN, - CF₃, -SF₅, -OH, -O-CH₃, -O-C₂H₅, -O-CH(CH₃)₂, -O-C(CH₃)₃, -NH₂, - NO₂, -O-CF₃, -S-CF₃, -SH, -S-CH₃, -S-C₂H₅, -S-CH(CH₃)₂, -S-C(CH₃)₃, methyl, ethyl, n-propyl, isopropyl, n-butyl, sec.-butyl, isobutyl, tert.-butyl and n-pentyl;
or denote a residue selected from the group consisting of cyclopropyl, cyclobutyl, cyclopentyl, cyclohexyl and cycloheptyl;
providing that R⁷ and R⁸ do not in each case denote a hydrogen residue;
or
R⁷ and R⁸, in each case together with the carbon atom joining them together as a ring member, form a residue selected from the group consisting of cyclopropyl, cyclobutyl, cyclopentyl, cyclohexyl and cycloheptyl;
T denotes C-R²⁹ and U denotes C-R³⁰ and V denotes N and W denotes C-R³² or
T denotes C-R²⁹ and U denotes N and V denotes C-R³¹ and W denotes C-R³² or
T denotes N and U denotes C-R³⁰ and V denotes C-R³¹ and W denotes C-R³² or
T denotes N and U denotes N and V denotes C-R³¹ and W denotes C-R³² or
T denotes N and U denotes C-R³⁰ and V denotes N and W denotes C-R³² or
T denotes C-R²⁹ and U denotes N and V denotes N and W denotes C-R³²
R⁹ denotes F; Cl; Br; I; -SF₅; -OR¹³; -SR¹⁴; -S(=O)-R²³; -S(=O)₂-R²⁴;
denotes a residue selected from the group consisting of methyl, -CF₃, - CCl₃, -CBr₃, -CHF₂, -CH₂F, -CF₂Cl, -CCl₂F, -CH₂-CN, -CH₂-O-CH₃, - CH₂-O-CF₃, -CH₂-SF₃, ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, -CH₂-CH₂-CN, n-propyl, -CF₂-CF₂-CF₃, -CF(CF₃)₂, isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-butyl, -CF₂-CF₂-CF₂-CF₃, -CH₂-CH₂-CH₂-CH₂-CN, n-butyl, sec.-butyl, isobutyl, -C(CH₃)₂(CH₂OH) and tert.-butyl;
or denotes a residue selected from the group consisting of cyclopropyl, cyclobutyl, cyclopentyl, cyclohexyl, cycloheptyl, cyclopentenyl and cyclohexenyl, which may optionally in each case be substituted with 1, 2, 3, 4 or 5 substituents mutually independently selected from the group consisting of oxo (=O), thioxo (=S), -OH, -O-CH₃, -O-C₂H₅, -0-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, methyl, ethyl, n-propyl, isopropyl, n-butyl, sec.-butyl, isobutyl, tert.-butyl and n-pentyl;
R¹⁰, R¹¹, R¹², R¹³, R¹⁴, R²¹ and R²³ and R²⁴, mutually independently, in each case denote a residue selected from the group consisting of methyl, - CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-propyl, -CF₂-CF₂-CF₃, -CF(CF₃)₂, isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-butyl, -CF₂-CF₂-CF₂-CF₃, -CH₂-CH₂-CH₂-CH₂-CN, sec.-butyl, isobutyl, tert.-butyl, n-pentyl, 3-pentyl, n-heptyl, 4-heptyl, n-octyl, n-nonyl, 5-nonyl, (2,6)-dimethyl-hept-4-yl, 3-methylbutyl, n-hexyl, (3,3)-dimethylbutyl, -CH₂-CH₂-O-CH₃, -CH₂-CH₂-O-C₂H₅, -CH₂-CH₂-CH₂-O-CH₃, ethenyl, propenyl, 2-butenyl, 3-butenyl, 2-pentenyl and 3-pentenyl;
denote a residue selected from the group consisting of 2,3-dihydro-1H-indenyl, cyclopropyl, oxetanyl, cyclobutyl, cyclopentyl, cyclohexyl, imidazolidinyl, tetrahydrofuranyl, tetrahydrothiophenyl, pyrrolidinyl, piperidinyl, morpholinyl, piperazinyl, azepanyl, diazepanyl, azocanyl and thiomorpholinyl, which may in each case be attached via a -CH₂-O-, - CH₂-CH₂-O-, -CH₂-CH₂-O-CH₂-, -CH₂-CH(CH₃)-O-CH₂-, -(CH₂)-, - (CH₂)₂- or -(CH₂)₃- group and/or may in each case be unsubstituted or optionally substituted with 1, 2, 3, 4 or 5 substituents mutually independently selected from the group consisting of oxo (=O), thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, methyl, ethyl, n-propyl, isopropyl, n-butyl, sec.-butyl, isobutyl, tert.-butyl, n-pentyl, -C(=O)-CH₃, - C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂ and -C(=O)-O-C(CH₃)₃;
or denote a residue selected from the group consisting of phenyl, naphthyl, thiophenyl, furanyl, pyrrolyl, pyrazolyl, pyrazinyl, pyranyl, triazolyl, pyridinyl, imidazolyl, thiazolyl, oxazolyl and isoxazolyl, wherein the residue may in each case be attached via a -(CH₂)-, -(CH₂)₂- or-(CH₂)₃- group and/or may in each case be unsubstituted or optionally substituted with 1, 2, 3, 4 or 5 substituents mutually independently selected from the group consisting of F, Cl, Br, I, -CN, -CF₃, -SF₅, -OH, - O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, methyl, ethyl, n-propyl, isopropyl, n-butyl, sec.-butyl, isobutyl, tert.-butyl, n-pentyl, - C(=O)-OH, -C(=O)-O-CH₃, -C(=O)-O-C₂H₅, -C(=O)-O-CH(CH₃)₂, - C(=O)-O-C(CH₃)₃, -NH-CH₃, -NH-C₂H₅, -NH-C(CH₃)₃, -N(CH₃)₂, - N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-phenyl, -O-benzyl, phenyl and benzyl;
R²⁵, R²⁶ and R²⁷, mutually independently, in each case denote an alkyl residue selected from the group consisting of -CF₃, -CH₂-CF₃, methyl, ethyl, n-propyl, isopropyl, tert.-butyl, n-butyl, sec.-butyl and isobutyl;
R²⁸ denotes a residue selected from the group consisting of methyl, -CF₃, - CCl₃, -CBr₃, -CHF₂, -CH₂F, -CF₂Cl, -CH₂-CN, -CH₂-O-CH₃, -CH₂-O-CF₃, -CH₂-SF₃, ethyl, -CF₂-CH₃, -CH₂-CF₃, -C₂F₅, -CH₂-CCl₃, -CH₂-CBr₃, - CHF-CF₂Cl, -CF₂-CF₂Cl, -CFCl-CF₂Cl, -CH₂-CH₂-CN, n-propyl, isopropyl, sec.-butyl, isobutyl, tert.-butyl, n-pentyl, 3-pentyl, n-heptyl, 4-heptyl, n-octyl, n-nonyl, 5-nonyl, (2,6)-dimethyl-hept-4-yl, 3-methylbutyl, n-hexyl, (3,3)-dimethylbutyl, ethenyl, propenyl, 2-butenyl, 3-butenyl, 2-pentenyl and 3-pentenyl;
R²⁹, R³⁰ and R³¹, mutually independently, in each case denote H; F; Cl; Br; I; - SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR¹⁰; -NR¹¹R¹²; -OR¹³; - SR¹⁴; -C(=O)-OR²¹; -S(=O)-R²³; -S(=O)₂-R²⁴ ; denote a residue selected from the group consisting of -CH₂-OH, methyl, -CF₃, -CCl₃, -CBr₃, - CHF₂, -CH₂F, -CF₂Cl, -CCl₂F, ethyl, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-propyl, - CF₂-CF₂-CF₃, -CF(CF₃)₂, isopropyl, n-butyl, sec.-butyl, isobutyl and tert.-butyl or denote a phenyl residue, which may be unsubstituted or optionally substituted with 1, 2, 3, 4 or 5 substituents mutually independently selected from the group consisting of F, Cl, Br, I, -CN, - CF₃, -SF₅, -OH, -O-CH₃, -O-C₂H₅, -O-CH(CH₃)₂, -O-C(CH₃)₃, -NH₂, - NO₂, -O-CF₃, -S-CF₃, -SH, -S-CH₃, -S-C₂H₅, -S-CH(CH₃)₂, -S-C(CH₃)₃, methyl, ethyl, n-propyl, isopropyl, n-butyl, sec.-butyl, isobutyl, tert.-butyl and n-pentyl;
R³² denotes H; -SF₅; -NO₂; -CN; -NH₂; -OH; -SH; -C(=O)-NH₂; -S(=O)₂-NH₂; -C(=O)-NH-OH; -C(=O)-OH; -C(=O)-H; -S(=O)₂-OH; -NHR³³; -NR³⁴R³⁵; - OR³⁶; -SR³⁷; -C(=O)-OR⁴⁴; -S(=O)-R⁴⁶; -S(=O)₂-R⁴⁷; -C(=NH)-NH₂; - C(=NH)-NH-R⁴⁸; -N=C(NH₂)₂; -N=C(NHR⁴⁹)(NHR⁵⁰);
denotes a residue selected from the group consisting of cyclopropyl, cyclobutyl, cyclopentyl, cyclohexyl, cyclohexenyl, imidazolidinyl, tetrahydrofuranyl, tetrahydrothiophenyl, pyrrolidinyl, piperidinyl, morpholinyl, piperazinyl, azepanyl, diazepanyl, azocanyl and thiomorpholinyl, which is in each case attached via a carbon atom of the rings of the above-stated residues or via a -(CH=CH)-, -C=C- or -C=C-CH₂- group to the parent structure and may be unsubstituted or optionally in each case substituted with 1, 2, 3, 4 or 5 substituents mutually independently selected from the group consisting of -CN, - CH₂-N(CH₃)₂, -CH₂-N(C₂H₅)₂, -CH₂-NH-CH₃, -CH₂-NH-C₂H₅, -N-[C(=O)-C₂H₅]-phenyl, -N-[C(=O)-CH₃]-phenyl, oxo (=O), thioxo (=S), -OH, -0-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, methyl, ethyl, n-propyl, isopropyl, n-butyl, sec.-butyl, isobutyl, tert.-butyl, n-pentyl, -C(=O)-CH₃, -C(=O)-C₂H₅, -C(=O)-CH(CH₃)₂, -C(=O)-C(CH₃)₃, -C(=O)-OH, -C(=O)-O-CH₃, - C(=O)-O-C₂H₅, -C(=O)-O-CH(CH₃)₂ and -C(=O)-O-C(CH₃)₃;
or denotes a residue selected from the group consisting of (1,3)-benzodioxolyl, (1,4)-benzodioxanyl, tetrazolyl, (2,3)-dihydrothieno[3,4-b][1,4]dioxinyl, benzo[b]furanyl, phenyl, naphthyl, thiophenyl, furanyl, pyrrolyl, pyrazolyl, pyrazinyl, pyranyl, triazolyl, pyridinyl, imidazolyl, indolyl, isoindolyl, thiazolyl, oxazolyl, isoxazolyl, pyridazinyl, pyrazinyl, pyrimidinyl, indazolyl, quinoxalinyl, quinolinyl and isoquinolinyl, which may in each case be attached via a -(CH=CH)-, -C≡C-, -(CH₂)-, -(CH₂)₂-or -(CH₂)₃- group and/or may in each case be unsubstituted or optionally substituted with 1, 2, 3, 4 or 5 substituents mutually independently selected from the group consisting of F, Cl, Br, I, -CN, - CF₃, -SF₅, -OH, -O-CH₃, -O-C₂H₅, -O-CH(CH₃)₂, -O-C(CH₃)₃, -NH₂, - NO₂, -O-CF₃, -S-CF₃, -SH, -S-CH₃, -S-C₂H₅, -S-CH(CH₃)₂, -S-C(CH₃)₃, methyl, ethyl, n-propyl, isopropyl, n-butyl, sec.-butyl, isobutyl, tert.-butyl, n-pentyl, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, - N(CH₃)₂, -N(C₂H₅)₂, -N(CH₃)(C₂H₅), -NH-C(=O)-O-CH₃, -NH-S(=O)₂-CH₃, -NH-S(=O₂)-C₂H₅, -NH-S(=O)₂-CH(CH₃)₂, -NH-C(=O)-O-C₂H₅, - NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-phenyl, -O-benzyl, phenyl and benzyl;
R³³, R³⁴, R³⁵, R³⁶, R³⁷, R⁴⁴, R⁴⁶, R⁴⁷, R⁴⁸, R⁴⁹ and R⁵⁰, mutually independently, in each case
denote a residue selected from the group consisting of methyl, -CF₃, - CCl₃, -CBr₃, -CHF₂, -CH₂F, -CF₂Cl, -CCl₂F, -CH₂-CN, -CH₂-O-CH₃, - CH₂-O-CF₃, -CH₂-SF₃, ethyl, -CF₂-CH₃, -CH₂-CF₃, -C₂F₅, -CH₂-CCl₃, - CH₂-CBr₃, -CHF-CF₂Cl, -CF₂-CF₂Cl, -CFCl-CF₂Cl, -CH₂-CH₂-CN, n-propyl, -CF₂-CF₂-CF₃, -CF(CF₃)₂, isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-butyl, -CF₂-CF₂-CF₂-CF₃, -CH₂-CH₂-CH₂-CH₂-CN, sec.-butyl, isobutyl, tert.-butyl, n-pentyl, 3-pentyl, n-heptyl, 4-heptyl, n-octyl, n-nonyl, 5-nonyl, (2,6)-dimethyl-hept-4-yl, 3-methylbutyl, n-hexyl, (3,3)-dimethylbutyl, -CH₂-CH₂-O-CH₃, -CH₂-CH₂-O-C₂H₅, -CH₂-CH₂-CH₂-O-CH₃, ethenyl, propenyl, 2-butenyl, 3-butenyl, 2-pentenyl and 3-pentenyl;
denote a residue selected from the group consisting of 2,3-dihydro-1H-indenyl, cyclopropyl, oxetanyl, cyclobutyl, cyclopentyl, cyclohexyl, imidazolidinyl, tetrahydrofuranyl, tetrahydrothiophenyl, pyrrolidinyl, piperidinyl, morpholinyl, piperazinyl, azepanyl, diazepanyl, azocanyl and thiomorpholinyl, which may in each case be attached via a -CH₂-O-, - CH₂-CH₂-O-, -CH₂-CH₂-O-CH₂-, -CH₂-CH(CH₃)-O-CH₂-, -(CH₂)-, - (CH₂)₂- or -(CH₂)₃- group and/or may in each case be unsubstituted or optionally substituted with 1, 2, 3, 4 or 5 substituents mutually independently selected from the group consisting of oxo (=O), thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, methyl, ethyl, n-propyl, isopropyl, n-butyl, sec.-butyl, isobutyl, tert.-butyl, n-pentyl, -C(=O)-CH₃, - C(=O)-C₂H₅, -C(=O)-CH(CH₃)₂, -C(=O)-C(CH₃)₃, -C(=O)-OH, -C(=O)-O-CH₃, -C(=O)-O-C₂H₅, -C(=O)-O-CH(CH₃)₂ and -C(=O)-O-C(CH₃)₃;
or denote a residue selected from the group consisting of phenyl, naphthyl, thiophenyl, furanyl, pyrrolyl, pyrazolyl, pyrazinyl, pyranyl, triazolyl, pyridinyl, imidazolyl, thiazolyl, oxazolyl and isoxazolyl, wherein the residue may in each case be attached via a -(CH₂)-, -(CH₂)₂- or - (CH₂)₃- group and/or may in each case be unsubstituted or optionally substituted with 1, 2, 3, 4 or 5 substituents mutually independently selected from the group consisting of F, Cl, Br, I, -CN, -CF₃, -SF₅, -OH, - O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, methyl, ethyl, n-propyl, isopropyl, n-butyl, sec.-butyl, isobutyl, tert.-butyl, n-pentyl, - C(=O)-OH, -C(=O)-O-CH₃, -C(=O)-O-C₂H₅, -C(=O)-O-CH(CH₃)₂, - C(=O)-O-C(CH₃)₃, -NH-CH₃, -NH-C₂H₅, -NH-C(CH₃)₃, -N(CH₃)₂, - N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-phenyl, -O-benzyl, phenyl and benzyl;
or
R³⁴ and R³⁵ in each case together with the nitrogen atom joining them together as a ring member form a residue selected from the group consisting of 3-aza-bicyclo[3.1.1]heptyl, 6-aza-spiro[2.5]octyl, 3-aza-bicyclo[3.2.1]octyl, 6-aza-bicyclo[3.3.1]heptyl, 8-aza-bicyclo[3.2.1]octyl, 1-oxa-2,8-diaza-spiro[4.5]dec-2-enyl, azocanyl, isoindolyl, indolyl, (1,2,3,6)-tetrahydropyridinyl, (4,5,6,7)-tetrahydroisoxazolo[5,4-c]pyridinyl, pyrrolidinyl, piperidinyl, piperazinyl, morpholinyl, azepanyl, diazepanyl and thiomorpholinyl, the heterocycloaliphatic moiety of which may in each case be unsubstituted or substituted with 1, 2, 3, 4 or 5 residues R⁵¹;
R⁵¹ denotes -NHR⁵², -NR⁵³R⁵⁴ or denotes an alkyl residue selected from the group consisting of -CF₃, -CH₂-CF₃, methyl, ethyl, n-propyl, isopropyl, tert.-butyl, n-butyl, sec.-butyl and isobutyl;
R⁵², R⁵³ and R⁵⁴, mutually independently, in each case denote-C(=O)-R⁵⁵; denote an alkyl residue selected from the group consisting of -CF₃, - CH₂-CF₃, methyl, ethyl, n-propyl, isopropyl, tert.-butyl, n-butyl, sec.-butyl, and isobutyl;
or denote a residue selected from the group consisting of phenyl, naphthyl, thiophenyl, furanyl, pyrrolyl, pyrazolyl, pyrazinyl, pyranyl, triazolyl, pyridinyl, imidazolyl, thiazolyl, oxazolyl and isoxazolyl, wherein the residue may in each case be attached via a -(CH₂)-, -(CH₂)₂- or - (CH₂)₃- group and/or may in each case be unsubstituted or optionally substituted with 1, 2, 3, 4 or 5 substituents mutually independently selected from the group consisting of F, Cl, Br, I, -CN, -CF₃, -SF₅, -OH, - O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, methyl, ethyl, n-propyl, isopropyl, n-butyl, sec.-butyl, isobutyl, tert.-butyl, n-pentyl, - C(=O)-OH, -C(=O)-O-CH₃, -C(=O)-O-C₂H₅, -C(=O)-O-CH(CH₃)₂, - C(=O)-O-C(CH₃)₃, -NH-CH₃, -NH-C₂H₅, -NH-C(CH₃)₃, -N(CH₃)₂, - N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -O-phenyl, -O-benzyl, phenyl and benzyl;
and
R⁵⁵ denotes an alkyl residue selected from the group consisting of -CF₃, - CH₂-CF₃, methyl, ethyl, n-propyl, isopropyl, tert.-butyl, n-butyl, sec.-butyl, and isobutyl;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

3. Compounds according to claim 1 or claim 2, **characterised in that**
X denotes O;
n denotes 1;
R¹, R³ and R⁴, mutually independently, in each case denote H; F; Cl; Br; I; - NHR¹⁰; --NR¹¹R¹²; -OR¹³; -SR¹⁴ or denote a residue selected from the group consisting of methyl, -CF₃, -CHF₂, -CH₂F, ethyl, n-propyl, -CF₂-CF₂-CF₃, -CF(CF₃)₂, isopropyl, n-butyl, sec.-butyl, isobutyl and tert.-butyl;
R² denotes H; F; Cl; Br; I or methyl;
R⁵ denotes an alkyl residue selected from the group consisting of -CF₃, - CH₂-CF₃, methyl, ethyl, n-propyl, isopropyl, tert.-butyl, n-butyl, sec.-butyl, and isobutyl;
R⁶ denotes -C(=O)-R²⁸ or denotes a residue selected from the group consisting of methyl, -CH₂-CN, ethyl, -CH₂-CH₂-CN, n-propyl, -CH₂-CH₂-CH₂-CN, n-butyl, -CH₂-CH₂-CH₂-CH₂-CN, sec.-butyl, isobutyl, tert.-butyl, n-pentyl, 3-pentyl, n-heptyl, ethenyl, propenyl, 2-butenyl, 3-butenyl, 2-pentenyl and 3-pentenyl;
R⁷ denotes an alkyl residue selected from the group consisting of -CH₂-OH, -CH₂-CH₂-OH, -CH₂-CH₂-CH₂-OH, -CH₂-CH₂-CH₂-CH₂-OH, isopropyl, n-butyl, sec.-butyl, isobutyl, methyl, ethyl and n-propyl;
or denotes a residue selected from the group consisting of cyclopropyl, cyclobutyl, cyclopentyl, cyclohexyl and cycloheptyl;
R⁸ denotes a hydrogen residue;
or
R⁷ and R⁸, in each case together with the carbon atom joining them together as a ring member, form a residue selected from the group consisting of cyclopropyl, cyclobutyl, cyclopentyl, cyclohexyl and cycloheptyl;
T denotes C-R²⁹ and U denotes C-R³⁰ and V denotes N and W denotes C-R³²
R⁹ denotes F; Cl; Br; I; -SF₅; -O-CF₃; -O-CCl₃; -O-CBr₃; -O-CHF₂; -O-CH₂F; -O-CF₂Cl; -O-CCl₂F; -O-CF₂-CH₃; -S-CF₃; -S-CCl₃; -S-CBr₃; -S-CHF₂; - S-CH₂F; -S-CF₂Cl; -S-CCl₂F; -S-CF₂-CH₃; or denotes a residue selected from the group consisting of methyl, -CF₃, -CHF₂, -CH₂F, ethyl, n-propyl, isopropyl, n-butyl, sec.-butyl, isobutyl, -C(CH₃)₂(CH₂OH) and tert.-butyl;
R¹⁰, R¹¹, R¹², R¹³ and R¹⁴, mutually independently, in each case
denote a residue selected from the group consisting of methyl, -CF₃, - CHF₂, -CH₂F, ethyl, n-propyl, isopropyl, n-butyl, sec.-butyl, isobutyl, tert.-butyl and n-pentyl;
R²⁸ denotes a residue selected from the group consisting of methyl, ethyl, n-propyl, isopropyl, sec.-butyl, isobutyl, tert.-butyl, n-pentyl, 3-pentyl, n-heptyl, 4-heptyl, n-octyl, n-nonyl, 5-nonyl, (2,6)-dimethyl-hept-4-yl, 3-methylbutyl, n-hexyl, (3,3)-dimethylbutyl, ethenyl, propenyl, 2-butenyl, 3-butenyl, 2-pentenyl and 3-pentenyl;
R²⁹, R³⁰ and R³¹, mutually independently, in each case denote H; F; Cl; Br; I; - SF₅; -NO₂; -CN; -NH₂; -OH; -SH; -NHR¹⁰; -NR¹¹R¹²; -OR¹³; -SR¹⁴; or denote a residue selected from the group consisting of -CH₂-OH, methyl, -CF₃, ethyl, n-propyl, isopropyl, n-butyl, sec.-butyl, isobutyl and tert.-butyl;
R³² denotes H; -SF₅; -NO₂; -CN; -NH₂; -OH; -; -NHR³³; -NR³⁴R³⁵; -OR³⁶; SR³⁷;
denotes a residue selected from the group consisting of cyclopropyl, cyclobutyl, cyclopentyl, cyclohexyl, cyclohexenyl, imidazolidinyl, tetrahydrofuranyl, tetrahydrothiophenyl, pyrrolidinyl, piperidinyl, morpholinyl, piperazinyl, azepanyl, diazepanyl, azocanyl and thiomorpholinyl, which is in each case attached via a carbon atom of the rings of the above-stated residues to the parent structure and may be unsubstituted or optionally in each case substituted with 1, 2 , 3, 4 or 5 substituents mutually independently selected from the group consisting of -CH₂-N(CH₃)₂, -CH₂-N(C₂H₅)₂, -CH₂-NH-CH₃, -CH₂-NH-C₂H₅, -N-[C(=O)-C₂H₅]-phenyl, -N-[C(=O)-CH₃]-phenyl, methyl, ethyl, n-propyl, isopropyl, n-butyl, sec.-butyl, isobutyl and tert.-butyl;
or denotes a residue selected from the group consisting of phenyl, thiophenyl, furanyl, pyrrolyl, pyrazolyl, pyrazinyl, pyranyl, triazolyl, pyridinyl, imidazolyl, thiazolyl, oxazolyl, isoxazolyl, pyridazinyl, pyrazinyl and pyrimidinyl, which may in each case optionally be substituted with 1, 2, 3, 4 or 5 substituents mutually independently selected from the group consisting of F, Cl, Br, I, -CN, -CF₃, -SF₅, -OH, -O-CH₃, -O-C₂H₅, - O-CH(CH₃)₂, -O-C(CH₃)₃, -O-CF₃, methyl, ethyl, n-propyl, isopropyl, n-butyl, sec.-butyl, isobutyl, tert.-butyl and n-pentyl;
R³³, R³⁴, R³⁵, R³⁶ and R³⁷, mutually independently, in each case
denote a residue selected from the group consisting of methyl, -CH₂-O-CH₃, ethyl, n-propyl, n-butyl, sec.-butyl, isobutyl, tert.-butyl, n-pentyl, 3-pentyl, n-hexyl, (3,3)-dimethylbutyl, -CH₂-CH₂-O-CH₃, -CH₂-CH₂-O-C₂H₅ and -CH₂-CH₂-CH₂-O-CH₃;
denote a residue selected from the group consisting of 2,3-dihydro-1H-indenyl, cyclopropyl, oxetanyl, cyclobutyl, cyclopentyl, cyclohexyl, imidazolidinyl, tetrahydrofuranyl, tetrahydrothiophenyl, pyrrolidinyl, piperidinyl, morpholinyl, piperazinyl, azepanyl, diazepanyl, azocanyl and thiomorpholinyl, which may in each case optionally be substituted with 1, 2, 3, 4 or 5 substituents mutually independently selected from the group consisting of methyl, ethyl, n-propyl, isopropyl, n-butyl, sec.-butyl, isobutyl, tert.-butyl, n-pentyl;
or
R³⁴ and R³⁵ in each case together with the nitrogen atom joining them together as a ring member form a residue selected from the group consisting of 3-aza-bicyclo[3.1.1]heptyl, 6-aza-spiro[2.5]octyl, 3-aza-bicyclo[3.2.1]octyl, 6-aza-bicyclo[3.3.1]heptyl, 8-aza-bicyclo[3.2.1]octyl, 1-oxa-2,8-diaza-spiro[4.5]dec-2-enyl, azocanyl, isoindolyl, indolyl, (1,2,3,6)-tetrahydropyridinyl, (4,5,6,7)-tetrahydroisoxazolo[5,4-c]pyridinyl, pyrrolidinyl, piperidinyl, piperazinyl, morpholinyl, azepanyl, diazepanyl and thiomorpholinyl, the heterocycloaliphatic moiety of which may in each case be unsubstituted or substituted with 1, 2, 3, 4 or 5 residues R⁵¹;
R⁵¹ denotes -NHR⁵², -NR⁵³R⁵⁴ or denotes an alkyl residue selected from the group consisting of -CF₃, -CH₂-CF₃, methyl, ethyl, n-propyl, isopropyl, tert.-butyl, n-butyl, sec.-butyl and isobutyl;
R⁵², R⁵³ and R⁵⁴, mutually independently, in each case denote-C(=O)-R⁵⁵; denote an alkyl residue selected from the group consisting of -CF₃, - CH₂-CF₃, methyl, ethyl, n-propyl, isopropyl, tert.-butyl, n-butyl, sec.-butyl and isobutyl;
or denote a residue selected from the group consisting of phenyl and naphthyl, wherein the residue may in each case be attached via a - (CH₂)-, -(CH₂)₂- or -(CH₂)₃- group and/or in each case unsubstituted or optionally substituted with 1, 2, 3, 4 or 5 substituents mutually independently selected from the group consisting of F, Cl, Br, I, -CN, - CF₃, -O-CH₃, -O-C₂H₅, -O-CH(CH₃)₂, -O-C(CH₃)₃, methyl, ethyl, n-propyl, isopropyl, n-butyl, sec.-butyl, isobutyl, tert.-butyl and n-pentyl;
and
R⁵⁵ denotes an alkyl residue selected from the group consisting of -CF₃, - CH₂-CF₃, methyl, ethyl, n-propyl, isopropyl, tert.-butyl, n-butyl, sec.-butyl, and isobutyl;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

4. Compounds according to one or more of claims 1 to 3, **characterised in that**
X denotes O;
n denotes 1;
R¹, R³ and R⁴ in each case denote H;
R² denotes F; Cl or Br;
R⁵ denotes an alkyl residue selected from the group consisting of methyl, ethyl, n-propyl and isopropyl;
R⁶ denotes -C(=O)-R²⁸ or denotes a residue selected from the group consisting of methyl, -CH₂-CN, ethyl, -CH₂-CH₂-CN, n-propyl, -CH₂-CH₂-CH₂-CN, n-butyl, -CH₂-CH₂-CH₂-CH₂-CN, sec.-butyl, isobutyl, tert.-butyl and n-pentyl;
R⁷ denotes an alkyl residue selected from the group consisting of -CH₂-OH, -CH₂-CH₂-OH, -CH₂-CH₂-CH₂-OH, -CH₂-CH₂-CH₂-CH₂-OH, isopropyl, n-butyl, sec.-butyl, isobutyl, methyl, ethyl and n-propyl;
R⁸ denotes a hydrogen residue;
T denotes C-R²⁹ and U denotes C-R³⁰ and V denotes N and W denotes C-R³²
R⁹ denotes -SF₅; -O-CF₃; -CF₃; -CHF₂; -CH₂F; -C(CH₃)₂(CH₂OH) or tert.-butyl;
R²⁸ denotes a residue selected from the group consisting of methyl, ethyl, n-propyl, isopropyl, sec.-butyl, isobutyl, tert.-butyl, ethenyl and propenyl;
R²⁹, R³⁰ and R³¹ in each case denote H;
R³² denotes H; -NHR³³; -NR³⁴R³⁵; -OR³⁶ or -SR³⁷;
R³³, R³⁶ and R³⁷, mutually independently, in each case
denote a residue selected from the group consisting of cyclopropyl, cyclobutyl, cyclopentyl, cyclohexyl and cycloheptyl, which may in each case optionally be substituted with 1, 2, 3, 4 or 5 substituents mutually independently selected from the group consisting of methyl, ethyl, n-propyl, isopropyl, n-butyl, sec.-butyl, isobutyl, tert.-butyl, n-pentyl;
R³⁴ and R³⁵ in each case together with the nitrogen atom joining them together as a ring member form a residue selected from the group consisting of pyrrolidinyl, piperidinyl, piperazinyl, morpholinyl and azepanyl, the heterocycloaliphatic moiety of which may in each case be unsubstituted or substituted with 1, 2, 3, 4 or 5 residues R⁵¹;
R⁵¹ denotes -NHR⁵², -NR⁵³R⁵⁴ or denotes an alkyl residue selected from the group consisting of methyl, ethyl, n-propyl, isopropyl, tert.-butyl, n-butyl, sec.-butyl and isobutyl;
R⁵², R⁵³ and R⁵⁴, mutually independently, in each case denote -C(=O)-R⁵⁵; denote an alkyl residue selected from the group consisting of methyl, ethyl, n-propyl, isopropyl, tert.-butyl, n-butyl, sec.-butyl, and isobutyl;
or denote a phenyl residue, wherein the residue may be substituted with 1, 2, 3, 4 or 5 substituents mutually independently selected from the group consisting of F, Cl, Br, methyl, ethyl, n-propyl and isopropyl;
and
R⁵⁵ denotes an alkyl residue selected from the group consisting of methyl, ethyl, n-propyl, isopropyl, tert.-butyl, n-butyl, sec.-butyl, and isobutyl;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

5. Compounds of the general formula Ia, according to one or more of claims 1 to 4, 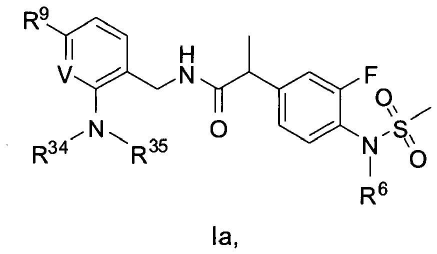 in which
V denotes N;
and R⁶, R⁹, R³⁴ and R³⁵ have the meaning according to claim 3;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

6. Compounds of the general formula Ia, according to one or more of claims 1 to 5, 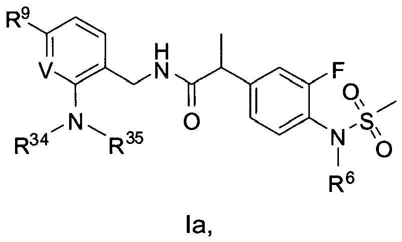 in which
V denotes N;
and R⁶, R⁹, R³⁴ and R³⁵ have the meaning according to claim 4;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

7. Compounds of the general formula Ib, according to one or more of claims 1 to 4, 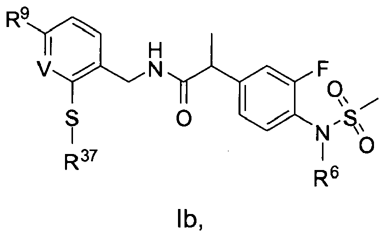 V denotes N;
and R⁶, R⁹ and R³⁷ have the meaning according to claim 3;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

8. Compounds of the general formula Ib, according to one or more of claims 1 to 7, 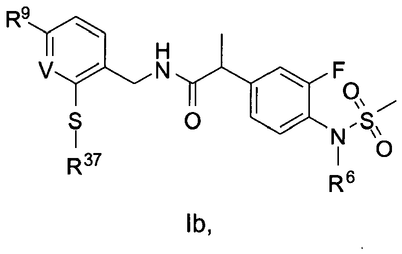 V denotes N;
and R⁶, R⁹ and R³⁷ have the meaning according to claim 4;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

9. Compounds according to one or more of claims 1 to 8 selected from the group consisting of
[1] N-(2-fluoro-4-(1-oxo-1-((2-(4-(N-pheny)propionamido)piperidin-1-yl)-6-(trifluoromethyl)pyridin-3-yl)methylamino)propan-2-yl)phenyl)-N-(methylsulfonyl)propionamide,
[2] 2-(3-fluoro-4-(N-(methylsulfonyl)acetamido)phenyl)-N-((2-(4-methylpiperidin-1-yl)-6-(trifluoromethyl)pyridin-3-yl)methyl)propanamide,
[3] 2-(3-fluoro-4-(N-methylmethylsulfonamido)phenyl)-N-((2-(4-methylpiperidin-1-yl)-6-(trifluoromethyl)pyridin-3-yl)methyl)propanamide,
[7] N-((2-(cyclohexylthio)-6-(trifluoromethyl)pyridin-3-yl)methyl)-2-(3-fluoro-4-(N-methylmethylsulfonamido)phenyl)propanamide,
[8] N-((2-(cyclohexylthio)-6-(trifluoromethyl)pyridin-3-yl)methyl)-2-(4-(N-ethylmethylsulfonamido)-3-fluorophenyl)propanamide,
[9] 2-(4-(N-ethylmethylsulfonamido)-3-fluorophenyl)-N-((2-(4-methylpiperidin-1-yl)-6-(trifluoromethyl)pyridin-3-yl)methyl)propanamide,
[10] N-((6-tert.-butyl-2-(4-methylpiperidin-1-yl)pyridin-3-yl)methyl)-2-(3-fluoro-4-(N-methylmethylsulfonamido)phenyl)propanamide,
[11] N-((6-tert.-butyl-2-(cyclohexylthio)pyridin-3-yl)methyl)-2-(3-fluoro-4-(N-methylmethylsulfonamido)phenyl)propanamide,
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

10. A method for producing a compound according to one or more of claims 1 to 9, **characterised in that** at least one compound of the general formula II, 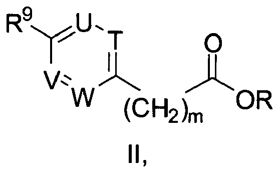 in which R⁹, U, T, V, and W have the meaning according to one or more of claims 1 to 9, m denotes 0, 1, 2 or 3 and R denotes hydrogen or denotes a linear or branched C₁₋₆ alkyl residue, is reacted in a reaction medium, in the presence of at least one reducing agent to yield at least one compound of the general formula III, 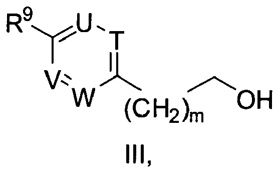 in which R⁹, U, T, V and W have the meaning according to one or more of claims 1 to 9 and m denotes 0, 1, 2 or 3,
and at least one compound of the general formula III is reacted in a reaction medium in the presence of diphenylphosphoryl azide or in the presence of HN₃ to yield at least one compound of the general formula IV, 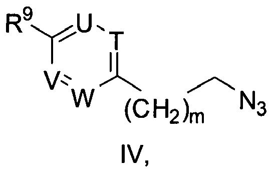 in which R⁹, U, T, V and W have the meaning according to one or more of claims 1 to 9 and m denotes 0, 1, 2 or 3,
and at least one compound of the general formula IV is reacted in a reaction medium in the presence of at least one reducing agent or in a reaction medium in the presence of a catalyst and in the presence of hydrogen or in the presence of hydrazine
or in a reaction medium in the presence of triphenylphosphine
to yield at least one compound of the general formula V, 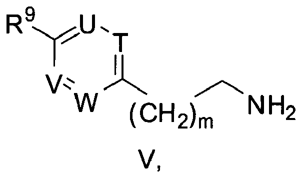 in which R⁹, U, T, V and W have the meaning according to one or more of claims 1 to 9 and m denotes 0, 1, 2 or 3,
or at least one compound of the general formula VI, 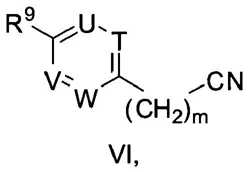 in which R⁹, U, T, V and W have the meaning according to one or more of claims 1 to 9 and m denotes 0, 1, 2 or 3, is reacted in a reaction medium
in the presence of at least one catalyst under a hydrogen atmosphere,
or in the presence of at least one reducing agent selected from the group consisting of BH₃·S(CH₃)₂, lithium aluminium hydride and sodium borohydride,
to yield at least one compound of the general formula V, optionally in the form of a corresponding salt,
and at least one compound of the general formula V is reacted with at least one compound of the general formula VII, 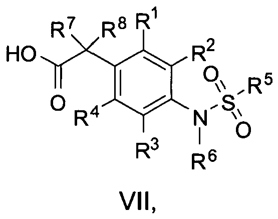 in which R¹, R², R³, R⁴, R⁵, R⁶, R⁷ and R⁸ have the meaning according to one or more of claims 1 to 9 have and R⁶ may additionally denote a hydrogen residue, in a reaction medium,
or with at least one compound of the general formula VIII, 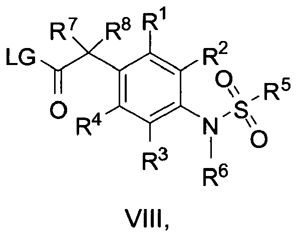 in which R¹, R², R³, R⁴, R⁵, R⁶, R⁷ and R⁸ have the meaning according to one or more of claims 1 to 9, R⁶ may additionally denote a hydrogen residue and LG denotes a leaving group, in a reaction medium to yield at least one compound of the general formula Ih, 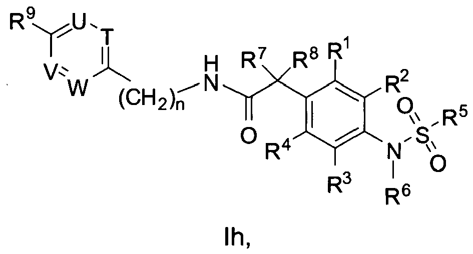 in which T, U, V, W, R¹, R², R³, R⁴, R⁵, R⁶, R⁷, R⁸ and R⁹ have the meaning according to one or more of claims 1 to 9, R⁶ may additionally denote a hydrogen residue and n denotes 1, 2, 3 or 4,
and optionally at least one compound of the general formula Ih is reacted in a reaction medium with at least one compound of the general formula IX, 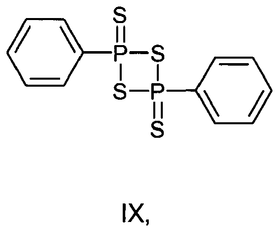 in which the phenyl residues are in each case substituted with 1 or 2 substituents mutually independently selected from the group consisting of methoxy, phenoxy, Cl, methyl and Br, or with phosphorus pentasulfide, to yield at least one compound of the general formula Ik, 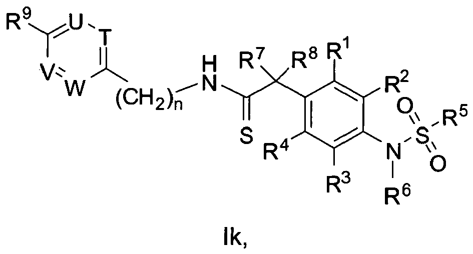 in which T, U, V, W, R¹, R², R³, R⁴, R⁵, R⁶, R⁷, R⁸ and R⁹ have the meaning according to one or more of claims 1 to 9, R⁶ may additionally denote a hydrogen residue and n denotes 1, 2, 3 or 4;
and optionally at least one compound of the general formula Io, 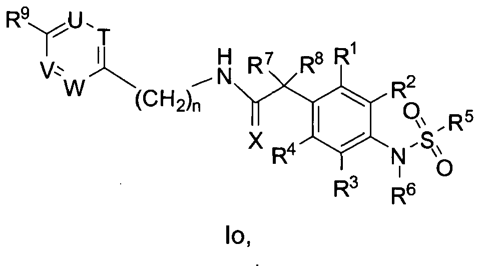 in which X, T, U, V, W, R¹, R², R³, R⁴, R⁵, R⁷, R⁸ and R⁹ have the meaning according to one or more of claims 1 to 9, R⁶ denotes a hydrogen residue and n denotes 1, 2, 3 or 4, is reacted in a reaction medium in the presence of at least one compound of the general formula R²⁸-C(=O)-O-C(=O)-R²⁸, in which R²⁸ has the meaning according to one or more of claims 1 to 9, to yield at least one compound of the general formula I, in which X, T, U, V, W, R¹, R², R³, R⁴, R⁵, R⁷, R⁸ and R⁹ have the meaning according to one or more of claims 1 to 9, R⁶ denotes -C(=O)-R²⁸ and n denotes 1, 2, 3 or 4.

11. A method for producing at least one compound according to one or more of claims 1 to 9, **characterised in that** at least one compound of the general formula X, 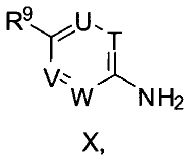 in which R⁹, U, T, V and W have the meaning according to one or more of claims 1 to 9, is reacted with at least one compound of the general formula VII, 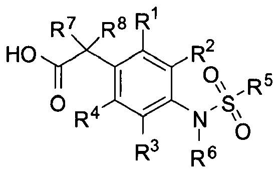 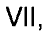 in which R¹, R², R³, R⁴, R⁵, R⁶, R⁷ and R⁸ have the meaning according to one or more of claims 1 to 9 and R⁶ may additionally denote a hydrogen residue, in a reaction medium,
or with at least one compound of the general formula VIII, 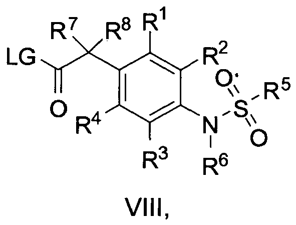 in which R¹, R², R³, R⁴, R⁵, R⁶, R⁷ and R⁸ have the meaning according to one or more of claims 1 to 9, R⁶ may additionally denote a hydrogen residue and LG denotes a leaving group, in a reaction medium to yield at least one compound of the general formula Im, 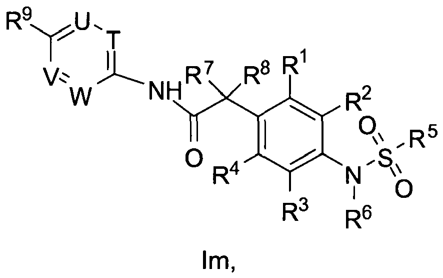 in which T, U, V, W, R¹, R², R³, R⁴, R⁵, R⁷, R⁸ and R⁹ have the meaning according to one or more of claims 1 to 9,
and optionally at least one compound of the general formula Im is reacted in a reaction medium with at least one compound of the general formula IX, 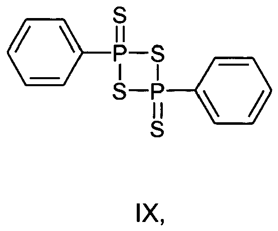 in which the phenyl residues are in each case substituted with 1 or 2 substituents mutually independently selected from the group consisting of methoxy, phenoxy, Cl, methyl and Br, or with phosphorus pentasulfide, to yield at least one compound of the general formula In, 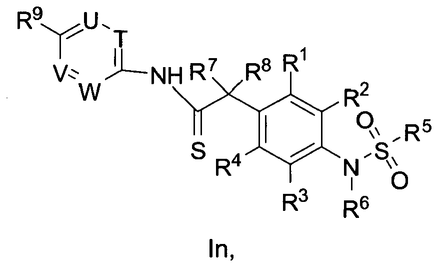 in which T, U, V, W, R¹, R², R³, R⁴, R⁵, R⁶, R⁷, R⁸ and R⁹ have the meaning according to one or more of claims 1 to 9 and R⁶ may additionally denote a hydrogen residue;
and optionally at least one compound of the general formula Ip, 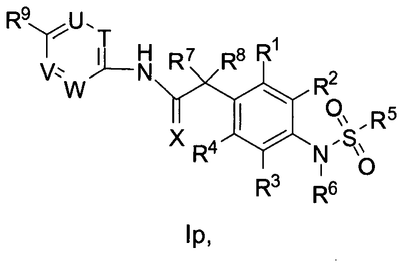 in which X, T, U, V, W, R¹, R², R³, R⁴, R⁵, R⁷, R⁸ and R⁹ have the meaning according to one or more of claims 1 to 9 and R⁶ denotes a hydrogen residue, is reacted in a reaction medium in the presence of at least one compound of the general formula R²⁸-C(=O)-O-C(=O)-R²⁸, in which R²⁸ has the meaning according to one or more of claims 1 to 9, to yield at least one compound of the general formula I, in which X, T, U, V, W, R¹, R², R³, R⁴, R⁵, R⁷, R⁸ and R⁹ have the meaning according to one or more of claims 1 to 9 and R⁶ denotes - C(=O)-R²⁸.

12. A medicament containing at least one compound according to one or more of claims 1 to 9 and optionally one or more physiologically acceptable auxiliary substances.

13. Use of at least one compound according to one or more of claims 1 to 9 for the production of a medicament for the treatment and/or prevention of one or more diseases selected from the group consisting of pain; joint pain; hyperalgesia; allodynia; causalgia and migraine.

14. Use of at least one compound according to one or more claims 1 to 9 for the production of a medicament for the treatment and/or prevention of one or more diseases selected from the group consisting of depression; neuropathy; nerve injury; neurodegenerative diseases; cognitive dysfunction; and epilepsy.

15. Use of at least one compound according to one or more of claims 1 to 9 for the production of a medicament for the treatment and/or prevention of one or more diseases selected from the group consisting of airways diseases; coughing; urinary incontinence; an overactive bladder (OAB); diseases and/or injuries of the gastrointestinal tract; duodenal ulcers; gastric ulcers; irritable bowel syndrome; strokes; eye irritation; skin irritation; neurotic skin conditions; allergic skin diseases; psoriasis; vitiligo; herpes simplex; inflammation, preferably inflammation of the intestines, the eyes, the bladder, the skin or the nasal mucosa; diarrhoea; pruritus; osteoporosis; arthritis; osteoarthritis; rheumatic diseases; disorders of food intake; dependency on medicines; abuse of medicines; withdrawal symptoms associated with dependency on medicines; development of tolerance towards medicines; dependency on drugs; drug abuse; withdrawal symptoms associated with dependency on drugs; dependency on alcohol; alcohol abuse and withdrawal symptoms associated with dependency on alcohol; for diuresis; for antinatriuresis; for influencing the cardiovascular system; for increasing vigilance; for the treatment of wounds and/or bums; for the treatment of severed nerves; for increasing libido; for modulating locomotor activity; for anxiolysis; for local anaesthesia and/or for inhibiting undesired side-effects triggered by the administration of vanilloid receptor 1 (VR1/TRPV1 receptor) agonists.

## Revendications

1. Composés substitués de formule générale I, 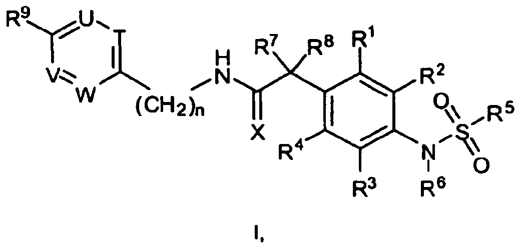 dans laquelle
X représente 0, S ou N-C≡N ;
n vaut 0, 1, 2, 3 ou 4 ;
R¹, R², R³ et R⁴ représentent, indépendamment les uns des autres, à chaque fois H ; F ; Cl ; Br ; I ; -SF₅ ; -NO₂ ; -CN ; -NH₂ ; -OH ; -SH ; -C(=O)-NH₂ ; -S(=O)₂-NH₂ ; -C(=O)-NH-OH ; -C(=O)-OH ; -C(=O)-H ; -S(=O)₂-OH ; -NHR¹⁰ ; -NR¹¹R¹²; -OR¹³; -SR¹⁴ ; -C(=O)-NHR¹⁵ ; -C(=O)-NR¹⁶R¹⁷ -S(=O)₂-NHR¹⁸ ; -S(=O)₂-NR¹⁹R²⁰ ; -C(=O)-OR²¹ ; -C(=O)-R²² ; -S (=O)-R²³ ; -S(=O)₂-R²⁴ ou un radical aliphatique en C₁₋₁₀, linéaire ou ramifié, saturé ou insaturé, non substitué ou au moins monosubstitué ;
R⁵ représente -NH₂ ; -NHR²⁵ ; -NR²⁶R²⁷ ou un radical aliphatique en C₁₋₁₀, linéaire ou ramifié, saturé ou insaturé, non substitué ou au moins monosubstitué ;
R⁶ représente -C(=O)-R²⁸ ou un radical aliphatique en C₁₋₁₀, linéaire ou ramifié, saturé ou insaturé, non substitué ou au moins monosubstitué ;
R⁷ et R⁸ représentent, indépendamment l'un de l'autre, à chaque fois un radical hydrogène ;
un radical aliphatique en C₁₋₁₀ linéaire ou ramifié, saturé ou insaturé, non substitué ou au moins monosubstitué ;
un radical aryle ou hétéroaryle de 5 à 14 chaînons, non substitué ou au moins monosubstitué, qui peut être condensé avec un système cyclique monocyclique ou polycyclique saturé ou insaturé,
non substitué ou au moins monosubstitué et/ou lié via un groupe C₁₋₆-alkylène ou un groupe C₂₋₆-alcénylène ou un groupe C₂₋₆-alcynylène linéaire ou ramifié, non substitué ou au moins monosubstitué ; ou un radical cycloaliphatique de 3, 4, 5, 6, 7, 8 ou 9 chaînons, insaturé ou saturé, non substitué ou au moins monosubstitué, présentant le cas échéant au moins un hétéroatome comme élément de cycle ;
à condition que R⁷ et R⁸ ne représentent pas tous les deux à chaque fois un radical hydrogène ; ou
R⁷ et R⁸ représentent à chaque fois ensemble avec l'atome de carbone qui les relie comme élément de cycle un radical cycloaliphatique de 3, 4, 5 ou 6 chaînons, saturé ou insaturé, non substitué ou au moins monosubstitué ;
T représente C-R²⁹ et U représente C-R³⁰ et V représente N et W représente C-R³² ou
T représente C-R²⁹ et U représente N et V représente C-R³¹ et W représente C-R³² ou
T représente N et U représente C-R³⁰ et V représente C-R³¹ et W représente C-R³² ou
T représente N et U représente N et V représente C-R³¹ et W représente C-R³² ou
T représente N et U représente C-R³⁰ et V représente N et W représente C-R³² ou
T représente C-R²⁹ et U représente N et V représente N et W représente C-R³² ;
R⁹ représente F ; Cl ; Br ; I ; -SF₅ ; -NO₂ ; -CF₃ ; -CN ; -NH₂ ; -OH ; -SH ; -C(=O)-NH₂ ; -S(=O)₂-NH₂ ; -C(=O)-NH-OH; -C(=O)-OH ; -C(=O)-H ; -S(=O)₂-OH ; -NHR¹⁰ ; -NR¹¹R¹², -OR¹³ ; -SR¹⁴ ; -C(=O)-NHR¹⁵ ; -C(=O)-NR¹⁶R¹⁷; -S(=O)₂-NHR¹⁸ ; -S (=O)₂-NR¹⁹R²⁰ ; -C(=O)-OR²¹ ; -C(=O)-R²² ; -S(=O)-R²³ ; -S(=O)₂-R²⁴ ; un radical aliphatique en C₁₋₁₀ linéaire ou ramifié, saturé ou insaturé, non substitué ou au moins monosubstitué ;
ou un radical cycloaliphatique de 3, 4, 5, 6, 7, 8 ou 9 chaînons, insaturé ou saturé, non substitué ou au moins monosubstitué, présentant le cas échéant au moins un hétéroatome comme élément de cycle ;
R¹⁰, R¹¹, R¹², R¹³, R¹⁴, R¹⁵, R¹⁶, R¹⁷, R¹⁸, R¹⁹, R²⁰, R²¹, R²², R²³ et R²⁴ représentent, indépendamment les uns des autres, à chaque fois
un radical aliphatique en C₁₋₁₀ linéaire ou ramifié, saturé ou insaturé, non substitué ou au moins monosubstitué ;
un radical cycloaliphatique de 3, 4, 5, 6, 7, 8 ou 9 chaînons, insaturé ou saturé, non substitué ou au moins monosubstitué, présentant le cas échéant au moins un hétéroatome comme élément de cycle, qui peut être condensé avec un système cyclique monocyclique ou polycyclique saturé ou insaturé, non substitué ou au moins monosubstitué et/ou lié via un groupe C₁₋₆-alkylène ou un groupe hétéroalkylène de 2 à 6 chaînons linéaire ou ramifié, non substitué ou au moins monosubstitué ; ou un radical aryle ou hétéroaryle de 5 à 14 chaînons, non substitué ou au moins monosubstitué, qui peut être condensé avec un système cyclique monocyclique ou polycyclique saturé ou insaturé, non substitué ou au moins monosubstitué et/ou lié via un groupe C₁₋₆-alkylène ou hétéroalkylène de 2 à 6 chaînons linéaire ou ramifié, non substitué ou au moins monosubstitué ;
R²⁵, R²⁶ et R²⁷ représentent, indépendamment les uns des autres, à chaque fois un radical aliphatique en C₁₋₁₀, linéaire ou ramifié, saturé ou insaturé, non substitué ou au moins monosubstitué ;
R²⁸ représente un radical en C₁₋₁₀ aliphatique, linéaire ou ramifié, saturé ou insaturé, non substitué ou au moins monosubstitué ;
R²⁹, R³⁰ et R³¹ représentent, indépendamment les uns des autres, à chaque fois H ; F ; Cl ; Br ; I ; -SF₅ ; -NO₂ ; -CF₃ ; -CN ; -NH₂ ; -OH ; -SH ; -C(=O)-NH₂ ; -S(=O)₂-NH₂ ; -C(=O)-NH-OH ; -C(=O)-OH ; -C(=O)-H ; -S(=O)₂-OH ; -NHR¹⁰ ; -NR¹¹-R¹² ; -OR¹³ ; -SR¹⁴ ; -C(=O)-NHR¹⁵ ; -C(=O)-NR¹⁶R¹⁷ ; -S(=O)₂-NHR¹⁸ ; -S(=O)₂-NR¹⁹R²⁰ ; -C(=O)-OR²¹ ; -C(=O)-R²² ; -S(=O)-R²³ ; -S(=O)₂-R²⁴ ;
un radical aliphatique en C₁₋₁₀ linéaire ou ramifié, saturé ou insaturé, non substitué ou au moins monosubstitué ;
ou un radical aryle ou hétéroaryle de 5 à 14 chaînons, non substitué ou au moins monosubstitué, qui peut être condensé avec un système cyclique monocyclique ou polycyclique saturé ou insaturé, non substitué ou au moins monosubstitué et/ou lié via un groupe C₁₋₆-alkylène, un groupe C₂₋₆-alcénylène ou un groupe C₂₋₆-alcynylène linéaire ou ramifié, non substitué ou au moins monosubstitué ;
R³² représente H ; F ; Cl ; Br ; I ; -SF₅ ; -NO₂ ; -CF₃ ; -CF₂Cl ; -CN ; -NH ; -OH ; -SH ; -C(=O)-NH ; -S(=O)₂-NH₂ ; -C(=O)-NH-OH ; -C(=O)-OH ; -C(=O)-H ; -S(=O)₂-OH ; -NHR³³ ; -NR³⁴R³⁵ ; -OR³⁶ ; -SR³⁷ ; -C(=O)-NHR³⁸ -C(=O)-NR³⁹R⁴⁰ ; -S(=O)₂-NHR⁴¹ ; -S(=O)₂-NR⁴²R⁴³ ; -C(=O)-OR⁴⁴ ; -C(=O)-R⁴⁵ ; -S(=O)-R⁴⁶ ; -S(=O)₂-R⁴⁷ ; -C(=NH)-NH₂ ; -C(=NH)-NH-R⁴⁸ ; -N=C(NH₂)₂ ; -N=C(NHR⁴⁹) (NHR⁵⁰) ;
un radical aliphatique en C₁₋₁₀ linéaire ou ramifié, saturé ou insaturé, non substitué ou au moins monosubstitué ;
un radical cycloaliphatique de 3, 4, 5, 6, 7, 8 ou 9 chaînons, insaturé ou saturé, non substitué ou au moins monosubstitué, présentant le cas échéant au moins un hétéroatome comme élément de cycle, qui est à chaque fois lié via un atome de carbone dans le cycle du radical cycloaliphatique à la structure de base et qui peut être condensé avec un système cyclique monocyclique ou polycyclique saturé ou insaturé, non substitué ou au moins monosubstitué et/ou lié via un groupe C₁₋₆-alkylène ou un groupe C₂₋₆-alcénylène ou un groupe C₂₋₆-alcynylène linéaire ou ramifié, non substitué ou au moins monosubstitué ;
ou un radical aryle ou hétéroaryle de 5 à 14 chaînons, non substitué ou au moins monosubstitué, qui peut être condensé avec un système cyclique monocyclique ou polycyclique saturé ou insaturé, non substitué ou au moins monosubstitué et/ou lié via un groupe C₁₋₆-alkylène, un groupe C₂₋₆-alcénylène ou un groupe C₂₋₆-alcynylène linéaire ou ramifié, non substitué ou au moins monosubstitué ;
R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁴⁸, R⁴⁹ et R⁵⁰ représentent, indépendamment les uns des autres, à chaque fois
un radical aliphatique en C₁₋₁₀ linéaire ou ramifié, saturé ou insaturé, non substitué ou au moins monosubstitué ;
un radical cycloaliphatique de 3, 4, 5, 6, 7, 8 ou 9 chaînons, insaturé ou saturé, non substitué ou au moins monosubstitué, présentant le cas échéant au moins un hétéroatome comme élément de cycle, qui peut être condensé avec un système cyclique monocyclique ou polycyclique saturé ou insaturé, non substitué ou au moins monosubstitué et/ou lié via un groupe C₁₋₆-alkylène ou un groupe hétéroalkylène de 2 à 6 chaînons linéaire ou ramifié, non substitué ou au moins monosubstitué ; ou un radical aryle ou hétéroaryle de 5 à 14 chaînons, non substitué ou au moins monosubstitué, qui peut être condensé avec un système cyclique monocyclique ou polycyclique saturé ou insaturé, non substitué ou au moins monosubstitué et/ou lié via un groupe C₁₋₆-alkylène ou hétéroalkylène de 2 à 6 chaînons linéaire ou ramifié, non substitué ou au moins monosubstitué ; ou
R³⁴ et R³⁵ représentent à chaque fois ensemble avec l'atome d'azote qui les relie comme élément de cycle un radical hétérocycloaliphatique de 4, 5, 6, 7, 8 ou 9 chaînons, saturé ou insaturé, non substitué ou substitué par 1, 2, 3, 4 ou 5 radicaux R⁵¹, présentant le cas échéant au moins un autre hétéroatome comme élément de cycle, qui peut être condensé avec un système cyclique monocyclique ou polycyclique saturé ou insaturé, non substitué ou au moins monosubstitué ;
R⁵¹ représente -NHR⁵², -NR⁵³R⁵⁴ ou un radical aliphatique en C₁₋₁₀, linéaire ou ramifié, saturé ou insaturé, non substitué ou au moins monosubstitué ;
R⁵², R⁵³ et R⁵⁴ représentent, indépendamment les uns des autres, à chaque fois C-(=O)-R⁵⁵ ; un radical aliphatique en C₁₋₁₀, linéaire ou ramifié, saturé ou insaturé, non substitué ou au moins monosubstitué ;
ou un radical aryle ou hétéroaryle de 5 à 14 chaînons, non substitué ou au moins monosubstitué, qui peut être condensé avec un système cyclique monocyclique ou polycyclique saturé ou insaturé, non substitué ou au moins monosubstitué et/ou lié via un groupe C₁₋₆-alkylène, un groupe C₂₋₆-alcénylène ou un groupe C₂₋₆-alcynylène linéaire ou ramifié, non substitué ou au moins monosubstitué ; et
R⁵⁵ représente un radical aliphatique en C₁₋₁₀, linéaire ou ramifié, saturé ou insaturé, non substitué ou au moins monosubstitué ;
le cas échéant à chaque fois sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants ;
où
les radicaux aliphatiques en C₁₋₁₀ susmentionnés peuvent le cas échéant à chaque fois être substitués par 1, 2, 3, 4, 5, 6, 7, 8 ou 9 substituants choisis, indépendamment les uns des autres, dans le groupe constitué par F, Cl, Br, I, -CN, -NO₂, -OH, -NH₂, -SH, -O(C₁₋₅-alkyle), -S(C₁₋₅-alkyle), -NH(C₁₋₅-alkyle), -N(C₁₋₅-alkyl) (C₁₋₅-alkyle), -C(=O)-O-C₁₋₅-alkyle, -0-C(=O)-C₁₋₅-alkyle, -0-phényle, phényle, -OCF₃ et -SCF₃ ; les groupes hétéroalkylène de 2 à 6 chaînons, les groupes C₁₋₆-alkylène et les groupes C₂₋₆-alcénylène et les groupes C₂₋₆-alcynylène susmentionnés peuvent le cas échéant à chaque fois être substitués par 1, 2, 3, 4, 5, 6, 7, 8 ou 9 substituants choisis, indépendamment les uns des autres, dans le groupe constitué par F, Cl, Br, I, -CN, -NO₂, -OH, -NH₂, -SH, -O(C₁₋₅-alkyle), -S(C₁₋₅-alkyle), -NH (C₁₋₅-alkyle), -N (C₁₋₅-alkyl) (C₁₋₅-alkyle), -OCF₃ et -SCF₃ ;
les groupes hétéroalkylène susmentionnés présentant le cas échéant à chaque fois 1, 2 ou 3 hétéroatome(s) comme élément(s) de cycle, choisi(s), indépendamment les uns des autres, dans le groupe constitué par oxygène, soufre et azote (NH) ;
les radicaux (hétéro)cycloaliphatiques susmentionnés sont le cas échéant à chaque fois substitués par 1, 2, 3, 4 ou 5 substituants choisis, indépendamment les uns des autres, dans le groupe constitué par -C₁₋₆-alkylène-OH, =CH₂, -O-C₁₋₅-alkylène-oxétanyle, -C₁₋₅-alkylène-OC₁₋₅-alkylène-oxétanyle, -CH₂-NH-C₁₋₅-alkyle, -CH₂-N(C₁₋₅-alkyle)₂, -N[C(=O)-C₁₋₅-alkyl]-phényle, -CH₂-O-C₁₋₅-alkyle, oxo (=O), thioxo (=S), F, Cl, Br, I, -CN, -CF₃, -SF₅, -OH, -O-C₁₋₅-alkyle, -O-C (=O) -C₁₋₅-alkyle, -NH₂, -NO₂, -O-CF₃, -S-CF₃, -SH, -S-C₁₋₅-alkyle, -C₁₋₅-alkyle, -C(=O)-C₁₋₅-alkyle, -C(=O)-OH, -C(=O)-O-C₁₋₅-alkyle, -NH-C₁₋₅-alkyle, -N(C₁₋₅-alkyle)₂, -NH-phényle, -N(C₁₋₅-alkyl)-phényle, cyclohexyle, cyclopentyle, (4,5)-dihydro-isoxazolyle, thiazolyle, (1,2,5)-thiadiazolyle, thiophényle, phénéthyle, pipéridinyle, pyrrolidinyle, -(CH₂)-pyridinyle, pyridinyle, -0-phényle, -0-benzyle, phényle et benzyle, la partie cyclique des radicaux oxétanyle, (4,5)-dihydro-isoxalolyle, thiazolyle, (1,2,5)-thiadiazolyle, thiophényle, phénéthyle, -N[C(=O)-C₁₋₅-alkyl]-phényle, -NH-phényle, -N(C₁₋₅-alkyl)-phényle, -(CH₂)-pyridinyle, pyridinyle, -0-phényle, -O-benzyle, phényle et benzyle pouvant à chaque fois être substituée par 1, 2, 3, 4 ou 5 substituants choisis, indépendamment les uns des autres, dans le groupe constitué par F, Cl, Br, -OH, -CF₃, -SF₅, -CN, -NO₂, -C₁₋₅-alkyle, -O-C₁₋₅-alkyle, -0-CF₃, -S-CF₃, phényle et -0-benzyle,
et, sauf indication contraire, les radicaux (hétéro)cycloaliphatiques susmentionnés pouvant présenter le cas échéant à chaque fois 1, 2 ou 3 (autre(s)) hétéroatome(s) choisi(s), indépendamment les uns des autres, dans le groupe constitué par oxygène, azote et soufre ;
les cycles des systèmes monocycliques ou polycycliques susmentionnés peuvent le cas échéant à chaque fois être substitués par 1, 2, 3, 4 ou 5 substituants choisis, indépendamment les uns des autres, dans le groupe constitué par oxo (=O), thioxo (=S), F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₅-alkyle, -C(=O)-C₁₋₅-alkyle, -C(=O)-OH, -C(=O)-O-C₁₋₅-alkyle, -NH-C₁₋₅-alkyle, -N(C₁₋₅-alkyle)₂, -O-phényle, -0-benzyle, phényle et benzyle, la partie cyclique des radicaux -0-phényle, -0-benzyle, phényle et benzyle pouvant à chaque fois être substituée par 1, 2, 3, 4 ou 5 substituants choisis, indépendamment les uns des autres, dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -O-benzyle,
et les cycles des systèmes cycliques monocycliques ou polycycliques susmentionnés présentant à chaque fois 5, 6 ou 7 chaînons et pouvant présenter le cas échant à chaque fois 1, 2, 3, 4 ou 5 hétéroatome(s) comme élément (s) de cycle, choisi(s), indépendamment les uns des autres, dans le groupe constitué par oxygène, azote et soufre ;
et les radicaux aryle ou hétéroaryle susmentionnés pouvant le cas échéant à chaque fois être substitués par 1, 2, 3, 4 ou 5 substituants choisis, indépendamment les uns des autres, dans le groupe constitué par F, Cl, Br, I, -CN, -CF₃, -SF₅, -OH, -0-C₁₋₅-alkyle, -NH₂, -NO₂, -O-CF₃, -S-CF₃, -SH, -S-C₁₋₅-alkyle, -C₁₋₅-alkyle, -C(=O)-OH, -C (=O) -O-C₁₋₅-alkyle, -NH-C₁₋₅-alkyle, -N(C₁₋₅-alkyle)₂, -NH-S((=O)₂-C₁₋₅-alkyle, -NH-C((=O)-O-C₁₋₅-alkyle, -C(=O)-H, -C(=O)-C₁₋₅-alkyle, -C(=O)-NH₂, -C(=O)-NH-C₁₋₅-alkyle, -C(=O)-N-(C₁₋₅-alkyle)₂, -0-phényle, -0-benzyle, phényle et benzyle, la partie cyclique des radicaux -0-phényle, -0-benzyle, phényle et benzyle pouvant être substituée par 1, 2, 3, 4 ou 5 substituants choisis, indépendamment les uns des autres, dans le groupe constitué par F, Cl, Br, -OH, -CF₃, -SF₅, -CN, -NO₂, -C₁₋₅-alkyle, -O-C₁₋₅-alkyle, -0-CF₃, -S-CF₃, phényle et -0-benzyle, et
et les radicaux hétéroaryle susmentionnés présentant le cas échéant à chaque fois 1, 2, 3, 4 ou 5 hétéroatome(s) comme élément(s) de cycle, choisi(s), indépendamment les uns des autres, dans le groupe constitué par oxygène, azote et soufre.

2. Composés selon la revendication 1, **caractérisés en ce que**
X représente O ;
n vaut 0, 1 ou 2 ;
R¹, R², R³ et R⁴ représentent, indépendamment les uns des autres, à chaque fois H ; F ; Cl ; Br ; I ; -SF₅ ; -NO₂ ; -CN ; -NH ; -OH ; -SH ; -C(=O)-NH₂ ; -S(=O)₂-NH₂ ; -C(=O)-NH-OH ; -C(=O)-OH ; -C(=O)-H ; -S(=O)₂-OH ; -NHR¹⁰ ; -NR¹¹R¹² ; -OR¹³ ; -SR¹⁴ ; -S(=O)-R²³ ; -S(=O)₂-R²⁴ ou un radical choisi dans le groupe constitué par méthyle, -CF₃, -CCl₃, -CBr₃, -CHF₂, -CH₂F, -CF₂Cl, -CCl₂F, éthyle, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-propyle, -CF₂-CF₂-CF₃, -CF(CF₃)₂, isopropyle, n-butyle, sec-butyle, isobutyle et tert-butyle ;
R⁵ représente -NH₂ ; -NHR²⁵ ; -NR²⁶R²⁷ ; un radical alkyle choisi dans le groupe constitué par -CF₃, -CH₂-CF₃, méthyle, éthyle, n-propyle, isopropyle, tert-butyle, n-butyle, sec-butyle et isobutyle ;
R⁶ représente -C(=O)-R²⁸ ou un radical choisi dans le groupe constitué par méthyle, -CF₃, -CCl₃ -CBr₃, -CHF₂, -CH₂F, -CF₂Cl, -CCl₂F -CH₂-CN, -CH₂-O-CH₃, -CH₂-O-CF₃, -CH₂-SF₃, éthyle, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-propyle, -CF₂-CF₂-CF₃, -CF(CF₃)₂, isopropyle, -CH₂-CH₂-CH₂-CN, -CH₂-O-CH₂-CH₃, -CH₂-CH₂-SF₃, -CH₂-CH₂-OCF₃, -CH(CH₃) (O-CH₃), -CH (CH₃) (S-CH₃), n-butyle, -CF₂-CF₂-CF₂-CF₃, -CH₂-CH₂-CH₂-CH₂-CN, sec-butyle, isobutyle, tert-butyle, n-pentyle, 3-pentyle, n-heptyle, 4-heptyle, n-octyle, n-nonyle, 5-nonyle, (2,6)-diméthylhept-4-yle, 3-méthylbutyle, n-hexyle, (3,3)-diméthylbutyle, -CH₂ -CH₂-O-CH₃, -CH₂-CH₂-O-C₂H₅, -CH₂-CH₂-CH₂-O-CH₃, éthényle, propényle, 2-butényle, 3-butényle, 2-pentényle et 3-pentényle ;
R⁷ et R⁸ représentent, indépendamment l'un de l'autre, à chaque fois un radical hydrogène ;
un radical alkyle choisi dans le groupe constitué par -CH₂-OH, -CH₂-CH₂-OH, -CH₂-CH₂-CH₂-OH, -CH₂-CH₂-CH₂-CH₂-OH, isopropyle, n-butyle, sec-butyle, isobutyle, méthyle, éthyle et n-propyle ;
un radical choisi dans le groupe constitué par phényle, naphtyle, thiophényle, furannyle, pyrrolyle, pyrazolyle, pyrazinyle, pyrannyle, triazolyle, pyridinyle, imidazolyle, indolyle, iso-indolyle, thiazolyle, oxazolyle, isoxazolyle, pyridazinyle, pyrazinyle, pyrimidinyle, indazolyle, quinoxalinyle, quinoléinyle et isoquinoléinyle, qui peut à chaque fois être lié via un groupe -(CH₂)-, -(CH₂)₂- ou -(CH₂)₃- et/ou à chaque fois être non substitué ou le cas échéant substitué par 1, 2, 3, 4 ou 5 substituants choisis, indépendamment les uns des autres, dans le groupe constitué par F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, méthyle, éthyle, n-propyle, isopropyle, n-butyle, sec-butyle, isobutyle, tert-butyle et n-pentyle ;
ou un radical choisi dans le groupe constitué par cyclopropyle, cyclobutyle, cyclopentyle, cyclohexyle et cyclopentyle ;
à condition que R⁷ et R⁸ ne représentent pas à chaque fois un radical hydrogène ; ou
R⁷ et R⁸ forment à chaque fois ensemble avec l'atome de carbone qui les relie comme élément de cycle un radical choisi dans le groupe constitué par cyclopropyle, cyclobutyle, cyclopentyle, cyclohexyle et cycloheptyle ;
T représente C-R²⁹ et U représente C-R³⁰ et V représente N et W représente C-R³² ou
T représente C-R²⁹ et U représente N et V représente C-R³¹ et W représente C-R³² ou
T représente N et U représente C-R³⁰ et V représente C-R³¹ et W représente C-R³² ou
T représente N et U représente N et V représente C-R³¹ et W représente C-R³² ou
T représente N et U représente C-R³⁰ et V représente N et W représente C-R³² ou
T représente C-R²⁹ et U représente N et V représente N et W représente C-R³²
R⁹ représente F ; Cl ; Br ; I ; -SF₅ ; -OR¹³ ; -SR¹⁴ ; -S(=O)-R²³ ; -S(=O)₂-R²⁴ ;
un radical choisi dans le groupe constitué par méthyle, -CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, éthyle, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-propyle, -CF₂-CF₂-CF₃, -CF(CF₃)₂, isopropyle, -CH₂-CH₂-CH₂-CN, -CH₂-O-CH₂-CH₃, -CH₂-CH₂-SF₃, -CH₂-CH₂-OCF₃ , -CH (CH₃) (O-CH₃) -CH (CH₃) (S-CH₃), n-butyle, -CF₂-CF₂-CF₂-CF₃, -CH₂-CH₂-CH₂-CH₂-CN, n-butyle, sec-butyle, isobutyle, -C(CH₃)₂(CH₂OH) et tert-butyle ;
ou un radical choisi dans le groupe constitué par cyclopropyle, cyclobutyle, cyclopentyle, cyclohexyle, cycloheptyle, cyclopentényle et cyclohexényle, qui peut le cas échéant à chaque fois être substitué par 1, 2, 3, 4 ou 5 substituants choisis, indépendamment les uns des autres, dans le groupe constitué par oxo (=O), thioxo (=S), -OH, -O-CH₃, -O-C₂H₅, -O-CH(CH₃)₂, -OC(CH₃)₃, -O-CF₃, -S-CF₃, -SH, -S-CH₃, -S-C₂H₅, -S-CH(CH₃)₂, -S-C(CH₃)₃, méthyle, éthyle, n-propyle, isopropyle, n-butyle, sec-butyle, isobutyle, tert-butyle et n-pentyle ;
R¹⁰, R¹¹, R¹², R¹³, R¹⁴, R²¹, R²³ et R²⁴ représentent,
indépendamment les uns des autres, à chaque fois un radical choisi dans le groupe constitué par méthyle, -CF₃, -CCl₃, -CBr₃, -CHF₂, -CH₂F, -CF₂Cl, -CCl₂F -CH₂-CN, -CH₂-O-CH₃, -CH₂-O-CF₃, -CH₂-SF₃, éthyle, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-propyle, -CF₂-CF₂-CF₃, -CF(CF₃)₂, isopropyle, -CH₂-CH₂-CH₂-CN, -CH₂-O-CH₂-CH₃, -CH₂-CH₂-SF₃, -CH₂-CH₂-OCF₃, -CH (CH₃) (O-CH₃), -CH (CH₃) (S-CH₃), n-butyle, -CF₂-CF₂-CF₂-CF₃, -CH₂-CH₂-CH₂-CH₂-CN, sec-butyle, isobutyle, tert-butyle, n-pentyle, 3-pentyle, n-heptyle, 4-heptyle, n-octyle, n-nonyle, 5-nonyle, (2,6)-diméthylhept-4-yle, 3-méthylbutyle, n-hexyle, (3,3)-diméthylbutyle, -CH₂-CH₂-O-CH₃, -CH₂-CH₂-O-C₂H₅, -CH₂-CH₂-CH₂-O-CH₃, éthényle, propényle, 2-butényle, 3-butényle, 2-pentényle et 3-pentényle ;
un radical choisi dans le groupe constitué par 2,3-dihydro-1H-indényle, cyclopropyle, oxétanyle, cyclobutyle, cyclopentyle, cyclohexyle, imidazolidinyle, tétrahydrofurannyle, tétrahydrothiophényle, pyrrolidinyle, pipéridinyle, morpholinyle, pipérazinyle, azépanyle, diazépanyle, azocanyle et thiomorpholinyle, qui peut à chaque fois être lié via un groupe -CH₂-O-, -CH₂-CH₂-O-, -CH₂-CH₂-O-CH₂-, -CH₂-CH(CH₃)-O-CH₂-, -(CH₂)-, -(CH₂)₂- ou -(CH₂)₃-et/ou à chaque fois être non substitué ou le cas échéant substitué par 1, 2, 3, 4 ou 5 substituants choisis, indépendamment les uns des autres, dans le groupe constitué par oxo (=O), thioxo (=S), -OH, -O-CH₃, -O-C₂H₅, -O-CH(CH₃)₂, -O-C(CH₃)₃, -0-CF₃, -S-CF₃, -SH, -S-CH₃, -S-C₂H₅, -S-CH(CH₃)₂, -S-C(CH₃)₃, méthyle, éthyle, n-propyle, isopropyle, n-butyle, sec-butyle, isobutyle, tert-butyle, n-pentyle, -C(=O)-CH₃, -C(=O)-C₂H₅, -C(=O)-CH(CH₃)₂, -C (=O) -C (CH₃)₃, -C (=O) -OH, -C (=O) -O-CH₃, -C (=O) -0-C₂H₅, -C(=O)-O-CH(CH₃) et -C(=O)-O-C(CH₃)₃ ;
ou un radical choisi dans le groupe constitué par phényle, naphtyle, thiophényle, furannyle, pyrrolyle, pyrazolyle, pyrazinyle, pyrannyle, triazolyle, pyridinyle, imidazolyle, thiazolyle, oxazolyle et isoxazolyle, le radical pouvant à chaque fois être lié via un groupe -(CH₂)-, -(CH₂)₂- ou -(CH₂)₃- et/ou à chaque fois être non substitué ou le cas échéant substitué par 1, 2, 3, 4 ou 5 substituants choisis, indépendamment les uns des autres, dans le groupe constitué par F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, méthyle, éthyle, n-propyle, isopropyle, n-butyle, sec-butyle, isobutyle, tert-butyle, n-pentyle, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃) (C₂H₅), -NH-C(=O)-O-CH₃, -NH-C(=O)-O-C₂H₅, -NH-C(=O)-O-C(CH₃)₃, -C (=O) -H, -C (=O) -CH₃, -C (=O) -C₂H₅, -C (=O) -CH (CH₃)₂, -C (=O) -C (CH₃)₃, -C (=O) -NH₂, -C (=O) -NH-CH₃, -C (=O)-NH-C₂H₅, -C(=O)-N(CH₃)₂, -C(=O)-N(C₂H₅)₂, -0-phényle, -O-benzyle, phényle et benzyle ;
R²⁵, R²⁶ et R²⁷ représentent, indépendamment les uns des autres, à chaque fois un radical alkyle choisi dans le groupe constitué par -CF₃, -CH₂-CF₃, méthyle, éthyle, n-propyle, isopropyle, tert-butyle, n-butyle, sec-butyle et isobutyle ;
R²⁸ représente un radical choisi dans le groupe constitué par méthyle, -CF₃, -CCl₃, -CBr₃, -CHF₂, -CH₂F, -CF₂Cl, -CH₂-CN, -CH₂-O-CH₃, -CH₂-O-CF₃, -CH₂-SF₃, éthyle, -CF₂-CH₃, -CH₂-CF₃, -C₂F₅, -CH₂-CCl_{3,} -CH₂-CBr₃, -CHF-CF₂Cl, -CF₂-CF₂Cl, -CFCl-CF₂Cl, -CH₂-CH₂-CN, n-propyle, isopropyle, sec-butyle, isobutyle, tert-butyle, n-pentyle, 3-pentyle, n-heptyle, 4-heptyle, n-octyle, n-nonyle, 5-nonyle, (2,6)-diméthylhept-4-yle, 3-méthylbutyle, n-hexyle, (3,3)-diméthylbutyle, éthényle, propényle, 2-butényle, 3-butényle, 2-pentényle et 3-pentényle ;
R²⁹, R³⁰ et R³¹ représentent, indépendamment les uns des autres, à chaque fois H ; F ; Cl ; Br ; I ; -SF₅ ; -NO₂ ; -CN ; -NH ; -OH ; -SH ; -C(=O)-NH₂ ; -S(=O)₂-NH₂ ; -C(=O)-NH-OH ; -C(=O)-OH ; -C(=O)-H ; -S(=O)₂-OH ; -NHR¹⁰; -NR¹¹R¹² ; -OR¹³ ; -SR¹⁴ ; -C(=O)-OR^{21 ;} -S(=O)-R²³ ; ; S(=O)₂-R²⁴ ; un radical choisi dans le groupe constitué par -CH₂-OH, méthyle, -CF₃, -CCl₃ -CBr₃, -CHF₂, -CH₂F, -CF₂Cl, -CCl₂F éthyle, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, n-propyle, -CF₂-CF₂-CF₃, -CF(CF₃)₂, isopropyle, n-butyle, sec-butyle, isobutyle et tert-butyle ou un radical phényle, qui peut être non substitué ou le cas échéant substitué par 1, 2, 3, 4 ou 5 substituants choisis, indépendamment les uns des autres, dans le groupe constitué par F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, méthyle, éthyle, n-propyle, isopropyle, n-butyle, sec-butyle, isobutyle, tert-butyle et n-pentyle ;
R³² représente H ; -SF₅ ; -NO₂ ; -CN ; -NH₂ ; -OH ; -SH ; -C(=O)-NH₂ -S(=O)₂-NH₂ ; -C(=O)-NH-OH ; -C(=O)-OH ; -C(=O)-H ; -S(=O)₂-OH ; -NHR³³ ; -NR³⁴R³⁵ ; -OR³⁶ ; -SR³⁷ ; -C(=O)-OR⁴⁴ ; -S(=O)-R⁴⁶; -S(=O)₂-R⁴⁷ ; -C(=NH)-NH₂ ; -C(=NH)-NH-R⁴⁸ ; -N=C(NH₂)₂ ; -N=C(NHR⁴⁹)(NHR⁵⁰) ;
un radical choisi dans le groupe constitué par cyclopropyle, cyclobutyle, cyclopentyle, cyclohexyle, cyclohexényle, imidazolidinyle, tétrahydrofurannyle, tétrahydrothiophényle, pyrrolidinyle, pipéridinyle, morpholinyle, pipérazinyle, azépanyle, diazépanyle, azocanyle et thiomorpholinyle, qui est à chaque fois lié à la structure de base via un atome de carbone des cycles des radicaux susmentionnés ou via un groupe -(CH=CH)-, -C≡C- ou -C≡C-CH₂- et qui peut être non substitué ou le cas échéant à chaque fois substitué par 1, 2, 3, 4 ou 5 substituants choisis, indépendamment les uns des autres, dans le groupe constitué par -CN, -CH₂-N(CH₃)₂, -CH₂-N(C₂H₅)₂, -CH₂-NH-CH₃, -CH₂-NH-C₂H₅, -N-[C(=O)-C₂H₅]-phényle, -N[C(=O)-CH₃]-phényle, oxo (=O), thioxo (=S), -OH, -O-CH₃, -O-C₂H₅, -O-CH(CH₃)₂, -O-C(CH₃)₃, -O-CF₃, -S-CF₃, -SH, -S-CH₃, -S-C₂H₅, -S-CH(CH₃)₂, -S-C(CH₃)₃, méthyle, éthyle, n-propyle, isopropyle, n-butyle, sec-butyle, isobutyle, tert-butyle, n-pentyle, -C(=O)-CH₃, -C(=O)-C₂H₅, -C(=O)-CH(CH₃)₂, -C (=O) -C (CH₃)₃, -C (=O) -OH, -C (=O) -O-CH₃, -C (=O) -0-C₂H₅, -C(=O)-O-CH(CH₃)₂ et -C(=O)-O-C(CH₃)₃ ;
ou un radical choisi dans le groupe constitué par (1,3)-benzodioxolyle, (1,4)-benzodioxanyle, tétrazolyle, (2,3)-dihydrothiéno[3,4-b][1,4]dioxinyle, benzo[b]furannyle, phényle, naphtyle, thiophényle, furannyle, pyrrolyle, pyrazolyle, pyrazinyle, pyrannyle, triazolyle, pyridinyle, imidazolyle, indolyle, iso-indolyle, thiazolyle, oxazolyle, isoxazolyle, pyridazinyle, pyrazinyle, pyrimidinyle, indazolyle, quinoxalinyle, quinoléinyle et isoquinoléinyle, qui peut à chaque fois être lié via un groupe -(CH=CH)-, -C≡C-, -(CH₂)-, -(CH₂)₂- ou -(CH₂)₃-et/ou à chaque fois être non substitué ou le cas échéant substitué par 1, 2, 3, 4 ou 5 substituants choisis, indépendamment les uns des autres, dans le groupe constitué par F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, méthyle, éthyle, n-propyle, isopropyle, n-butyle, sec-butyle, isobutyle, tert-butyle, n-pentyle, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C (=O) -O-CH₃, -NH-S (=O)₂-CH₃, -NH-S (=O)₂-C₂H₅, -NH-S(=O)₂-CH (CH₃)₂, -NH-C (=O) -O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C (=O) -NH-C₂H₅, -C (=O) -N (CH₃)₂, -C(=O)-N(C₂H₅)₂, -O-phényle, -0-benzyle, phényle et benzyle ;
R³³, R³⁴, R³⁵, R³⁶, R³⁷, R⁴⁴, R⁴⁶, R⁴⁷, R⁴⁸, R⁴⁹ et R⁵⁰ représentent, indépendamment les uns des autres, à chaque fois
un radical choisi dans le groupe constitué par méthyle, -CF₃, -CCl₃, -CBr₃, -CHF₂, -CH₂F, -CF₂Cl, -CCl₂F, -CH₂-CN, -CH₂-O-CH₃, -CH₂-O-CF₃, -CH₂-SF₃, éthyle, -CF₂-CH₃, -CH₂-CF₃, -C₂F₅, -CH₂-CCl₃, -CH₂-CBr₃, -CHF-CF₂Cl, -CF₂-CF₂Cl, -CFCl-CF₂Cl, -CH₂-CH₂-CN, n-propyle, -CF₂-CF₂-CF₃, -CF(CF₃)₂, isopropyle, -CH₂-CH₂-CH₂-CN, -CH₂-O-CH₂-CH₃, -CH₂-CH₂-SF₃, -CH₂-CH₂-OCF₃, -CH (CH₃) (O-CH₃), -CH (CH₃) (S-CH₃), n-butyle, -CF₂-CF₂-CF₂-CF₃, -CH₂-CH₂-CH₂-CH₂-CN, sec-butyle, isobutyle, tert-butyle, n-pentyle, 3-pentyle, n-heptyle, 4-heptyle, n-octyle, n-nonyle, 5-nonyle, (2,6)-diméthylhept-4-yle, 3-méthylbutyle, n-hexyle, (3,3)-diméthylbutyle, -CH₂-CH₂-O-CH₃, -CH₂-CH₂-O-C₂H₅, -CH₂-CH₂-CH₂-O-CH₃, éthényle, propényle, 2-butényle, 3-butényle, 2-pentényle et 3-pentényle ;
un radical choisi dans le groupe constitué par 2,3-dihydro-1H-indényle, cyclopropyle, oxétanyle, cyclobutyle, cyclopentyle, cyclohexyle, imidazolidinyle, tétrahydrofurannyle, tétrahydrothiophényle, pyrrolidinyle, pipéridinyle, morpholinyle, pipérazinyle, azépanyle, diazépanyle, azocanyle et thiomorpholinyle, qui peut à chaque fois être lié via un groupe -CH₂-O-, -CH₂-CH₂-O-, -CH₂-CH₂-O-CH₂-, -CH₂-CH(CH₃)-O-CH₂-, -(CH₂)-, -(CH₂)₂- ou -(CH₂)₃-et/ou à chaque fois être non substitué ou le cas échéant substitué par 1, 2, 3, 4 ou 5 substituants choisis, indépendamment les uns des autres, dans le groupe constitué par oxo (=O), thioxo (=S), -OH, -O-CH₃, -O-C₂H₅, -O-CH(CH₃)₂, -O-C(CH₃)₃, -0-CF₃, -S-CF₃, -SH, -S-CH₃, -S-C₂H₅, -S-CH(CH₃)₂, -S-C(CH₃)₃, méthyle, éthyle, n-propyle, isopropyle, n-butyle, sec-butyle, isobutyle, tert-butyle, n-pentyle, -C(=O)-CH₃, -C(=O)-C₂H₅, -C(=O)-CH(CH₃)₂, -C (=O) -C (CH₃)₃, -C (=O) -OH, -C (=O) -O-CH₃, -C (=O) -0-C₂H₅, -C (=O)-O-CH (CH₃)₂ et -C (=O) -O-C (CH₃)₃ ;
ou un radical choisi dans le groupe constitué par phényle, naphtyle, thiophényle, furannyle, pyrrolyle, pyrazolyle, pyrazinyle, pyrannyle, triazolyle, pyridinyle, imidazolyle, thiazolyle, oxazolyle et isoxazolyle, le radical pouvant à chaque fois être lié via un groupe -(CH₂)-, -(CH₂)₂- ou -(CH₂)₃- et/ou à chaque fois être non substitué ou le cas échéant substitué par 1, 2, 3, 4 ou 5 substituants choisis, indépendamment les uns des autres, dans le groupe constitué par F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, méthyle, éthyle, n-propyle, isopropyle, n-butyle, sec-butyle, isobutyle, tert-butyle, n-pentyle, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃) (C₂H₅), -NH-C(=O)-O-CH₃, -NH-C (=O)-O-C₂H₅, -NH-C(=O) -O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -0-phényle, -O-benzyle, phényle et benzyle ; ou
R³⁴ et R³⁵ forment, à chaque fois ensemble avec l'atome d'azote qui les relie comme élément de cycle, un radical choisi dans le groupe constitué par 3-azabicyclo[3,1,1]heptyle, 6-azaspiro[2,5]octyle, 3-azabicyclo[3,2,1]octyle, 6-azabicyclo[3,3,1]heptyle, 8-azabicyclo[3,2,1]octyle, 1-oxa-2,8-diazaspiro[4,5]déc-2-ényle, azocanyle, iso-indolyle, indolyle, (1,2,3,6)-tétrahydropyridinyle, (4,5,6,7)-tétrahydro-isoxazolo[5,4-c]pyridinyle, pyrrolidinyle, pipéridinyle, pipérazinyle, morpholinyle, azépanyle, diazépanyle et thiomorpholinyle, dont la partie hétérocycloaliphatique peut à chaque fois être non substituée ou substituée par 1, 2, 3, 4 ou 5 radicaux R⁵¹
R⁵¹ représente -NHR⁵², -NR⁵³R⁵⁴ ou un radical alkyle choisi dans le groupe constitué par -CF₃, -CH₂-CF₃, méthyle, éthyle, n-propyle, isopropyle, tert-butyle, n-butyle, sec-butyle et isobutyle ;
R⁵², R⁵³ et R⁵⁴ représentent, indépendamment les uns des autres, à chaque fois -C(=O)-R⁵⁵;
un radical alkyle choisi dans le groupe constitué par -CF₃, -CH₂-CF₃, méthyle, éthyle, n-propyle, isopropyle, tert-butyle, n-butyle, sec-butyle et isobutyle ;
ou un radical choisi dans le groupe constitué par phényle, naphtyle, thiophényle, furannyle, pyrrolyle, pyrazolyle, pyrazinyle, pyrannyle, triazolyle, pyridinyle, imidazolyle, thiazolyle, oxazolyle et isoxazolyle, le radical pouvant à chaque fois être lié via un groupe -(CH₂)-, -(CH₂)₂- ou -(CH₂)₃- et/ou à chaque fois être non substitué ou le cas échéant substitué par 1, 2, 3, 4 ou 5 substituants choisis, indépendamment les uns des autres, dans le groupe constitué par F, Cl, Br, I, -CN, -CF₃, -SF₅, -OH, -O-CH₃, -O-C₂H₅, -O-CH(CH₃)₂, -O-C(CH₃)₃, -NH₂, -NO₂, -O-CF₃, -S-CF₃, -SH, -S-CH₃, -S-C₂H₅, -S-CH(CH₃)₂, -S-C(CH₃)₃, méthyle, éthyle, n-propyle, isopropyle, n-butyle, sec-butyle, isobutyle, tert-butyle, n-pentyle, -C(=O)-OH, -C(=O)-O-CH₃, -C(=O)-O-C₂H₅, -C(=O)-O-CH(CH₃)₂, -C(=O)-O-C(CH₃)₃, -NH-CH₃, -NH-C₂H₅, -NH-C(CH₃)₃, -N(CH₃)₂, -N(C₂H₅)₂, -N(CH₃)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂, -0-phényle, -O-benzyle, phényle et benzyle ; et
R⁵⁵ représente un radical alkyle choisi dans le groupe constitué par -CF₃, -CH₂-CF₃, méthyle, éthyle, n-propyle, isopropyle, tert-butyle, n-butyle, sec-butyle et isobutyle ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

3. Composés selon la revendication 1 ou 2, **caractérisés en ce que**
X représente O ;
n vaut 1 ;
R¹, R³ et R⁴ représentent, indépendamment les uns des autres, à chaque fois H ; F ; Cl ; Br ; I ; -NHR¹⁰ ; -NR¹¹R¹² ; -OR¹³ ; -SR¹⁴ ou un radical choisi dans le groupe constitué par méthyle, -CF₃, -CHF₂, -CH₂F, éthyle, n-propyle, -CF₂-CF₂-CF₃, -CF(CF₃)₂, isopropyle, n-butyle, sec-butyle, isobutyle et tert-butyle ;
R² représente H ; F ; Cl ; Br ; I ou méthyle ;
R⁵ représente un radical alkyle choisi dans le groupe constitué par -CF₃, -CH₂-CF₃, méthyle, éthyle, n-propyle, isopropyle, tert-butyle, n-butyle, sec-butyle et isobutyle ;
R⁶ représente -C(=O)-R²⁸ ou un radical choisi dans le groupe constitué par méthyle, -CH₂-CN, éthyle, -CH₂-CH₂-CN, n-propyle, -CH₂-CH₂-CH₂-CN, n-butyle, -CH₂-CH₂-CH₂-CH₂-CN, sec-butyle, isobutyle, tert-butyle, n-pentyle, 3-pentyle, n-heptyle, éthényle, propényle, 2-butényle, 3-butényle, 2-pentényle et 3-pentényle ;
R⁷ un radical alkyle choisi dans le groupe constitué par -CH₂-OH, -CH₂-CH₂-OH, -CH₂-CH₂-CH₂-OH, -CH₂-CH₂-CH₂-CH₂-OH, isopropyle, n-butyle, sec-butyle, isobutyle, méthyle, éthyle et n-propyle ;
ou un radical choisi dans le groupe constitué par cyclopropyle, cyclobutyle, cyclopentyle, cyclohexyle et cyclopentyle ;
R⁸ représente un radical hydrogène ; ou
R⁷ et R⁸ forment à chaque fois ensemble avec l'atome de carbone qui les relie comme élément de cycle un radical choisi dans le groupe constitué par cyclopropyle, cyclobutyle, cyclopentyle, cyclohexyle et cycloheptyle ;
T représente C-R²⁹ et U représente C-R³⁰ et V représente N et W représente C-R³² ;
R⁹ représente F ; Cl ; Br ; I ; -SF₅ ; -O-CF₃ ; -0-CCl₃ ; -O-CBr₃ ; -O-CHF₂ ; -O-CH₂F ; -O-CF₂Cl ; -0-CCl₂F ; -O-CF₂-CH₃ ; -S-CF₃ ; -S-CCl₃ ; -S-CBr₃ ; -S-CHF₂ ; -S-CH₂F ; -S-CF₂Cl ; -S-CCl₂F ; -S-CF₂-CH₃ ; ou un radical choisi dans le groupe constitué par méthyle, -CF₃, -CHF₂, -CH₂F, éthyle, n-propyle, isopropyle, n-butyle, sec-butyle, isobutyle, -C(CH₃)₂ (CH₂OH) et tert-butyle ;
R¹⁰, R¹¹, R¹², R¹³ et R¹⁴ représentent, indépendamment les uns des autres, à chaque fois
un radical choisi dans le groupe constitué par méthyle, -CF₃, -CHF₂, -CH₂F, éthyle, n-propyle, isopropyle, n-butyle, sec-butyle, isobutyle, tert-butyle et n-pentyle ;
R²⁸ représente un radical choisi dans le groupe constitué par méthyle, éthyle, n-propyle, isopropyle, sec-butyle, isobutyle, tert-butyle, n-pentyle, 3-pentyle, n-heptyle, 4-heptyle, n-octyle, n-nonyle, 5-nonyle, (2,6)-diméthylhept-4-yle, 3-méthylbutyle, n-hexyle, (3,3)-diméthylbutyle, éthényle, propényle, 2-butényle, 3-butényle, 2-pentényle et 3-pentényle ;
R²⁹ et R³⁰ représentent, indépendamment l'un de l'autre, à chaque fois H ; F ; Cl ; Br ; I ; -SF₅ ; -NO₂ ; -CN ; -NH ; -OH ; -SH ; -NHR¹⁰ ; -NR¹¹R¹² ; -OR¹³ ; -SR¹⁴ ; ou un radical choisi dans le groupe constitué par -CH₂-OH, méthyle, -CF₃, éthyle, n-propyle, isopropyle, n-butyle, sec-butyle, isobutyle et tert-butyle ;
R³² représente H ; -SF₅ ; -NO₂ ; -CN ; -NH ; -OH ; -NHR³³ ; -NR³⁴R³⁵ ; -OR³⁶ ; -SR³⁷ ;
un radical choisi dans le groupe constitué par cyclopropyle, cyclobutyle, cyclopentyle, cyclohexyle, cyclohexényle, imidazolidinyle, tétrahydrofurannyle, tétrahydrothiophényle, pyrrolidinyle, pipéridinyle, morpholinyle, pipérazinyle, azépanyle, diazépanyle, azocanyle et thiomorpholinyle, qui est à chaque fois lié via un atome de carbone des cycles des radicaux susmentionnés à la structure de base et qui peut être non substitué ou le cas échéant à chaque fois substitué par 1, 2, 3, 4 ou 5 substituants choisis, indépendamment les uns des autres, dans le groupe constitué par -CH₂-N(CH₃)₂, -CH₂-N(C₂H₅)₂, -CH₂-NH-CH₃, -CH₂-NH-C₂H₅, -N-[C(=O)-C₂H₅]-phényle, -N-[C(=O)-CH₃]-phényle, méthyle, éthyle, n-propyle, isopropyle, n-butyle, sec-butyle, isobutyle et tert-butyle ;
ou un radical choisi dans le groupe constitué par phényle, thiophényle, furannyle, pyrrolyle, pyrazolyle, pyrazinyle, pyrannyle, triazolyle, pyridinyle, imidazolyle, thiazolyle, oxazolyle, isoxazolyle, pyridazinyle, pyrazinyle et pyrimidinyle, qui peut à chaque fois le cas échéant être substitué par 1, 2, 3, 4 ou 5 substituants choisis, indépendamment les uns des autres, dans le groupe constitué par F, Cl, Br, I, -CN, -CF₃, -SF₅, -OH, -O-CH₃, -O-C₂H₅, -O-CH(CH₃)₂, -O-C(CH₃)₃, -O-CF₃, méthyle, éthyle, n-propyle, isopropyle, n-butyle, sec-butyle, isobutyle, tert-butyle et n-pentyle ;
R³³, R³⁴, R³⁵, R³⁶ et R³⁷ représentent, indépendamment les uns des autres, à chaque fois
un radical choisi dans le groupe constitué par méthyle, -CH₂-O-CH₃, éthyle, n-propyle, n-butyle, sec-butyle, isobutyle, tert-butyle, n-pentyle, 3-pentyle, n-hexyle, (3,3)-diméthylbutyle, -CH₂-CH₂-O-CH₃, -CH₂-CH₂-O-C₂H₅ et -CH₂-CH₂-CH₂-O-CH₃ ;
un radical choisi dans le groupe constitué par 2,3-dihydro-1H-indényle, cyclopropyle, oxétanyle, cyclobutyle, cyclopentyle, cyclohexyle, imidazolidinyle, tétrahydrofurannyle, tétrahydrothiophényle, pyrrolidinyle, pipéridinyle, morpholinyle, pipérazinyle, azépanyle, diazépanyle, azocanyle et thiomorpholinyle, qui peut à chaque fois le cas échéant être substitué par 1, 2, 3, 4 ou 5 substituants choisis, indépendamment les uns des autres, dans le groupe constitué par méthyle, éthyle, n-propyle, isopropyle, n-butyle, sec-butyle, isobutyle, tert-butyle, n-pentyle ; ou
R³⁴ et R³⁵ forment, à chaque fois ensemble avec l'atome d'azote qui les relie comme élément de cycle, un radical choisi dans le groupe constitué par 3-azabicyclo[3,1,1]heptyle, 6-azaspiro[2,5]octyle, 3-azabicyclo[3,2,1]octyle, 6-azabicyclo[3,3,1]heptyle, 8-azabicyclo[3,2,1]octyle, 1-oxa-2,8-diazaspiro[4,5]déc-2-ényle, azocanyle, iso-indolyle, indolyle, (1,2,3,6)-tétrahydropyridinyle, (4,5,6,7)-tétrahydro-isoxazolo[5,4-c]pyridinyle, pyrrolidinyle, pipéridinyle, pipérazinyle, morpholinyle, azépanyle, diazépanyle et thiomorpholinyle, dont la partie hétérocycloaliphatique peut à chaque fois être non substituée ou substituée par 1, 2, 3, 4 ou 5 radicaux R⁵¹;
R⁵¹ représente -NHR⁵², -NR⁵³R⁵⁴ ou un radical alkyle choisi dans le groupe constitué par -CF₃, -CH₂-CF₃, méthyle, éthyle, n-propyle, isopropyle, tert-butyle, n-butyle, sec-butyle et isobutyle ;
R⁵², R⁵³ et R⁵⁴ représentent, indépendamment les uns des autres, à chaque fois -C(=O)-R⁵⁵ ;
un radical alkyle choisi dans le groupe constitué par -CF₃, -CH₂-CF₃, méthyle, éthyle, n-propyle, isopropyle, tert-butyle, n-butyle, sec-butyle et isobutyle ;
ou un radical choisi dans le groupe constitué par phényle et naphtyle, le radical pouvant à chaque fois être lié via un groupe -(CH₂)-, -(CH₂)₂- ou - (CH₂)₃- et/ou à chaque fois être non substitué ou le cas échéant substitué par 1, 2, 3, 4 ou 5 substituants choisis, indépendamment les uns des autres, dans le groupe constitué par F, Cl, Br, I, -CN, -CF₃, -O-CH₃, -O-C₂H₅, -O-CH(CH₃)₂, -O-C(CH₃)₃, méthyle, éthyle, n-propyle, isopropyle, n-butyle, sec-butyle, isobutyle, tert-butyle et n-pentyle ; et
R⁵⁵ représente un radical alkyle choisi dans le groupe constitué par -CF₃, -CH₂-CF₃, méthyle, éthyle, n-propyle, isopropyle, tert-butyle, n-butyle, sec-butyle et isobutyle ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

4. Composés selon l'une ou plusieurs des revendications 1 à 3, **caractérisés en ce que**
X représente O ;
n vaut 1 ;
R¹, R³ et R⁴ représentent à chaque fois H ;
R² représente F ; Cl ou Br ;
R⁵ représente un radical alkyle choisi dans le groupe constitué par méthyle, éthyle, n-propyle et isopropyle ;
R⁶ représente -C(=O)-R²⁸ ou un radical choisi dans le groupe constitué par méthyle, -CH₂-CN, éthyle, -CH₂-CH₂-CN, n-propyle, -CH₂-CH₂-CH₂-CN, n-butyle, -CH₂-CH₂-CH₂-CH₂-CN, sec-butyle, isobutyle, tert-butyle et n-pentyle ;
R⁷ représente un radical alkyle choisi dans le groupe constitué par -CH₂-OH, -CH₂-CH₂-OH, -CH₂-CH₂-CH₂-OH, -CH₂-CH₂-CH₂-CH₂-OH, isopropyle, n-butyle, sec-butyle, isobutyle, méthyle, éthyle et n-propyle ;
R⁸ représente un radical hydrogène ;
T représente C-R²⁹ et U représente C-R³⁰ et V représente N et W représente C-R³² ;
R⁹ représente -SF₅ ; -O-CF₃ ; -CF₃ ; -CHF₂ ; -CH₂F; -C(CH₃)₂(CH₂OH) ou tert-butyle ;
R²⁸ représente un radical choisi dans le groupe constitué par méthyle, éthyle, n-propyle, isopropyle, sec-butyle, isobutyle, tert-butyle, éthényle et propényle ;
R²⁹ et R³⁰ représentent à chaque fois H ;
R³² représente H ; -NHR³³ ; -NR³⁴R³⁵; -OR³⁶ ou -SR³⁷ ;
R³³, R³⁶ et R³⁷ représentent, indépendamment les uns des autres, à chaque fois
un radical choisi dans le groupe constitué par cyclopropyle, cyclobutyle, cyclopentyle, cyclohexyle et cycloheptyle, qui peut à chaque fois le cas échéant être substitué par 1, 2, 3, 4 ou 5 substituants choisis, indépendamment les uns des autres, dans le groupe constitué par méthyle, éthyle, n-propyle, isopropyle, n-butyle, sec-butyle, isobutyle, tert-butyle, n-pentyle ;
R³⁴ et R³⁵ forment à chaque fois ensemble avec l'atome d'azote qui les relie comme élément de cycle un radical choisi dans le groupe constitué par pyrrolidinyle, pipéridinyle, pipérazinyle, morpholinyle et azépanyle, dont la partie hétérocycloaliphatique peut à chaque fois être non substituée ou substituée par 1, 2, 3, 4 ou 5 radicaux R⁵¹;
R⁵¹ représente -NHR⁵², -NR⁵³R⁵⁴ ou un radical alkyle choisi dans le groupe constitué par méthyle, éthyle, n-propyle, isopropyle, tert-butyle, n-butyle, sec-butyle et isobutyle ;
R⁵², R⁵³ et R⁵⁹ représentent, indépendamment les uns des autres, à chaque fois -C(=O)-R⁵⁵ ;
un radical alkyle choisi dans le groupe constitué par méthyle, éthyle, n-propyle, isopropyle, tert-butyle, n-butyle, sec-butyle et isobutyle ;
ou un radical phényle, le radical pouvant être substitué par 1, 2, 3, 4 ou 5 substituants choisis, indépendamment les uns des autres, dans le groupe constitué par F, Cl, Br, méthyle, éthyle, n-propyle et isopropyle ; et
R⁵⁵ représente un radical alkyle choisi dans le groupe constitué par méthyle, éthyle, n-propyle, isopropyle, tert-butyle, n-butyle, sec-butyle et isobutyle ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

5. Composés de formule générale Ia selon l'une ou plusieurs des revendications 1 à 4, 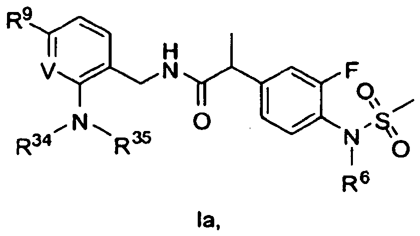 dans laquelle
V représente N ;
et R⁶, R⁹, R³⁴ et R³⁵ ont la signification selon la revendication 3 ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

6. Composés de formule générale Ia selon l'une ou plusieurs des revendications 1 à 5, 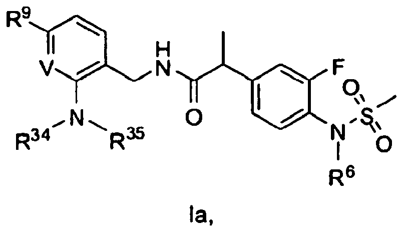 dans laquelle
V représente N ;
et R⁶, R⁹, R³⁴ et R³⁵ ont la signification selon la revendication 4 ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

7. Composés de formule générale Ib selon l'une ou plusieurs des revendications 1 à 4, 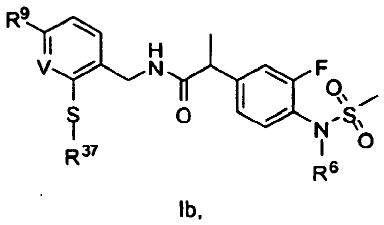 V représente N ;
et R⁶, R⁹ et R³⁷ ont la signification selon la revendication 3 ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

8. Composés de formule générale Ib selon l'une ou plusieurs des revendications 1 à 7, 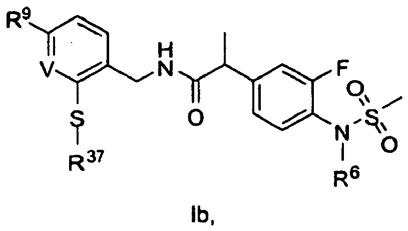 V représente N ;
et R⁶, R⁹ et R³⁷ ont la signification selon la revendication 4 ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

9. Composés selon l'une ou plusieurs des revendications 1 à 8 choisis dans le groupe constitué par :
[1] le N-(2-fluoro-4-(1-oxo-1-(2-(4-(N-phénylpropionamido)pipéridin-1-yl)-6-(trifluorométhyl)pyridin-3-yl)méthylamino)propan-2-yl)phényl)-N-(méthylsulfonyl)propionamide,
[2] le 2-(3-fluoro-4-(N-(méthylsulfonyl)acétamido)phényl)-N-((2-(4-méthylpipéridin-1-yl)-6-(trifluorométhyl)pyridin-3-yl)méthyl)propanamide,
[3] le 2-(3-fluoro-4-(N-méthylméthylsulfonamido)phényl)-N-((2-(4-méthylpipéridin-1-yl)-6-(trifluorométhyl)pyridin-3-yl)méthyl)propanamide,
[7] le N-((2-(cyclohexylthio)-6-(trifluorométhyl)pyridin-3-yl)méthyl)-2-(3-fluoro-4-(N-méthylméthylsulfonamido)phényl)propanamide,
[8] le N-((2-(cyclohexylthio)-6-(trifluorométhyl)pyridin-3-yl)méthyl)-2-(4-(N-éthylméthylsulfonamido)-3-fluorophényl)propanamide,
[9] le 2-(4-(N-éthylméthylsulfonamido)-3-fluorophényl)-N-((2-(4-méthylpipéridin-1-yl)-6-(trifluorométhyl)pyridin-3-yl)méthyl)propanamide,
[10] le N-((6-tert-butyl-2-(4-méthylpipéridin-1-yl)pyridin-3-yl)méthyl)-2-(3-fluoro-4-(N-méthylméthylsulfonamido)phényl)propanamide,
[11] le N-((6-tert-butyl-2-(cyclohexylthio)pyridin-3-yl)méthyl)-2-(3-fluoro-4-(N-méthylméthylsulfonamido)phényl)propanamide,
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

10. Procédé pour la préparation d'un composé selon l'une ou plusieurs des revendications 1 à 9, **caractérisé en ce qu'**on transforme au moins un composé de formule générale II, 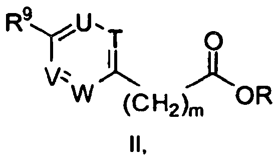 dans laquelle R⁹, U, T, V et W ont la signification selon l'une ou plusieurs des revendications 1 à 9, m vaut 0, 1, 2 ou 3 et R représente hydrogène ou un radical C₁₋₆-alkyle linéaire ou ramifié, dans un milieu réactionnel, en présence d'au moins un agent de réduction, en au moins un composé de formule générale III, 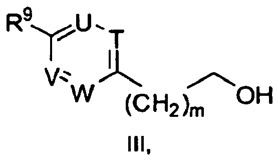 dans laquelle R⁹, U, T, V et W ont la signification selon l'une ou plusieurs des revendications 1 à 9 et m vaut 0, 1, 2 ou 3,
et on transforme au moins un composé de formule générale III dans un milieu réactionnel en présence d'azide de diphénylphosphoryle ou en présence de HN₃ en au moins un composé de formule générale IV, 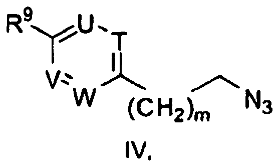 dans laquelle R⁹, U, T, V et W ont la signification selon l'une ou plusieurs des revendications 1 à 9 et m vaut 0, 1, 2 ou 3,
et on transforme au moins un composé de formule générale IV dans un milieu réactionnel en présence d'au moins un agent de réduction
ou dans un mélange réactionnel en présence d'un catalyseur et en présence d'hydrogène ou en présence d'hydrazine
ou dans un milieu réactionnel en présence de triphénylphosphine en au moins un composé de formule générale V, 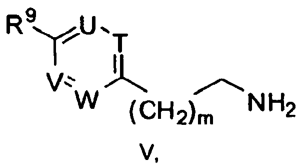 dans laquelle R⁹, U, T, V et W ont la signification selon l'une ou plusieurs des revendications 1 à 9 et m vaut 0, 1, 2 ou 3,
ou on transforme au moins un composé de formule générale VI 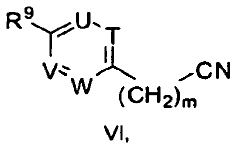 dans laquelle R⁹, U, T, V et W ont la signification selon l'une ou plusieurs des revendications 1 à 9 et m vaut 0, 1, 2 ou 3, dans un milieu réactionnel en présence d'au moins un catalyseur sous une atmosphère d'hydrogène,
ou en présence d'au moins un agent de réduction, choisi dans le groupe constitué par BH₃*S(CH₃)₂, l'hydrure de lithium-aluminium et le borohydrure de sodium,
en au moins un composé de formule générale V, le cas échéant sous forme d'un sel correspondant,
et on transforme au moins un composé de formule générale V avec au moins un composé de formule générale VII, 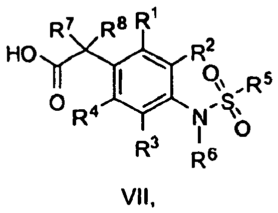 dans laquelle R¹, R², R³, R⁴, R⁵, R⁶, R⁷ et R⁸ ont la signification selon l'une ou plusieurs des revendications 1 à 9 et R⁶ peut représenter en outre un radical hydrogène, dans un milieu réactionnel,
ou avec au moins un composé de formule générale VIII 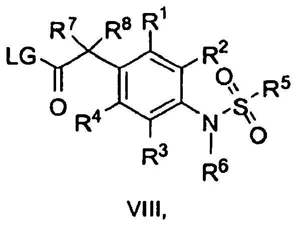 dans laquelle R¹, R², R³, R⁴, R⁵, R⁶, R⁷ et R⁸ ont la signification selon l'une ou plusieurs des revendications 1 à 9, R⁶ peut représenter en outre un radical hydrogène et LG représente un groupe partant, dans un milieu réactionnel, en au moins un composé de formule générale Ih 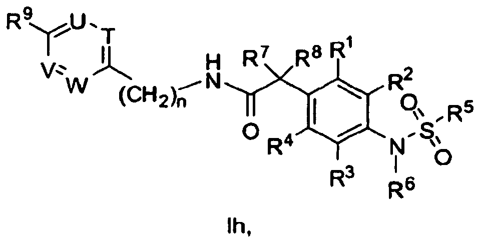 dans laquelle T, U, V, W, R¹, R², R³, R⁴, R⁵, R⁶, R⁷, R⁸ et R⁹ présentent la signification selon l'une ou plusieurs des revendications 1 à 9, R⁶ peut représenter en outre un radical hydrogène et n vaut 1, 2, 3 ou 4,
et le cas échéant on transforme au moins un composé de formule générale Ih dans un milieu réactionnel avec au moins un composé de formule générale IX, 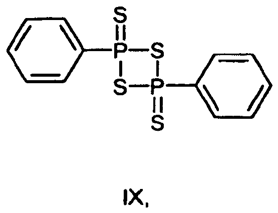 dans laquelle les radicaux phényle sont substitués à chaque fois par 1 ou 2 substituants choisis, indépendamment l'un de l'autre, dans le groupe constitué par méthoxy, phénoxy, Cl, méthyle et Br, ou avec du pentasulfure de phosphore, en au moins un composé de formule générale Ik, 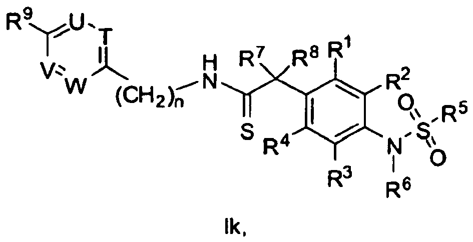 dans laquelle T, U, V, W, R¹, R², R³, R⁴, R⁵, R⁶, R⁷, R⁸ et R⁹ présentent la signification selon l'une ou plusieurs des revendications 1 à 9, R⁶ peut représenter en outre un radical hydrogène et n vaut 1, 2, 3 ou 4,
et le cas échéant on transforme au moins un composé de formule générale Io 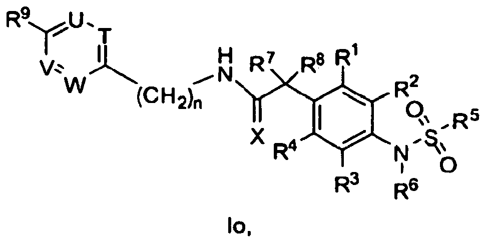 dans laquelle X, T, U, V, W, R¹, R², R³, R⁴, R⁵, R⁷, R⁸ et R⁹ ont la signification selon l'une ou plusieurs des revendications 1 à 9, R⁶ représente un radical hydrogène et n représente 1, 2, 3 ou 4, dans un milieu réactionnel en présence d'au moins un composé de formule générale R²⁸-C(=O)-O-C(=O)-R²⁸, dans laquelle R²⁸ a la signification selon l'une ou plusieurs des revendications 1 à 9, en au moins un composé de formule générale I, dans laquelle X, T, U, V, W, R¹, R², R³, R⁴, R⁵, R⁷, R⁸ et R⁹ ont la signification selon l'une ou plusieurs des revendications 1 à 9, R⁶ représente -C(=O)-R²⁸ et n vaut 1, 2, 3 ou 4.

11. Procédé pour la préparation d'au moins un composé selon l'une ou plusieurs des revendications 1 à 9, **caractérisé en ce qu'**on transforme au moins un composé de formule générale X 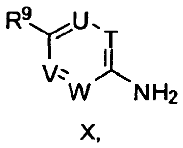 dans laquelle R⁹, U, T, V et W ont la signification selon l'une ou plusieurs des revendications 1 à 9 avec au moins un composé de formule générale VII, 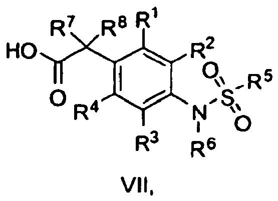 dans laquelle R¹, R², R³, R⁴, R⁵, R⁶, R⁷ et R⁸ ont la signification selon l'une ou plusieurs des revendications 1 à 9 et R⁶ peut représenter en outre un radical hydrogène, dans un milieu réactionnel,
ou avec au moins un composé de formule générale VIII 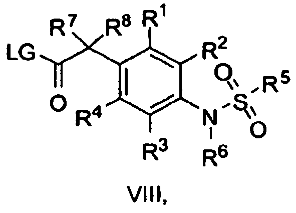 dans laquelle R¹, R², R³, R⁴, R⁵, R⁶, R⁷ et R⁸ ont la signification selon l'une ou plusieurs des revendications 1 à 9, R⁶ peut représenter en outre un radical hydrogène et LG représente un groupe partant, dans un milieu réactionnel, en au moins un composé de formule générale Im 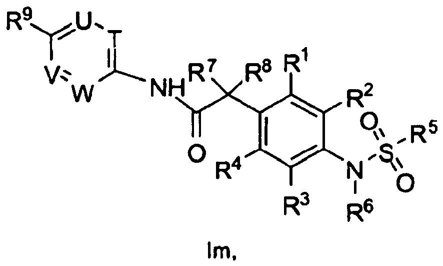 dans laquelle T, U, V, W, R¹, R², R³, R⁴, R⁵, R⁶, R⁷, R⁸ et R⁹ ont la signification selon l'une ou plusieurs des revendications 1 à 9,
et on transforme le cas échéant au moins un composé de formule générale Im dans un milieu réactionnel avec au moins un composé de formule générale IX, 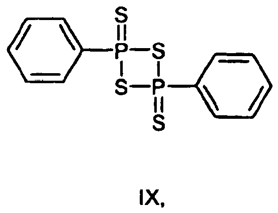 dans laquelle les radicaux phényle sont substitués à chaque fois par 1 ou 2 substituants, choisis indépendamment l'un de l'autre, dans le groupe constitué par méthoxy, phénoxy, Cl, méthyle et Br, ou avec du pentasulfure de phosphore, en au moins un composé de formule générale In, 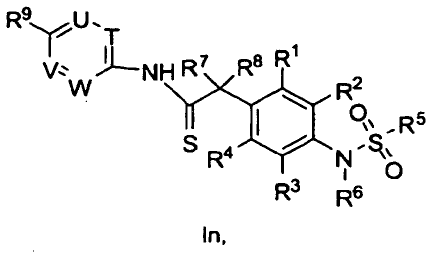 dans laquelle T, U, V, W, R¹, R², R³, R⁴, R⁵, R⁶, R⁷, R⁸ et R⁹ présentent la signification selon l'une ou plusieurs des revendications 1 à 9 et R⁶ peut représenter en outre un radical hydrogène ;
et le cas échéant on transforme au moins un composé de formule générale Ip 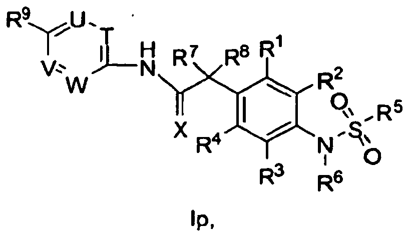 dans laquelle X, T, U, V, W, R¹, R², R³, R⁴, R⁵, R⁷, R⁸ et R⁹ ont la signification selon l'une ou plusieurs des revendications 1 à 9 et R⁶ représente un radical hydrogène, dans un milieu réactionnel en présence d'au moins un composé de formule générale R²⁸-C(=O)-O-C(=O)-R²⁸, dans laquelle R²⁸ a la signification selon l'une ou plusieurs des revendications 1 à 9, en au moins un composé de formule générale I, dans laquelle X, T, U, V, W, R¹, R², R³, R⁴, R⁵, R⁷, R⁸ et R⁹ ont la signification selon l'une ou plusieurs des revendications 1 à 9 et R⁶ représente -C(=O)-R²⁸.

12. Médicament contenant au moins un composé selon l'une ou plusieurs des revendications 1 à 9 et le cas échéant un ou plusieurs adjuvants physiologiquement acceptables.

13. Utilisation d'au moins un composé selon l'une ou plusieurs des revendications 1 à 9 pour la préparation d'un médicament destiné au traitement et/ou à la prophylaxie d'une ou de plusieurs maladies, choisies dans le groupe constitué par la douleur ; la douleur articulaire ; l'hyperalgésie ; l'allodynie ; la causalgie et la migraine.

14. Utilisation d'au moins un composé selon l'une ou plusieurs des revendications 1 à 9 pour la préparation d'un médicament destiné au traitement et/ou à la prophylaxie d'une ou de plusieurs maladies choisies dans le groupe constitué par les dépressions ; les névropathies ; les lésions nerveuses ; les maladies neurodégénératives ; les dysfonctionnements cognitifs ; et l'épilepsie.

15. Utilisation d'au moins un composé selon l'une ou plusieurs des revendications 1 à 9 pour la préparation d'un médicament destiné au traitement et/ou à la prophylaxie d'une ou de plusieurs maladies choisies dans le groupe constitué par les maladies des voies respiratoires ; la toux ; l'incontinence urinaire ; une vessie hyperactive (overactive bladder, OAB) ; les maladies et/ou lésions du tractus gastrointestinal ; les ulcères duodénaux ; les ulcères peptiques ; le syndrome de l'intestin irritable ; les attaques ; les irritations oculaires ; les irritations cutanées ; les maladies neurotiques cutanées ; les maladies allergiques cutanées ; le psoriasis ; le vitiligo ; l'herpes simplex ; les inflammations, de préférence les inflammations de l'intestin, des yeux, de la vessie, de la peau ou de la muqueuse nasale ; la diarrhée ; le prurit ; l'ostéoporose ; l'arthrite ; l'ostéoarthrite ; les maladies rhumatismales ; les troubles d'absorption des aliments ; la dépendance aux médicaments ; l'abus de médicaments ; les symptômes de sevrage lors de l'abus de médicaments ; le développement de la tolérance aux médicaments ; la dépendance aux drogues ; l'abus de drogues ; les symptômes de sevrage lors de la dépendances aux drogues ; la dépendance à l'alcool ; l'abus d'alcool et les symptômes de sevrage lors de la dépendance à l'alcool ; destiné à la diurèse ; à l'antinatriurèse ; à l'influence du système cardiovasculaire ; à l'augmentation de la vigilance ; au traitement de plaies et/ou de brûlures ; au traitement de nerfs sectionnés ; à l'augmentation de la libido ; à la modulation de l'activité motrice ; à l'anxiolyse ; à l'anesthésie locale et/ou à l'inhibition d'effets secondaires non souhaités, déclenchés par l'administration d'agonistes du récepteur 1 des vanilloïdes (récepteurs VR1/TRPV1).
